# EUROPEAN PATENT APPLICATION

(11) **EP 4 008 780 A1**
(43) Date of publication of application: **08.06.2022**
(21) Application number: 21186523.3
(22) Date of filing: 02.05.2016
(51) Int. Cl.: C12N 15/00, C12N 15/864, C12N 15/90, A61K 48/00

(54) **GENE THERAPY FOR AUTOSOMAL DOMINANT DISEASES**

(30) Priority: 30.04.2015 US 201562154966 P
(62) Divisional of application: 16787314.0
(71) Applicant: The Trustees of Columbia University in the City of New York, New York, NY 10027 (US)
(72) Inventor: WU, Wen-hsuan, New York, NY 10032 (US); TSAI, Yi-ting, New York, NY 10032 (US); CHAN, Lawrence, New York, NY 10032 (US); TSANG, Stephen, H., New York, NY 10032 (US)
(74) Representative: Cabinet Becker et Associés

(57) **Abstract**

The present disclosure provides methods for treating autosomal dominant diseases in a subject. In some aspects, the methods involve the use of a gene editing enzyme with a pair of unique guide RNA sequences that targets both mutant and wildtype forms of autosomal dominant disease-related gene for destruction in cells, and then supplying the cells with wildtype autosomal dominant disease-related gene cDNA which is codon modified to evade recognition by the guide RNAs. These methods are broadly applicable to any autosomal dominant disease.

SEQUENCE LISTING

<110> THE TRUSTEES OF COLUMBIA UNIVERSITY IN THE CITY OF NEW YORK

<120> GENE THERAPY FOR AUTOSOMAL DOMINANT DISORDERS

<130> 4361-0017

<140>
<141>

<150> 62/154,966
<151> 2015-04-30

<160> 27

<170> PatentIn version 3.5

<210> 1
<211> 20
<212> DNA
<213> Homo sapiens

<400> 1
ggacggtgac gtagagcgtg                                                    20

<210> 2
<211> 20
<212> DNA
<213> Homo sapiens

<400> 2
gacgaagtat ccatgcagag                                                    20

<210> 3
<211> 20
<212> DNA
<213> Homo sapiens

<400> 3
tgagaccaca aatgaatgct                                                    20

<210> 4
<211> 22
<212> DNA
<213> Mus musculus

<400> 4
aactactaca cactcaagcc tg                                                 22

<210> 5
<211> 20
<212> DNA
<213> Mus musculus

<400> 5
gtagtactgc ggctgctcga                                                    20

<210> 6
<211> 20
<212> DNA
<213> Mus musculus

<400> 6
gctcatgctg ccggcgattc                                                    20


<210> 7
<211> 20
<212> DNA
<213> Drosophila melanogaster

<400> 7
ggttttggac aatggaaccg                                                    20


<210> 8
<211> 1047
<212> DNA
<213> Homo sapiens

<400> 8
atgaatggca cagaaggccc taacttctac gtgcccttct ccaatgcgac gggtgtggta        60

cgcagcccct tcgagtaccc acagtactac ctggctgagc catggcagtt ctccatgctg       120

gccgcctaca tgtttctgct gatcgtgctg ggcttcccca tcaacttcct cacgctctac       180

gtcaccgtcc agcacaagaa gctgcgcacg cctctcaact acatcctgct caacctagcc       240

gtggctgacc tcttcatggt cctaggtggc ttcaccagca ccctctacac ctctctgcat       300

ggatacttcg tcttcgggcc cacaggatgc aatttggagg gcttctttgc caccctgggc       360

ggtgaaattg ccctgtggtc cttggtggtc ctggccatcg agcggtacgt ggtggtgtgt       420

aagcccatga gcaacttccg cttcggggag aaccatgcca tcatgggcgt tgccttcacc       480

tgggtcatgg cgctggcctg cgccgcaccc ccactcgccg gctggtccag gtacatcccc       540

gagggcctgc agtgctcgtg tggaatcgac tactacacgc tcaagccgga ggtcaacaac       600

gagtcttttg tcatctacat gttcgtggtc cacttcacca tccccatgat tatcatcttt       660

ttctgctatg ggcagctcgt cttcaccgtc aaggaggccg ctgcccagca gcaggagtca       720

gccaccacac agaaggcaga gaaggaggtc acccgcatgg tcatcatcat ggtcatcgct       780

ttcctgatct gctgggtgcc ctacgccagc gtggcattct acatcttcac ccaccagggc       840

tccaacttcg gtcccatctt catgaccatc ccagcgttct ttgccaagag cgccgccatc       900

tacaaccctg tcatctatat catgatgaac aagcagttcc ggaactgcat gctcaccacc       960

atctgctgcg gcaagaaccc actgggtgac gatgaggcct ctgctaccgt gtccaagacg      1020

gagacgagcc aggtggcccc ggcctaa                                          1047


<210> 9
<211> 1044

<212> DNA
<213> Homo sapiens

<400> 9
atgaatggca cagaaggccc taacttctac gtgcccttct ccaatgcgac gggtgtggta 60

cgcagcccct tcgagtaccc acagtactac ctggctgagc catggcagtt ctccatgctg 120

gccgcctaca tgtttctgct gatcgtgctg ggcttcccca tcaactttct tacactgtac 180

gtcaccgtcc agcacaagaa gctgcgcacg cctctcaact acatcctgct caacctagcc 240

gtggctgacc tcttcatggt cctaggtggc ttcaccagca ccctctacac gtcgcttcac 300

ggatatttcg tcttcgggcc cacaggatgc aatttggagg gcttctttgc caccctgggc 360

ggtgaaattg ccctgtggtc cttggtggtc ctggccatcg agcggtacgt ggtggtgtgt 420

aagcccatga gcaacttccg cttcggggag aaccatgcca tcatgggcgt tgccttcacc 480

tgggtcatgg cgctggcctg cgccgcaccc ccactcgccg gctggtccag gtacatcccc 540

gagggcctgc agtgctcgtg tggaatcgac tactacacgc tcaagccgga ggtcaacaac 600

gagtcttttg tcatctacat gttcgtggtc cacttcacca tccccatgat tatcatcttt 660

ttctgctatg ggcagctcgt cttcaccgtc aaggaggccg ctgcccagca gcaggagtca 720

gccaccacac agaaggcaga gaaggaggtc acccgcatgg tcatcatcat ggtcatcgct 780

ttcctgatct gctgggtgcc ctacgccagc gtggcattct acatcttcac ccaccagggc 840

tccaacttcg gtcccatctt catgaccatc ccagcgttct ttgccaagag cgccgccatc 900

tacaaccctg tcatctatat catgatgaac aagcagttcc ggaactgcat gctcaccacc 960

atctgctgcg gcaagaaccc actgggtgac gatgaggcct ctgctaccgt gtccaagacg 1020

gagacgagcc aggtggcccc ggcc 1044


<210> 10
<211> 797
<212> DNA
<213> Gallus gallus

<400> 10
cgttacataa cttacggtaa atggcccgcc tggctgaccg cccaacgacc cccgcccatt 60

gacgtcaata gtaacgccaa tagggacttt ccattgacgt caatgggtgg agtatttacg 120

gtaaactgcc cacttggcag tacatcaagt gtatcatatg ccaagtacgc cccctattga 180

cgtcaatgac ggtaaatggc ccgcctggca ttgtgcccag tacatgacct tatgggactt 240

tcctacttgg cagtacatct acgtattagt catcgctatt accatggtcg aggtgagccc 300

cacgttctgc ttcactctcc ccatctcccc cccctcccca cccccaattt tgtatttatt 360

tattttttaa ttattttgtg cagcgatggg ggcggggggg gggggggggc gcgcgccagg 420

cggggcgggg cggggcgagg ggcggggcgg ggcgaggcgg agaggtgcgg cggcagccaa 480

tcagagcggc gcgctccgaa agtttccttt tatggcgagg cggcggcggc ggcggcccta 540

taaaaagcga agcgcgcggc gggcgggagt cgctgcgacg ctgccttcgc cccgtgcccc 600

gctccgccgc cgcctcgcgc cgcccgcccc ggctctgact gaccgcgtta ctcccacagg 660

tgagcgggcg ggacggccct tctcctccgg gctgtaatta gctgagcaag aggtaagggt 720

ttaagggatg gttggttggt ggggtattaa tgtttaatta cctggagcac ctgcctgaaa 780

tcactttttt tcaggtt 797

<210> 11
<211> 232
<212> DNA
<213> Bos taurus

<400> 11
taagagctcg ctgatcagcc tcgactgtgc cttctagttg ccagccatct gttgtttgcc 60

cctcccccgt gccttccttg accctggaag gtgccactcc cactgtcctt tcctaataaa 120

atgaggaaat tgcatcgcat tgtctgagta ggtgtcattc tattctgggg ggtggggtgg 180

ggcaggacag caagggggag gattgggaag agaatagcag gcatgctggg ga 232

<210> 12
<211> 249
<212> DNA
<213> Homo sapiens

<400> 12
gagggcctat ttcccatgat tccttcatat ttgcatatac gatacaaggc tgttagagag 60

ataattggaa ttaatttgac tgtaaacaca aagatattag tacaaaatac gtgacgtaga 120

aagtaataat ttcttgggta gtttgcagtt ttaaaattat gttttaaaat ggactatcat 180

atgcttaccg taacttgaaa gtatttcgat ttcttggctt tatatatctt gtggaaagga 240

cgaaacacc 249

<210> 13
<211> 130
<212> DNA
<213> Adeno-associated virus-2

<400> 13
cgcgctcgct cgctcactga ggccgcccgg gcaaagcccg ggcgtcgggc gacctttggt 60

cgcccggcct cagtgagcga gcgagcgcgc agagagggag tggccaactc catcactagg 120

ggttccttgt 130

<210> 14
<211> 173
<212> DNA
<213> Human herpesvirus 5

<400> 14
actcacgggg atttccaagt ctccacccca ttgacgtcaa tgggagtttg ttttggcacc 60

aaaatcaacg ggactttcca aaatgtcgta ataaccccgc cccgttgacg caaatgggcg 120

gtaggcgtgt acggtgggag gtctatataa gcagagctcg tttagtgaac cgt 173

<210> 15
<211> 66
<212> DNA
<213> Artificial Sequence

<220>
<223> Description of Artificial Sequence: Synthetic
      oligonucleotide

<400> 15
gactataagg accacgacgg agactacaag gatcatgata ttgattacaa agacgatgac 60

gataag 66

<210> 16
<211> 21
<212> DNA
<213> Simian virus 40

<400> 16
ccaaagaaga agcggaaggt c 21

<210> 17
<211> 4101
<212> DNA
<213> Streptococcus pyogenes

<400> 17
gacaagaagt acagcatcgg cctggacatc ggcaccaact ctgtgggctg ggccgtgatc 60

accgacgagt acaaggtgcc cagcaagaaa ttcaaggtgc tgggcaacac cgaccggcac 120

agcatcaaga agaacctgat cggagccctg ctgttcgaca gcggcgaaac agccgaggcc 180

acccggctga agagaaccgc cagaagaaga tacaccagac ggaagaaccg gatctgctat 240

ctgcaagaga tcttcagcaa cgagatggcc aaggtggacg acagcttctt ccacagactg 300

gaagagtcct tcctggtgga agaggataag aagcacgagc ggcaccccat cttcggcaac 360

atcgtggacg aggtggccta ccacgagaag taccccacca tctaccacct gagaaagaaa 420

ctggtggaca gcaccgacaa ggccgacctg cggctgatct atctggccct ggcccacatg 480

atcaagttcc ggggccactt cctgatcgag ggcgacctga accccgacaa cagcgacgtg 540

gacaagctgt tcatccagct ggtgcagacc tacaaccagc tgttcgagga aaaccccatc 600

aacgccagcg gcgtggacgc caaggccatc ctgtctgcca gactgagcaa gagcagacgg 660

ctggaaaatc tgatcgccca gctgcccggc gagaagaaga atggcctgtt cggaaacctg 720

attgccctga gcctgggcct gacccccaac ttcaagagca acttcgacct ggccgaggat 780

gccaaactgc agctgagcaa ggacacctac gacgacgacc tggacaacct gctggcccag 840

atcggcgacc agtacgccga cctgtttctg gccgccaaga acctgtccga cgccatcctg 900

ctgagcgaca tcctgagagt gaacaccgag atcaccaagg cccccctgag cgcctctatg 960

atcaagagat acgacgagca ccaccaggac ctgaccctgc tgaaagctct cgtgcggcag 1020

cagctgcctg agaagtacaa agagattttc ttcgaccaga gcaagaacgg ctacgccggc 1080

tacattgacg gcggagccag ccaggaagag ttctacaagt tcatcaagcc catcctggaa 1140

aagatggacg gcaccgagga actgctcgtg aagctgaaca gagaggacct gctgcggaag 1200

cagcggacct tcgacaacgg cagcatcccc caccagatcc acctgggaga gctgcacgcc 1260

attctgcggc ggcaggaaga tttttaccca ttcctgaagg acaaccggga aaagatcgag 1320

aagatcctga ccttccgcat cccctactac gtgggccctc tggccagggg aaacagcaga 1380

ttcgcctgga tgaccagaaa gagcgaggaa accatcaccc cctggaactt cgaggaagtg 1440

gtggacaagg gcgcttccgc ccagagcttc atcgagcgga tgaccaactt cgataagaac 1500

ctgcccaacg agaaggtgct gcccaagcac agcctgctgt acgagtactt caccgtgtat 1560

aacgagctga ccaaagtgaa atacgtgacc gagggaatga gaaagcccgc cttcctgagc 1620

ggcgagcaga aaaaggccat cgtggacctg ctgttcaaga ccaaccggaa agtgaccgtg 1680

aagcagctga aagaggacta cttcaagaaa atcgagtgct tcgactccgt ggaaatctcc 1740

ggcgtggaag atcggttcaa cgcctccctg ggcacatacc acgatctgct gaaaattatc 1800

aaggacaagg acttcctgga caatgaggaa aacgaggaca ttctggaaga tatcgtgctg 1860

accctgacac tgtttgagga cagagagatg atcgaggaac ggctgaaaac ctatgcccac 1920

ctgttcgacg acaaagtgat gaagcagctg aagcggcgga gatacaccgg ctggggcagg 1980

ctgagccgga agctgatcaa cggcatccgg gacaagcagt ccggcaagac aatcctggat 2040

ttcctgaagt ccgacggctt cgccaacaga aacttcatgc agctgatcca cgacgacagc 2100

ctgaccttta aagaggacat ccagaaagcc caggtgtccg gccagggcga tagcctgcac 2160

gagcacattg ccaatctggc cggcagcccc gccattaaga agggcatcct gcagacagtg 2220

aaggtggtgg acgagctcgt gaaagtgatg ggccggcaca agcccgagaa catcgtgatc 2280

gaaatggcca gagagaacca gaccacccag aagggacaga agaacagccg cgagagaatg 2340

aagcggatcg aagagggcat caaagagctg ggcagccaga tcctgaaaga acaccccgtg 2400

gaaaacaccc agctgcagaa cgagaagctg tacctgtact acctgcagaa tgggcgggat 2460

atgtacgtgg accaggaact ggacatcaac cggctgtccg actacgatgt ggaccatatc 2520

gtgcctcaga gctttctgaa ggacgactcc atcgacaaca aggtgctgac cagaagcgac 2580

aagaaccggg gcaagagcga caacgtgccc tccgaagagg tcgtgaagaa gatgaagaac 2640

tactggcggc agctgctgaa cgccaagctg attacccaga gaaagttcga caatctgacc 2700

aaggccgaga gaggcggcct gagcgaactg gataaggccg gcttcatcaa gagacagctg 2760

gtggaaaccc ggcagatcac aaagcacgtg gcacagatcc tggactcccg gatgaacact 2820

aagtacgacg agaatgacaa gctgatccgg gaagtgaaag tgatcaccct gaagtccaag 2880

ctggtgtccg atttccggaa ggatttccag ttttacaaag tgcgcgagat caacaactac 2940

caccacgccc acgacgccta cctgaacgcc gtcgtgggaa ccgccctgat caaaaagtac 3000

cctaagctgg aaagcgagtt cgtgtacggc gactacaagg tgtacgacgt gcggaagatg 3060

atcgccaaga gcgagcagga aatcggcaag gctaccgcca agtacttctt ctacagcaac 3120

atcatgaact ttttcaagac cgagattacc ctggccaacg gcgagatccg gaagcggcct 3180

ctgatcgaga caaacggcga aaccggggag atcgtgtggg ataagggccg ggattttgcc 3240

accgtgcgga aagtgctgag catgccccaa gtgaatatcg tgaaaaagac cgaggtgcag 3300

acaggcggct tcagcaaaga gtctatcctg cccaagagga acagcgataa gctgatcgcc 3360

agaaagaagg actgggaccc taagaagtac ggcggcttcg acagccccac cgtggcctat 3420

tctgtgctgg tggtggccaa agtggaaaag ggcaagtcca agaaactgaa gagtgtgaaa 3480

gagctgctgg ggatcaccat catggaaaga agcagcttcg agaagaatcc catcgacttt 3540

ctggaagcca agggctacaa agaagtgaaa aaggacctga tcatcaagct gcctaagtac 3600

tccctgttcg agctggaaaa cggccggaag agaatgctgg cctctgccgg cgaactgcag 3660

aagggaaacg aactggccct gccctccaaa tatgtgaact tcctgtacct ggccagccac 3720

tatgagaagc tgaagggctc ccccgaggat aatgagcaga aacagctgtt tgtggaacag 3780

cacaagcact acctggacga gatcatcgag cagatcagcg agttctccaa gagagtgatc 3840

ctggccgacg ctaatctgga caaagtgctg tccgcctaca acaagcaccg ggataagccc 3900

atcagagagc aggccgagaa tatcatccac ctgtttaccc tgaccaatct gggagcccct 3960

gccgccttca agtactttga caccaccatc gaccggaaga ggtacaccag caccaaagag 4020

gtgctggacg ccaccctgat ccaccagagc atcaccggcc tgtacgagac acggatcgac 4080

ctgtctcagc tgggaggcga c 4101

<210> 18
<211> 48
<212> DNA
<213> Xenopus laevis

<400> 18
aaaaggccgg cggccacgaa aaaggccggc caggcaaaaa agaaaaag 48

<210> 19
<211> 49
<212> DNA

<213> Oryctolagus cuniculus

<400> 19
aataaaagat ctttattttc attagatctg tgtgttggtt ttttgtgtg          49


<210> 20
<211> 508
<212> DNA
<213> Human herpesvirus 5

<400> 20
cgttacataa cttacggtaa atggcccgcc tggctgaccg cccaacgacc cccgcccatt          60

gacgtcaata atgacgtatg ttcccatagt aacgccaata gggactttcc attgacgtca          120

atgggtggag tatttacggt aaactgccca cttggcagta catcaagtgt atcatatgcc          180

aagtacgccc cctattgacg tcaatgacgg taaatggccc gcctggcatt atgcccagta          240

catgacctta tgggactttc ctacttggca gtacatctac gtattagtca tcgctattac          300

catggtgatg cggttttggc agtacatcaa tgggcgtgga tagcggtttg actcacgggg          360

atttccaagt ctccacccca ttgacgtcaa tgggagtttg ttttggcacc aaaatcaacg          420

ggactttcca aaatgtcgta acaactccgc cccattgacg caaatgggcg gtaggcgtgt          480

acggtgggag gtctatataa gcagagct          508


<210> 21
<211> 720
<212> DNA
<213> Aequorea victoria

<400> 21
atggtgagca agggcgagga gctgttcacc ggggtggtgc ccatcctggt cgagctggac          60

ggcgacgtaa acggccacaa gttcagcgtg tccggcgagg gcgagggcga tgccacctac          120

ggcaagctga ccctgaagtt catctgcacc accggcaagc tgcccgtgcc ctggcccacc          180

ctcgtgacca ccctgaccta cggcgtgcag tgcttcagcc gctaccccga ccacatgaag          240

cagcacgact tcttcaagtc cgccatgccc gaaggctacg tccaggagcg caccatcttc          300

ttcaaggacg acggcaacta caagacccgc gccgaggtga agttcgaggg cgacaccctg          360

gtgaaccgca tcgagctgaa gggcatcgac ttcaaggagg acggcaacat cctggggcac          420

aagctggagt acaactacaa cagccacaac gtctatatca tggccgacaa gcagaagaac          480

ggcatcaagg tgaacttcaa gatccgccac aacatcgagg acggcagcgt gcagctcgcc          540

gaccactacc agcagaacac ccccatcggc gacggccccg tgctgctgcc cgacaaccac          600

tacctgagca cccagtccgc cctgagcaaa gaccccaacg agaagcgcga tcacatggtc          660

ctgctggagt tcgtgaccgc cgccgggatc actctcggca tggacgagct gtacaagtaa          720


<210> 22

<211> 9175
<212> DNA
<213> Streptococcus pyogenes

<400> 22
   gagggcctat ttcccatgat tccttcatat ttgcatatac gatacaaggc tgttagagag 60
   ataattggaa ttaatttgac tgtaaacaca aagatattag tacaaaatac gtgacgtaga 120
   aagtaataat ttcttgggta gtttgcagtt ttaaaattat gttttaaaat ggactatcat 180
   atgcttaccg taacttgaaa gtatttcgat ttcttggctt tatatatctt gtggaaagga 240
   cgaaacaccg ggtcttcgag aagacctgtt ttagagctag aaatagcaag ttaaaataag 300
   gctagtccgt tatcaacttg aaaaagtggc accgagtcgg tgcttttttg ttttagagct 360
   agaaatagca agttaaaata aggctagtcc gtttttagcg cgtgcgccaa ttctgcagac 420
   aaatggctct agaggtaccc gttacataac ttacggtaaa tggcccgcct ggctgaccgc 480
   ccaacgaccc ccgcccattg acgtcaatag taacgccaat agggactttc cattgacgtc 540
   aatgggtgga gtatttacgg taaactgccc acttggcagt acatcaagtg tatcatatgc 600
   caagtacgcc ccctattgac gtcaatgacg gtaaatggcc cgcctggcat tgtgcccagt 660
   acatgacctt atgggacttt cctacttggc agtacatcta cgtattagtc atcgctatta 720
   ccatggtcga ggtgagcccc acgttctgct tcactctccc catctccccc ccctccccac 780
   ccccaatttt gtatttattt atttttaatt tattttgtgc agcgatgggg gcgggggggg 840
   ggggggggcg cgcgccaggc ggggcggggc ggggcgaggg gcggggcggg gcgaggcgga 900
   gaggtgcggc ggcagccaat cagagcggcg cgctccgaaa gtttcctttt atggcgaggc 960
   ggcggcggcg gcggccctat aaaaagcgaa gcgcgcggcg ggcgggagtc gctgcgacgc 1020
   tgccttcgcc ccgtgccccg ctccgccgcc gcctcgcgcc gcccgccccg gctctgactg 1080
   accgcgttac tcccacaggt gagcgggcgg gacggccctt ctcctccggg ctgtaattag 1140
   ctgagcaaga ggtaagggtt taagggatgg ttggttggtg gggtattaat gtttaattac 1200
   ctggagcacc tgcctgaaat cacttttttt caggttggac cggtgccacc atggactata 1260
   aggaccacga cggagactac aaggatcatg atattgatta caaagacgat gacgataaga 1320
   tggccccaaa gaagaagcgg aaggtcggta tccacggagt cccagcagcc gacaagaagt 1380
   acagcatcgg cctggacatc ggcaccaact ctgtgggctg ggccgtgatc accgacgagt 1440
   acaaggtgcc cagcaagaaa ttcaaggtgc tgggcaacac cgaccggcac agcatcaaga 1500
   agaacctgat cggagccctg ctgttcgaca gcggcgaaac agccgaggcc acccggctga 1560
   agagaaccgc cagaagaaga tacaccagac ggaagaaccg gatctgctat ctgcaagaga 1620
   tcttcagcaa cgagatggcc aaggtggacg acagcttctt ccacagactg gaagagtcct 1680
   tcctggtgga agaggataag aagcacgagc ggcaccccat cttcggcaac atcgtggacg 1740
   aggtggccta ccacgagaag taccccacca tctaccacct gagaaagaaa ctggtggaca 1800
   gcaccgacaa ggccgacctg cggctgatct atctggccct ggcccacatg atcaagttcc 1860
   ggggccactt cctgatcgag ggcgacctga accccgacaa cagcgacgtg gacaagctgt 1920
   tcatccagct ggtgcagacc tacaaccagc tgttcgagga aaaccccatc aacgccagcg 1980
   gcgtggacgc caaggccatc ctgtctgcca gactgagcaa gagcagacgg ctggaaaatc 2040
   tgatcgccca gctgcccggc gagaagaaga atggcctgtt cggaaacctg attgccctga 2100
   gcctgggcct gacccccaac ttcaagagca acttcgacct ggccgaggat gccaaactgc 2160
   agctgagcaa ggacacctac gacgacgacc tggacaacct gctggcccag atcggcgacc 2220
   agtacgccga cctgtttctg gccgccaaga acctgtccga cgccatcctg ctgagcgaca 2280
   tcctgagagt gaacaccgag atcaccaagg cccccctgag cgcctctatg atcaagagat 2340
   acgacgagca ccaccaggac ctgaccctgc tgaaagctct cgtgcggcag cagctgcctg 2400
   agaagtacaa agagattttc ttcgaccaga gcaagaacgg ctacgccggc tacattgacg 2460
   gcggagccag ccaggaagag ttctacaagt tcatcaagcc catcctggaa aagatggacg 2520
   gcaccgagga actgctcgtg aagctgaaca gagaggacct gctgcggaag cagcggacct 2580
   tcgacaacgg cagcatcccc caccagatcc acctgggaga gctgcacgcc attctgcggc 2640
   ggcaggaaga tttttaccca ttcctgaagg acaaccggga aaagatcgag aagatcctga 2700
   ccttccgcat cccctactac gtgggccctc tggccagggg aaacagcaga ttcgcctgga 2760
   tgaccagaaa gagcgaggaa accatcaccc cctggaactt cgaggaagtg gtggacaagg 2820
   gcgcttccgc ccagagcttc atcgagcgga tgaccaactt cgataagaac ctgcccaacg 2880
   agaaggtgct gcccaagcac agcctgctgt acgagtactt caccgtgtat aacgagctga 2940
   ccaaagtgaa atacgtgacc gagggaatga gaaagcccgc cttcctgagc ggcgagcaga 3000
   aaaaggccat cgtggacctg ctgttcaaga ccaaccggaa agtgaccgtg aagcagctga 3060
   aagaggacta cttcaagaaa atcgagtgct tcgactccgt ggaaatctcc ggcgtggaag 3120
   atcggttcaa cgcctccctg ggcacatacc acgatctgct gaaaattatc aaggacaagg 3180
   acttcctgga caatgaggaa aacgaggaca ttctggaaga tatcgtgctg accctgacac 3240
   tgtttgagga cagagagatg atcgaggaac ggctgaaaac ctatgcccac ctgttcgacg 3300
   acaaagtgat gaagcagctg aagcggcgga gatacaccgg ctggggcagg ctgagccgga 3360
   agctgatcaa cggcatccgg gacaagcagt ccggcaagac aatcctggat ttcctgaagt 3420
   ccgacggctt cgccaacaga aacttcatgc agctgatcca cgacgacagc ctgaccttta 3480
   aagaggacat ccagaaagcc caggtgtccg gccagggcga tagcctgcac gagcacattg 3540
   ccaatctggc cggcagcccc gccattaaga agggcatcct gcagacagtg aaggtggtgg 3600
   acgagctcgt gaaagtgatg ggccggcaca agcccgagaa catcgtgatc gaaatggcca 3660
   gagagaacca gaccacccag aagggacaga agaacagccg cgagagaatg aagcggatcg 3720
   aagagggcat caaagagctg ggcagccaga tcctgaaaga acaccccgtg gaaaacaccc 3780
   agctgcagaa cgagaagctg tacctgtact acctgcagaa tgggcgggat atgtacgtgg 3840
   accaggaact ggacatcaac cggctgtccg actacgatgt ggaccatatc gtgcctcaga 3900
   gctttctgaa ggacgactcc atcgacaaca aggtgctgac cagaagcgac aagaaccggg 3960
   gcaagagcga caacgtgccc tccgaagagg tcgtgaagaa gatgaagaac tactggcggc 4020
   agctgctgaa cgccaagctg attacccaga gaaagttcga caatctgacc aaggccgaga 4080
   gaggcggcct gagcgaactg gataaggccg gcttcatcaa gagacagctg gtggaaaccc 4140
   ggcagatcac aaagcacgtg gcacagatcc tggactcccg gatgaacact aagtacgacg 4200
   agaatgacaa gctgatccgg gaagtgaaag tgatcaccct gaagtccaag ctggtgtccg 4260
   atttccggaa ggatttccag ttttacaaag tgcgcgagat caacaactac caccacgccc 4320
   acgacgccta cctgaacgcc gtcgtgggaa ccgccctgat caaaaagtac cctaagctgg 4380
   aaagcgagtt cgtgtacggc gactacaagg tgtacgacgt gcggaagatg atcgccaaga 4440
   gcgagcagga aatcggcaag gctaccgcca agtacttctt ctacagcaac atcatgaact 4500
   ttttcaagac cgagattacc ctggccaacg gcgagatccg gaagcggcct ctgatcgaga 4560
   caaacggcga aaccggggag atcgtgtggg ataagggccg ggattttgcc accgtgcgga 4620
   aagtgctgag catgccccaa gtgaatatcg tgaaaaagac cgaggtgcag acaggcggct 4680
   tcagcaaaga gtctatcctg cccaagagga acagcgataa gctgatcgcc agaaagaagg 4740
   actgggaccc taagaagtac ggcggcttcg acagccccac cgtggcctat tctgtgctgg 4800
   tggtggccaa agtggaaaag ggcaagtcca agaaactgaa gagtgtgaaa gagctgctgg 4860
   ggatcaccat catggaaaga agcagcttcg agaagaatcc catcgacttt ctggaagcca 4920
   agggctacaa agaagtgaaa aaggacctga tcatcaagct gcctaagtac tccctgttcg 4980
   agctggaaaa cggccggaag agaatgctgg cctctgccgg cgaactgcag aagggaaacg 5040
   aactggccct gccctccaaa tatgtgaact tcctgtacct ggccagccac tatgagaagc 5100
   tgaagggctc ccccgaggat aatgagcaga aacagctgtt tgtggaacag cacaagcact 5160
   acctggacga gatcatcgag cagatcagcg agttctccaa gagagtgatc ctggccgacg 5220
   ctaatctgga caaagtgctg tccgcctaca acaagcaccg ggataagccc atcagagagc 5280
   aggccgagaa tatcatccac ctgtttaccc tgaccaatct gggagcccct gccgccttca 5340
   agtactttga caccaccatc gaccggaaga ggtacaccag caccaaagag gtgctggacg 5400
   ccaccctgat ccaccagagc atcaccggcc tgtacgagac acggatcgac ctgtctcagc 5460
   tgggaggcga caaaaggccg gcggccacga aaaaggccgg ccaggcaaaa aagaaaaagg 5520
   aattcggcag tggagagggc agaggaagtc tgctaacatg cggtgacgtc gaggagaatc 5580
   ctggcccaat gaccgagtac aagcccacgg tgcgcctcgc cacccgcgac gacgtcccca 5640
   gggccgtacg caccctcgcc gccgcgttcg ccgactaccc cgccacgcgc cacaccgtcg 5700
   atccggaccg ccacatcgag cgggtcaccg agctgcaaga actcttcctc acgcgcgtcg 5760
   ggctcgacat cggcaaggtg tgggtcgcgg acgacggcgc cgcggtggcg gtctggacca 5820
   cgccggagag cgtcgaagcg ggggcggtgt tcgccgagat cggcccgcgc atggccgagt 5880
   tgagcggttc ccggctggcc gcgcagcaac agatggaagg cctcctggcg ccgcaccggc 5940
   ccaaggagcc cgcgtggttc ctggccaccg tcggagtctc gcccgaccac cagggcaagg 6000
   gtctgggcag cgccgtcgtg ctccccggag tggaggcggc cgagcgcgcc ggggtgcccg 6060
   ccttcctgga gacctccgcg ccccgcaacc tccccttcta cgagcggctc ggcttcaccg 6120
   tcaccgccga cgtcgaggtg cccgaaggac cgcgcacctg gtgcatgacc cgcaagcccg 6180
   gtgcctgaga attctaacta gagctcgctg atcagcctcg actgtgcctt ctagttgcca 6240
   gccatctgtt gtttgcccct cccccgtgcc ttccttgacc ctggaaggtg ccactcccac 6300
   tgtcctttcc taataaaatg aggaaattgc atcgcattgt ctgagtaggt gtcattctat 6360
   tctggggggt ggggtggggc aggacagcaa gggggaggat tgggaagaga atagcaggca 6420
   tgctggggag cggccgcagg aacccctagt gatggagttg gccactccct ctctgcgcgc 6480
   tcgctcgctc actgaggccg ggcgaccaaa ggtcgcccga cgcccgggct ttgcccgggc 6540
   ggcctcagtg agcgagcgag cgcgcagctg cctgcagggg cgcctgatgc ggtattttct 6600
   ccttacgcat ctgtgcggta tttcacaccg catacgtcaa agcaaccata gtacgcgccc 6660
   tgtagcggcg cattaagcgc ggcgggtgtg gtggttacgc gcagcgtgac cgctacactt 6720
   gccagcgccc tagcgcccgc tcctttcgct ttcttccctt cctttctcgc cacgttcgcc 6780
   ggctttcccc gtcaagctct aaatcggggg ctccctttag ggttccgatt tagtgcttta 6840
   cggcacctcg accccaaaaa acttgatttg ggtgatggtt cacgtagtgg gccatcgccc 6900
   tgatagacgg tttttcgccc tttgacgttg gagtccacgt tctttaatag tggactcttg 6960
   ttccaaactg gaacaacact caaccctatc tcgggctatt cttttgattt ataagggatt 7020
   ttgccgattt cggcctattg gttaaaaaat gagctgattt aacaaaaatt taacgcgaat 7080
   tttaacaaaa tattaacgtt tacaatttta tggtgcactc tcagtacaat ctgctctgat 7140
   gccgcatagt taagccagcc ccgacacccg ccaacacccg ctgacgcgcc ctgacgggct 7200
   tgtctgctcc cggcatccgc ttacagacaa gctgtgaccg tctccgggag ctgcatgtgt 7260
   cagaggtttt caccgtcatc accgaaacgc gcgagacgaa agggcctcgt gatacgccta 7320
   tttttatagg ttaatgtcat gataataatg gtttcttaga cgtcaggtgg cacttttcgg 7380
   ggaaatgtgc gcggaacccc tatttgttta tttttctaaa tacattcaaa tatgtatccg 7440
   ctcatgagac aataaccctg ataaatgctt caataatatt gaaaaaggaa gagtatgagt 7500
   attcaacatt tccgtgtcgc ccttattccc ttttttgcgg cattttgcct tcctgttttt 7560
   gctcacccag aaacgctggt gaaagtaaaa gatgctgaag atcagttggg tgcacgagtg 7620
   ggttacatcg aactggatct caacagcggt aagatccttg agagttttcg ccccgaagaa 7680
   cgttttccaa tgatgagcac ttttaaagtt ctgctatgtg gcgcggtatt atcccgtatt 7740
   gacgccgggc aagagcaact cggtcgccgc atacactatt ctcagaatga cttggttgag 7800
   tactcaccag tcacagaaaa gcatcttacg gatggcatga cagtaagaga attatgcagt 7860
   gctgccataa ccatgagtga taacactgcg gccaacttac ttctgacaac gatcggagga 7920
   ccgaaggagc taaccgcttt tttgcacaac atgggggatc atgtaactcg ccttgatcgt 7980
   tgggaaccgg agctgaatga agccatacca aacgacgagc gtgacaccac gatgcctgta 8040
   gcaatggcaa caacgttgcg caaactatta actggcgaac tacttactct agcttcccgg 8100
   caacaattaa tagactggat ggaggcggat aaagttgcag gaccacttct gcgctcggcc 8160
   cttccggctg gctggtttat tgctgataaa tctggagccg gtgagcgtgg aagccgcggt 8220
   atcattgcag cactggggcc agatggtaag ccctcccgta tcgtagttat ctacacgacg 8280
   gggagtcagg caactatgga tgaacgaaat agacagatcg ctgagatagg tgcctcactg 8340
   attaagcatt ggtaactgtc agaccaagtt tactcatata tactttagat tgatttaaaa 8400
   cttcattttt aatttaaaag gatctaggtg aagatccttt ttgataatct catgaccaaa 8460
   atcccttaac gtgagttttc gttccactga gcgtcagacc ccgtagaaaa gatcaaagga 8520
   tcttcttgag atcctttttt tctgcgcgta atctgctgct tgcaaacaaa aaaaccaccg 8580
   ctaccagcgg tggtttgttt gccggatcaa gagctaccaa ctctttttcc gaaggtaact 8640
   ggcttcagca gagcgcagat accaaatact gtccttctag tgtagccgta gttaggccac 8700
   cacttcaaga actctgtagc accgcctaca tacctcgctc tgctaatcct gttaccagtg 8760
   gctgctgcca gtggcgataa gtcgtgtctt accgggttgg actcaagacg atagttaccg 8820
   gataaggcgc agcggtcggg ctgaacgggg ggttcgtgca cacagcccag cttggagcga 8880
   acgacctaca ccgaactgag atacctacag cgtgagctat gagaaagcgc cacgcttccc 8940
   gaagggagaa aggcggacag gtatccggta agcggcaggg tcggaacagg agagcgcacg 9000
   agggagcttc cagggggaaa cgcctggtat ctttatagtc ctgtcgggtt tcgccacctc 9060
   tgacttgagc gtcgattttt gtgatgctcg tcaggggggc ggagcctatg gaaaaacgcc 9120
   agcaacgcgg cctttttacg gttcctggcc ttttgctggc cttttgctca catgt 9175

<210> 23
<211> 380
<212> DNA

<213> Mus musculus

<400> 23
tcccttaacc accgaaggca gggcagcagg ctagtggagc agagctgcgt ggtcaagtgg 60

cagggagctt aagaatcgtc caagggcgga gaccagtaag tctcattagg tgatggggcc 120

agcaggtaaa agccattcat gcttatgtcc agctgggcgt gtgttctctt cctgttttat 180

catcccttgc gctgaccatc aggtacatcc ctgagggcat gcaatgttca tgcgggattg 240

actattatac ccttaagccg gaggtcaaca acgaatcctt tgtcatctac atgttcgtgg 300

tccacttcac cattcctatg atcgtcatct tcttctgcta tgggcagctg gtcttcacag 360

tcaaggaggt atgagcaggg 380


<210> 24
<211> 1958
<212> DNA
<213> Unknown

<220>
<223> Description of Unknown:
      Mouse or Human polynucleotide

<400> 24
atgctcacct gaataacctg gcagcctgct ccctcatgca gggaccacgt cctgctgcac 60

ccagcaggcc atcccgtctc catagcccat ggtcatccct ccctggacag gaatgtgtct 120

cctccccggg ctgagtcttg ctcaagctag aagcactccg aacagggtta tgggcgcctc 180

ctccatctcc caagtggctg gcttatgaat gtttaatgta catgtgagtg aacaaattcc 240

aattgaacgc aacaaatagt tatcgagccg ctgagccggg gggcgggggg tgtgagactg 300

gaggcgatgg acggagctga cggcacacac agctcagatc tgtcaagtga gccattgtca 360

gggcttgggg actggataag tcagggggtc tcctgggaag agatgggata ggtgagttca 420

ggaggagaca ttgtcaactg gagccatgtg gagaagtgaa tttagggccc aaaggttcca 480

gtcgcagcct gaggccacca gactgacatg gggaggaatt cccagaggac tctggggcag 540

acaagatgag acaccctttc ctttctttac ctaagggcct ccacccgatg tcaccttggc 600

ccctctgcaa gccaattagg ccccggtggc agcagtggga ttagcgttag tatgatatct 660

cgcggatgct gaatcagcct ctggcttagg gagagaaggt cactttataa gggtctgggg 720

ggggtcagtg cctggagttg cgctgtggga gcgagtcatc cagctggagc cctgagtggc 780

tgagctcagg ccttcgcagc attcttgggt gggagcagcc acgggtcagc cacaagggcc 840

acagccatga atggcacaga aggccctaac ttctacgtgc ccttctccaa tgcgacgggt 900

gtggtacgca gccccttcga gtacccacag tactacctgg ctgagccatg gcagttctcc 960

atgctggccg cctacatgtt tctgctgatc gtgctgggct tccccatcaa cttcctcacg 1020

ctctacgtca ccgtccagca caagaagctg cgcacgcctc tcaactacat cctgctcaac 1080

ctagccgtgg ctgacctctt catggtccta ggtggcttca ccagcaccct ctacacctct 1140

ctgcatggat acttcgtctt cgggcccaca ggatgcaatt tggagggctt ctttgccacc 1200

ctgggcggta tgagcagaga gactggggcg ggggggtgta gcatgggagc caaggggcca 1260

cgaaagggcc tgggagggtc tgcagcttac ttgagtctct ttaattggtc tcatctaaag 1320

gcccagctta ttcattggca aacactgtga ccctgagcta ggctgctgtt gagagcaggc 1380

acggaacatt catctatctc atcttgagca atgcaagaaa catgggttca gagaggccaa 1440

ggactcaccg aggagtcaca gagtgtgggg gtgtcctctg aggcagctga gctggggcac 1500

acacagactg agcaccagga gtgagctcta gctttttttt ttctatgtgt cttttctaaa 1560

agacacatag gtttaggact gtccctggtc caggtaagaa ctggttcagt aaacttgtac 1620

atctcactgc ctggccagcc ctgtcagctt ccaccagagt gcgtgcacta cacacccggc 1680

atctcaaagg attcattcct atctttccta tctttggagt gaggcacagt ctcacgtagt 1740

ccagtccaga ctggccttaa attctgcagc tgaggatgta cttaaacttg tcatcctcct 1800

gccccagcct ctcaagtgct gtgatcacag gcacggacca ctatgctacg ccaggtgttt 1860

ccaaacattt tctctccctt aactggaagg tcaatgaggc tctttcgaga agcaacagag 1920

cctgtttagc tgagaaaact gaggcaggga gcaggcaa 1958

<210> 25
<211> 1958
<212> DNA
<213> Unknown

<220>
<223> Description of Unknown:
      Mouse or Human polynucleotide

<400> 25
atgctcacct gaataacctg gcagcctgct ccctcatgca gggaccacgt cctgctgcac 60

ccagcaggcc atcccgtctc catagcccat ggtcatccct ccctggacag gaatgtgtct 120

cctccccggg ctgagtcttg ctcaagctag aagcactccg aacaggttta tgggcgcctc 180

ctccatctcc caagtggctg gcttatgaat gtttaatgta catgtgagtg aacaaattcc 240

aattgaacgc aacaaatagt tatcgagccg ctgagccggg gggcgggggg tgtgagactg 300

gaggcgatgg acggagctga cggcacacac agctcagatc tgtcaagtga gccattgtca 360

gggcttgggg actggataag tcagggggtc tcctgggaag agatgggata ggtgagttca 420

ggaggagaca ttgtcaactg gagccatgtg gagaagtgaa tttagggccc aaaggttcca 480

gtcgcagcct gaggccacca gactgacatg ggaggaattt cccagaggac tctggggcag 540

acaagatgag acaccctttc ctttctttac ctaagggcct ccacccgatg tcaccttggc 600

ccctctgcaa gccaattagg ccccggtggc agcagtggga ttagcgttag tatgatatct 660

cgcggatgct gaatcagcct ctggcttagg gagagaaggt cactttataa gggtctgggg 720

ggggtcagtg cctggagttg cgctgtggga gcgagtcatc cagctggagc cctgagtggc 780

tgagctcagg ccttcgcagc attcttgggt gggagcagcc acgggtcagc cacaagggcc 840

acagccatga atggcacaga aggccctaac ttctacgtgc ccttctccaa tgcgacgggt 900

gtggtacgca gccccttcga gtacccacag tactacctgg ctgagccatg gcagttctcc 960

atgctggccg cctacatgtt tctgctgatc gtgctgggct tccccatcaa cttcctcacg 1020

ctctacgtca ccgtccagca caagaagctg cgcacgcctc tcaactacat cctgctcaac 1080

ctagccgtgg ctgacctctt catggtccta ggtggcttca ccagcaccct ctacacctct 1140

ctgcatggat acttcgtctt cgggcccaca ggacgcaatt tggagggctt ctttgccacc 1200

ctgggcggta tgagcagaga gactggggcg ggggggtgta gcatgggagc caaggggcca 1260

cgaaagggcc tgggagggtc tgcagcttac ttgagtctct ttaattggtc tcatctaaag 1320

gcccagctta ttcattggca aacactgtga ccctgagcta ggctgctgtt gagagcaggc 1380

acggaacatt catctatctc atcttgagca atgcaagaaa catgggttca gagaggccaa 1440

ggactcaccg aggagtcaca gagtgtgggg gtgtcctctg aggcagctga gctggggcac 1500

acacagactg agcaccagga gtgagctcta gctttttttt ttctatgtgt cttttctaaa 1560

agacacatag gtttaggact gtccctggtc caggtaagaa ctggttcagt aaacttgtac 1620

atctcactgc ctggccagcc ctgtcagctt ccaccagagt gcgtgcacta cacacccggc 1680

atctcaaagg attcattcct atctttccta tctttggagt gaggcacagt ctcacgtagt 1740

ccagtccaga ctggccttaa attctgcagc tgaggatgta cttaaacttg tcatcctcct 1800

gccccagcct ctcaagtgct gtgatcacag gcacggacca ctatgctacg ccaggtgttt 1860

ccaaacattt tctctccctt aactggaagg tcaatgaggc tctttcgaga agcaacagag 1920

cctgtttagc tgagaaaact gaggcaggga gcaggcaa 1958

<210> 26
<211> 469
<212> DNA
<213> Mus musculus

<400> 26
tgagagatga ggtagggtgg cgcccatctc gagggcagct tgcgtgagca caggcagcct 60

tcttgccatt ggctgaggct gtcattgccg tcaccacttc gggtgggcac cggaagaaga 120

gtgaccttat tgccagcacc atttctcaaa cgttgtctaa ttcttttctc tagagcctga 180

atatcttcca gaatctcaac cgacgtcaac acgagcatgc gatccacatg atggacatcg 240

cgatcattgc cacagacctt gccttgtatt tcaagtgggt atttctcctc actttaatag 300

tagcagtgtg ggggctggag agatggttca gtggttaaca gcactgactg ctcttccaga 360

ggtcctgagt tcaaatccca gcaaccacat ggtggctcac aactatctgt aatgggatct 420

gataccctct tctggtgtgt gtctgaagac agcgatggag tactcacat 469

<210> 27
<211> 128
<212> DNA
<213> Homo sapiens

<400> 27
tagttagtaa actctttgac cctacatctc tacagatata aatgagtgtg agaccacaaa 60

cgagtgccgg gaggatgaaa tgtgttggaa ttatcatggc ggcttccgtt gttatccacg 120

aaatcctt 128

## Description

### BACKGROUND OF THE DISCLOSURE

The present disclosure relates to using CRISPR-based methods to perform gene editing in patients in order to treat autosomal dominant diseases.

There are currently no cures for numerous autosomal dominant or recessive diseases that have a profoundly negative impact on quality of life. Dominant forms of retinitis pigmentosa (adRP), cone-rod dystrophies and juvenile macular degenerations are prime examples of dominant autosomal diseases the affect the eye. These autosomal dominant diseases are characterized by the presence of a mutant gene expressing a defective protein. These diseases are not, therefore, readily amenable to therapies that simply add a normal, healthy gene (so called "gene supplementation" or "gene addition"), since the disease causing gene and protein are still present. Instead, gene editing offers the only means to directly repair the defective gene and, thus, the most promising therapeutic strategy.

Retinal degenerative diseases affects at least 9 million Americans (Friedman DS et al. Arch Ophthalmol. 2004 Apr;122(4):564-72; Schmier JK et al. Pharmacoeconomics. 2006;24(4):319-34). Among the most devastating retinal degenerative disease is retinitis pigmentosa (RP), a common form of inherited neurodegeneration, which affects 1.5 million people worldwide and for which treatment is inadequate. RP is a degenerative eye disease that results in retinal degeneration and vision loss. Hereditary mutations in the rhodopsin gene (*RHO*) are the most common cause of autosomal dominant RP, accounting for 20-30% of the cases. Currently, there is no cure for RP.

Gene therapy for RP was tested in proof-of-concept animal models, and later used as clinical treatment, where it improved vision in up-to half of the patients. In these trials, patients' genetic abnormalities were corrected by a gene supplementation approach (i.e., rescue via overexpression of a wild type (wt) gene). Initially, the gene supplementation appeared to work because patients experienced a functional rescue, but follow-up examination showed that degeneration of photoreceptors continued, and vision loss progressed in 3 years (Cideciyan et al. Proc Natl Acad Sci USA. 2013 Feb 5;110(6):E517-25; Bainbridge et al. N Engl J Med. 2015 May 14;372(20):1887-97; Jacobson et al. N Engl J Med. 2015 May 14;372(20):1920-6). Current gene therapy trials for other RP genes are also taking a gene supplementation approach and are likely to face similar hurdles unless the reasons for failure are addressed. Since supplementation with a wt gene leaves the patient's mutant gene intact, the presence of the mutant gene can continuously trigger ongoing damage despite the presence of a wt gene. The gene editing approach described in the present disclosure overcomes these and other obstacles to treatment of autosomal dominant as well as recessive diseases.

### Summary of the Disclosure

The method of the disclosure provides for treating an autosomal dominant ocular disease in a subject, comprising, administering to the subject a therapeutically effective amount of at least one type of recombinant adeno-associated viral (AAV) vector encoding a CRISPR-Cas enzyme system directed to an autosomal dominant disease-related gene, wherein at least one type of recombinant AAV vector comprises: (i) a first sequence (or first sequences) encoding at least one guide RNA that hybridizes to the endogenous autosomal dominant disease-related gene in the subject; (ii) a second sequence comprising a codon-modified autosomal dominant disease-related gene or fragment thereof, wherein at least one disease related mutation has been corrected in the codon-modified autosomal dominant disease-related gene or fragment thereof, and where the codon-modified autosomal dominant disease related gene or fragment is not recognized by the guide RNA; and, (iii) a third sequence encoding a Cas nuclease such as Cas9.

The endogenous autosomal dominant disease-related gene targeted by the present method may be wildtype and/or mutant.

A full-length or a fragment of a codon-modified autosomal dominant disease-related gene may be introduced into the subject in the present method.

In one embodiment, the two types of AAV vectors can be administered to the subject, where the first type of recombinant AAV vector comprises (i) the first sequence encoding at least one guide RNA and (ii) the second sequence comprising a codon-modified autosomal dominant disease-related gene or fragment thereof, and the second type of recombinant AAV vector comprises the third sequence, which encodes the Cas nuclease.

In one embodiment, the AAV vector(s) can encode two guide RNAs.

The ocular disease can include, but is not limited to, autosomal dominant chorioretinal atrophy or degeneration, autosomal dominant cone or cone-rod dystrophy, autosomal dominant congenital stationary night blindness, autosomal dominant leber congenital amaurosis, autosomal dominant macular degeneration, autosomal dominant ocular-retinal developmental disease, autosomal dominant optic atrophy, autosomal dominant retinitis pigmentosa, autosomal dominant syndromic/systemic diseases with retinopathy, sorsby macular dystrophy, age-related macular degeneration, doyne honeycomb macular disease, and juvenile macular degeneration.

In one embodiment, the ocular disease is retinitis pigmentosa. Retinitis pigmentosa can be caused by a mutation in *RHO* gene. The autosomal dominant disease-related gene may be the *RHO* gene. In another embodiment, the ocular disease is age-related macular degeneration. In a third embodiment, the ocular disease is doyne honeycomb. Doyne honeycomb may be caused by a mutation in the *EFEMP1* gene. The autosomal dominant disease-related gene may be the *EFEMP1* gene.

The recombinant AAV vector may be an AAV2 vector. Alternatively, the AAV vector is an AAV8 vector. Other suitable AAV vectors may also be used.

The Cas nuclease can be Cas9. The CRISPR-Cas system can be under the control of a promoter which controls the expression of the codon-modified autosomal dominant disease-related gene product in ocular cells.

The codon-modified autosomal dominant disease-related gene sequence or fragment thereof may be integrated into the endogenous autosomal disease-related gene. Alternatively, the codon-modified autosomal dominant disease-related gene sequence or fragment is not integrated into the endogenous autosomal disease-related gene, but is present episomally.

The (first) sequence encoding the guide RNA may be selected from the group consisting of, SEQ ID NO: 1, SEQ ID NO: 2, SEQ ID NO: 3, SEQ ID NO: 4, SEQ ID NO: 5, SEQ ID NO: 6, SEQ ID NO: 7, or any combinations thereof.

The autosomal dominant disease-related gene may include, but is not limited to, PRDM13, RGR, TEAD1, AIPL1, CRX, GUCA1A, GUCY2D, PITPNM3, PROM1, PRPH2, RIMS1, SEMA4A, UNC119, GNAT1, PDE6B, RHO, WSF1, IMPDH1, OTX2, BEST1, C1QTNF5, CTNNA1, EFEMP1, ELOVL4, FSCN2,GUCA1B, HMCN1, IMPG1, RP1L1, TIMP 3, VCAN, MFN2, NR2F1, OPA1, ARL3, CA4, HK1, KLHL7, NR2E3, NRL, PRPF3, PRPF4, PRPF6, PRPF8, PRPF31, RDH12, ROM1, RP1, RP9, RPE65, SNRNP200, SPP2, TOPORS, ABCC6, ATXN7, COL11A1, COL2A1, JAG1, KCNJ13, KIF11, OPA3, PAX2, TREX1, CAPN5, CRB1, FZD4, ITM2B, LRP5, MAPKAPK3, MIR204, OPN1SW, RB1, TSPAN12, and ZNF408.

The recombinant AAV vector(s) may be administered by injection into the eye.

The codon-modified autosomal dominant disease-related gene or fragment thereof can be selected from the group consisting of, SEQ ID NO: 8, SEQ ID NO: 9, SEQ ID NO: 23, SEQ ID NO: 24, SEQ ID NO: 25, SEQ ID NO: 26, SEQ ID NO: 27, or combinations thereof.

The methods of the disclosure also provide for treating an autosomal dominant ocular disease in a subject, comprising administering to the subject a therapeutically effective amount of: (a) a first recombinant adeno-associated viral (AAV) vector encoding a CRISPR-Cas system directed to an autosomal dominant disease-related gene, where at least one type of recombinant AAV vector comprises an AAV virus carrying a nucleic acid sequence encoding, (i) at least one guide RNA sequence that hybridizes to the autosomal dominant disease-related gene in the subject; (ii) a second codon-modified autosomal dominant disease-related gene or fragment thereof, wherein at least one disease related mutation has been corrected in the modified autosomal dominant disease-related gene or fragment and where the modified autosomal dominant disease related gene or fragment is not recognized by the guide RNA sequence; and, (b) a second recombinant AAV virus comprising a nucleic acid encoding a Cas nuclease, such as Cas9.

### BRIEF DESCRIPTION OF THE DRAWINGS

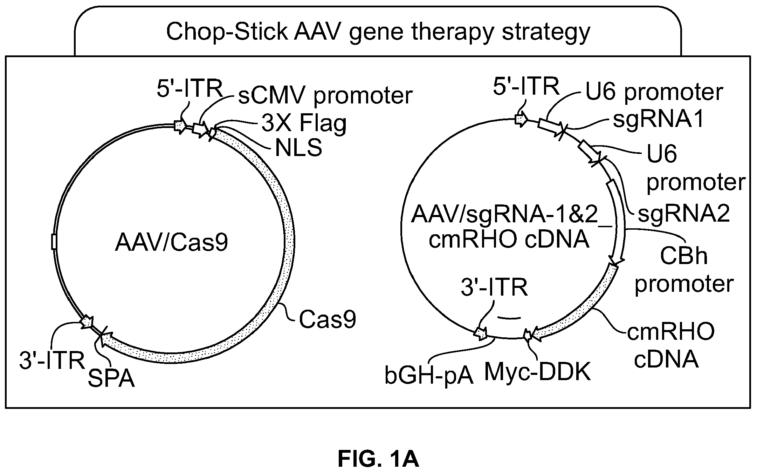

Figure 1A is schematic representations of the ChopStick AAV vectors. The left side shows a schematic representation of the AAV/Cas9 vector. Cas9 from *S. pyogenes* is driven by a 173-bp short CMV promoter (sCMV, SEQ ID NO: 14) and is terminated by a 50-bp synthetic poly-A signal (SPA) (SEQ ID NO: 19). The right side shows a schematic representation of the *RHO* sgRNAs and codon-modified cDNA (cm*RHO*, (SEQ ID NO: 9) expression vector. sgRNA1 and sgRNA2 are driven by U6 promoter (SEQ ID NO: 12). cm*RHO* cDNA with c-terminal tagged c-Myc is driven by CBh promoter (SEQ ID NO: 10) and terminated by bGH poly-A signal (SEQ ID NO: 11) Arrows indicate the direction of transcription. 5'-and 3'-ITR, inverted terminal repeats of AAV.

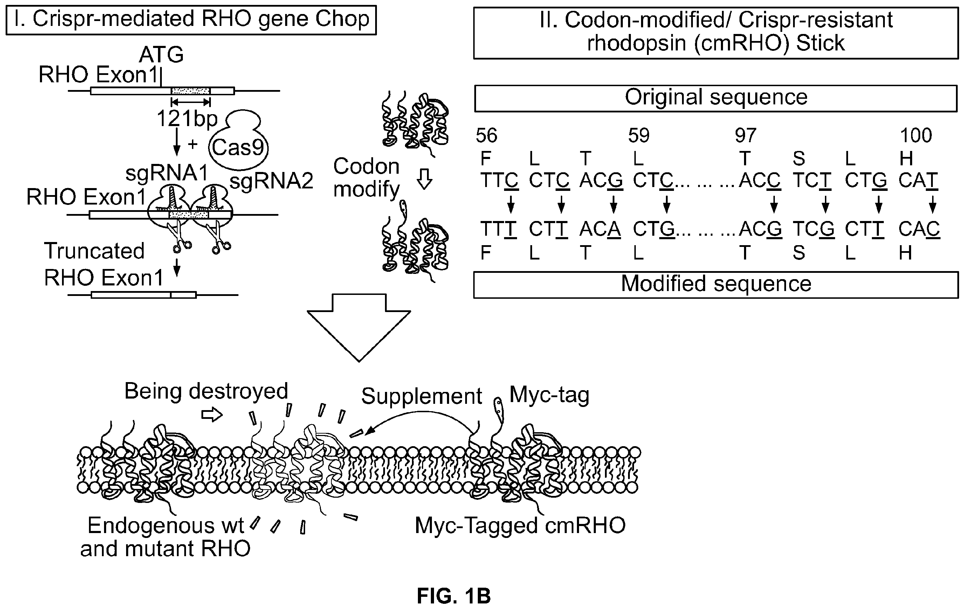

Figure 1B is a schematic representation of the ChopStick AAV gene therapy strategy. The left side schematic representation (I) shows that following co-infection of AAV/Cas9 and AAV/sgRNA1&2_cm*RHO*, the co-expression of Cas9 protein and two h*RHO* exon 1-specific sgRNAs, sgRNA1 and sgRNA2, will lead to a 121- bp deletion in the *host RHO* Exon1. The right side of the figure (II) shows the original and codon-modified rhodopsin sequence.

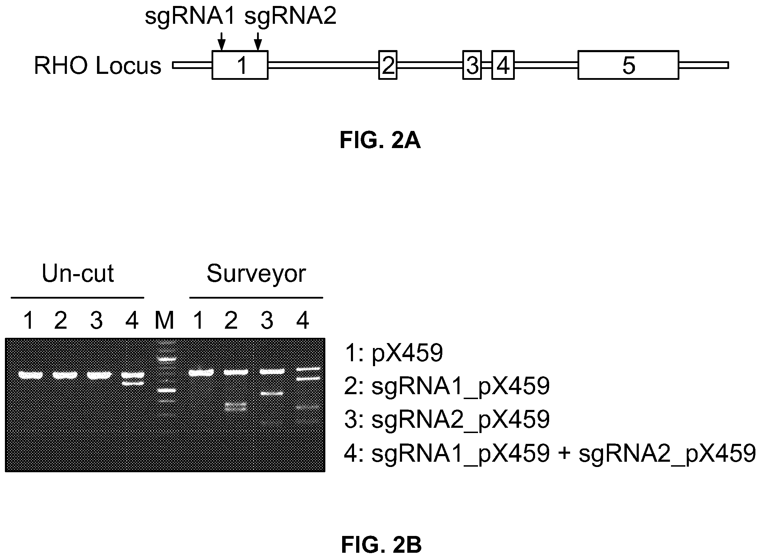

Figures 2A-B show that dual sgRNA provides more efficient "Chop" *of RHO* than single sgRNA. Figure 2A is a schematic representation of the target sites of sgRNA1 and sgRNA2 on RHO. The two sgRNAs both target *RHO* exon1, which is the beginning of the translation. Once the gene editing occurs, independent of whether one or two sgRNAs sites are targeted, most of the coding region will be affected. This design ensures the greatest disruption of gene expression and can be applied to many different types *of RHO* mutations. Figure 2B shows improved efficiency in truncating genes by the "Chop" strategy in human kidney cell line compared to using only one sgRNA. HEK293FT cells were transfected with Cas9 vector (pX459) carrying either no sgRNA, single sgRNA1, single sgRNA2, or both. Ninety-six hours later, DNA was extracted, and the RHO locus was amplified and analyzed by mismatch detection SURVEYOR assay. Applying two sgRNAs together resulted in gene deletion of approximately 30-40%, which indicated that "Chop" (gene deletion/disruption) strategy works efficiently in mammalian cells (lane 4). Using one sgRNA (lanes 2 and 3) at a time in contrast does not result in change in size of the RHO gene. Approximately 30% of the genomic DNA underwent non-homologous end joining (NHEJ) by one sgRNA. In contrast, up to 80% was edited (deletion and NHEJ) when two sgRNAs were used. AS a control, equal amounts of plasmid DNA (1 µg / 1 × 10⁵ 293FT cells) were used in each group.

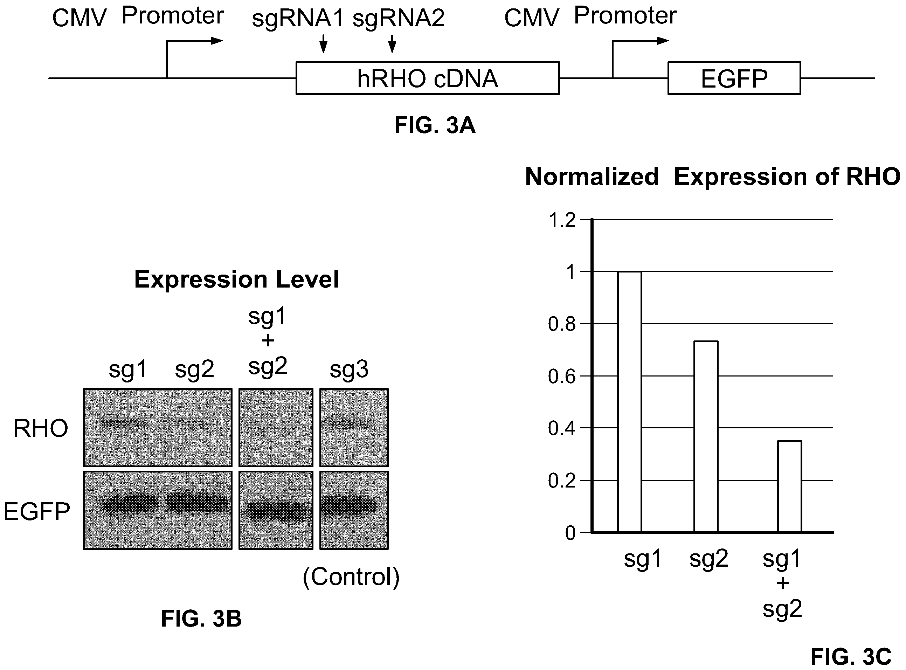

Figures 3A-C show improved efficacy of inactivating a gene by dual sgRNA ("Chop" or gene deletion/disruption) when compared with a single sgRNA. Figure 3A is schematic representation of the target sites of sgRNA1 and sgRNA2 on a *RHO* expression vector. The two sgRNAs target the 5' end *of RHO* cDNA as indicated. Wt *RHO* cDNA was driven by a CMV promoter. EGFP driven by CMV promoter was used as an internal control in immunoblot assay, which normalizes the difference in transfection efficiency and protein loading. Figure 3B shows protein levels as measured by immunoblot when the HEK293FT cells were co-transfected with *RHO* expression vector and another vector expressing Cas9 machinery (pX459) carrying either sgRNA1, sgRNA2, or both. The sg3 group is a non-specific control sgRNA. Figure 3C indicates that, after normalization with EGFP, two sgRNAs together lower *RHO* expression by 70%, while using single sgRNA reduced expression only by 0-30% (compared to the control group (sg3)). This result indicated that "Chop" strategy can be used to significantly reduce or inactivate protein expression.

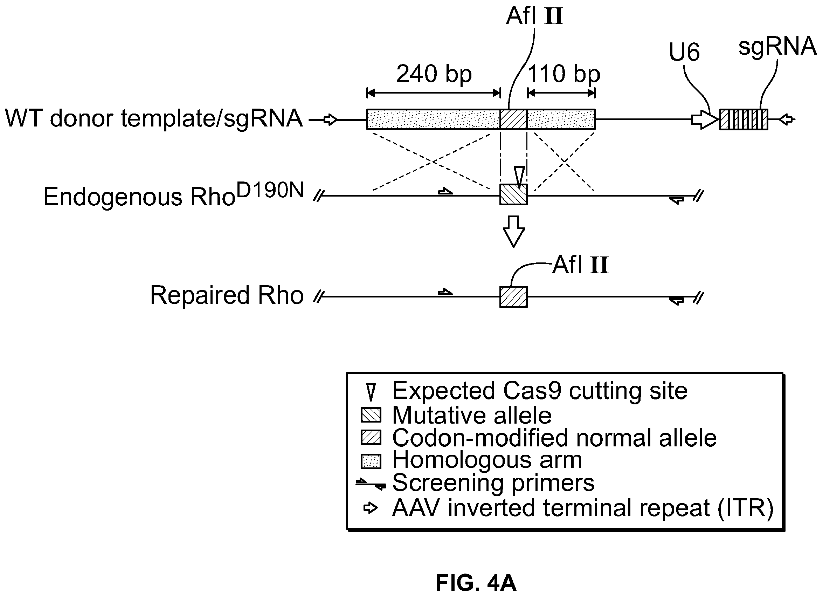

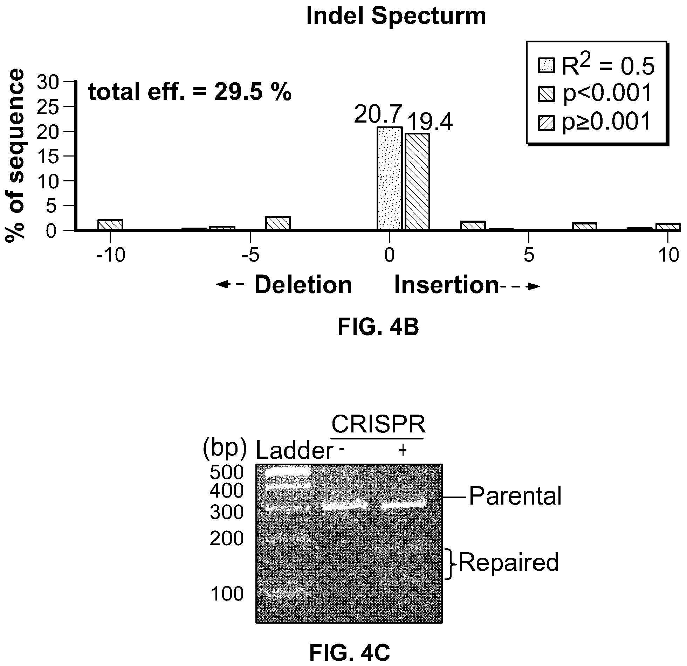

Figures 4A-C show that "Chop" (gene deletion or disruption strategy) has a potential to create a double strand break in order to facilitate precise repair through mechanism like homologous recombination. Figure 4A is schematic representation of the AAV-mediated CRISPR editing in *Rho*^{D190N} mouse RP model. Dual virus treatment of AAV/Cas9 vector and a bicistronic AAV vector containing wt donor template and an sgRNA targeting D190N mutation would result in mutation-specific repair. Donor template construct contains wild-type *Rho* sequence with two modifications: 1) creation of an additional *Afl*II site upstream of the D190 codon for the identification of DNA replacement following CRISPR-induced homologous recombination and 2) introduction of 5 wobble base pairs (bps) to render the donor template unrecognizable by sgRNA and thus, Cas9-resistant. Figure 4B shows editing efficiency evaluated using tracking of indels (insertions and deletions) by decomposition (TIDE) analysis (publically available at http://tide-calculator.nki.nl: retrieved April 30, 2016 ) in mouse retina DNA treated with aforementioned AAV viruses, which showed that ~50% of photoreceptors underwent NHEJ. Figure 4C is a representative *AflII* digestion of retinal DNA from a *Rho*^{D190N/+} mouse showing a large portion of photoreceptors being repaired through homologous recombination (lane 2). *Rho*^{D190N/+} mice were treated with the Cas9 vector with (lane 2) or without (lane 1) the wild-type donor template, and retinal DNA was extracted and amplified with indicated screening primers.

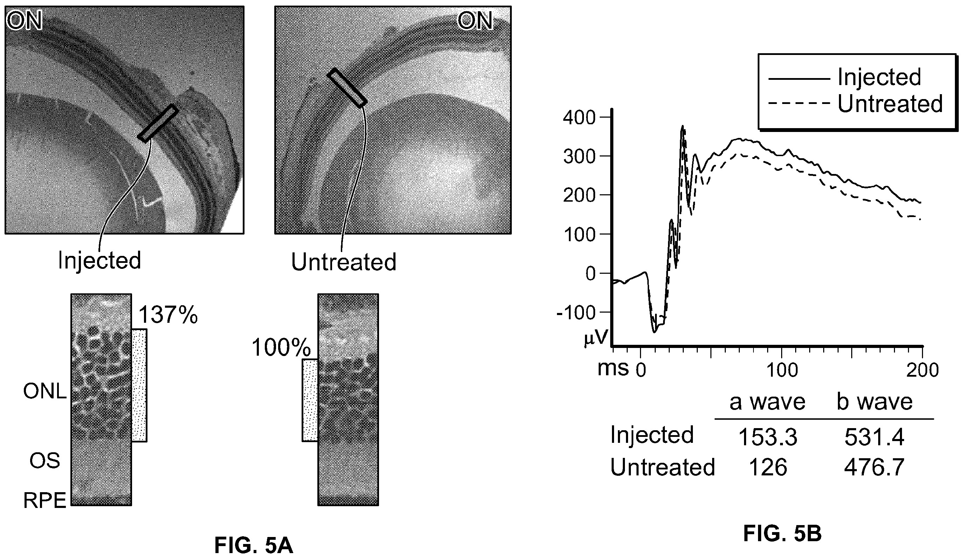

Figures 5A-B show the histological and functional rescue by CRISPR/donor template-mediated repair. *Rho*^{D190N/+} heterozygote mice were treated with dual virus treatment described in Figure 4A-C by subretinal injection at postnatal day 3. Figure 5B shows a visual function of mice evaluated by ERG following the treatment. Figure 5A shows a histological evaluation of the retinal tissue section. The H&E staining of retinal section shows the increase of photoreceptors (outernuclear layer, ONL) survival at 137%, as compared to the untreated eye (Figure 5A). The rectangular bars show an enlarged cross-sectional area of an ONL of photoreceptors in CRISPR/Cas9 (injected) and control eyes (untreated). The electroretinograms (ERGs) indicate a noticeable improvement in both a wave and b wave, of gene specific CRISPR-mediated therapy of 3-month old Rho^{D190N/+} heterozygote (Figure 5B).

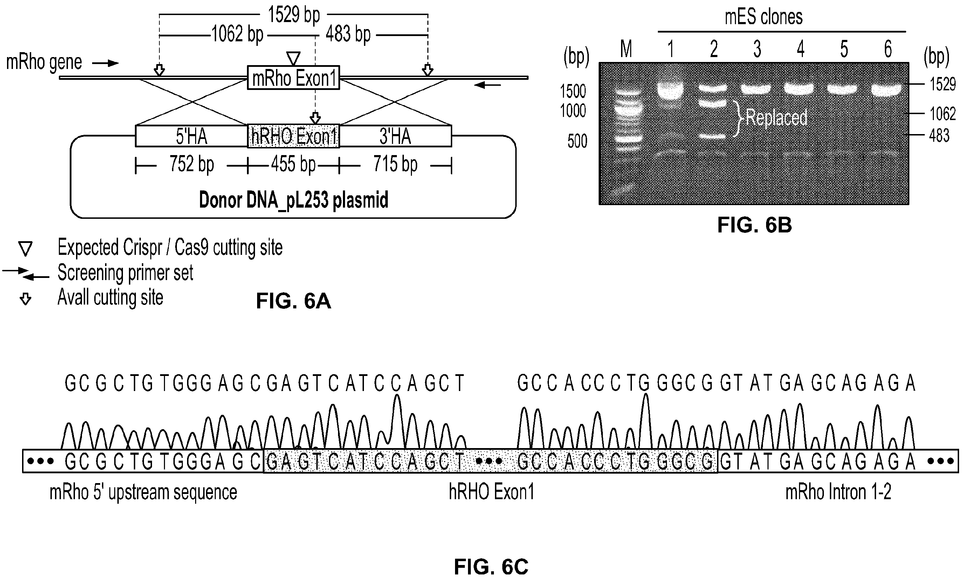

Figures 6A-C describe the generation of RHO-humanized animal model by CRISPR-mediated exon 1 replacement at the mouse *Rho* locus. This system enables the researchers to test CRISPR components *in vivo.* Figure 6A is an illustration of the strategy of replacing mouse (m) *Rho* exon 1 with either wild-type (wt) or mutant human (h) *RHO* exon 1. By co-electroporation of plasmid pX459 encoding Cas9 and *Rho* exon 1-specific sgRNA, a double strand break can be created in mouse exon1 that facilitates the homologous recombination in ES cells. Figure 6B shows restriction fragment length polymorphism (RFLP) assay results of ES cell DNA featuring additional *Ava*II site indicating the replacement of mouse *Rho* exon 1 with human *RHO* exon 1 (lane 1 and 2). Figure 6C: Sequence electropherogram of PCR amplicons reveals fusion of human and mouse sequence from one targeted ES clone.

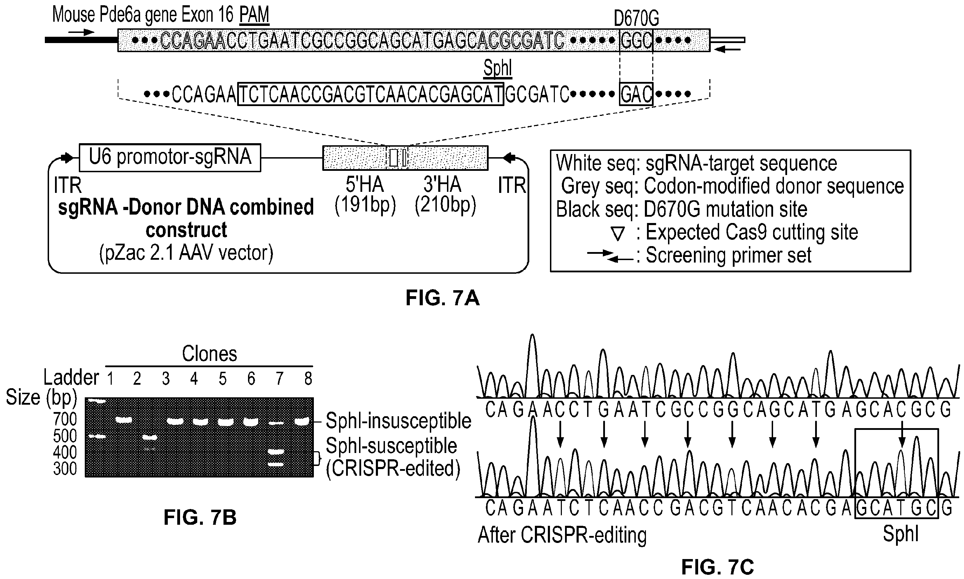

Figures 7A-C show the successful gene replacement of the D670G allele in the gene encoding *Pde6a* by CRISPR in mouse embryonic stem cells. Figure 7A is a schematic of donor construct which contains *Pde6a* with two changes: (1) a *Pde6a-*codon modification was introduced which creates an additional *SphI* site upstream from the D670G codon; and (2) eight wobble base pairs were introduced, making the donor template resistant to sgRNA targeting. Figure 7B shows PCR amplicons generated from ES cells that underwent homologous recombination. Figure 7C shows sequencing electropherogram data of target ES clone DNA, featuring an expected replacement of the D670G allele with donor template.

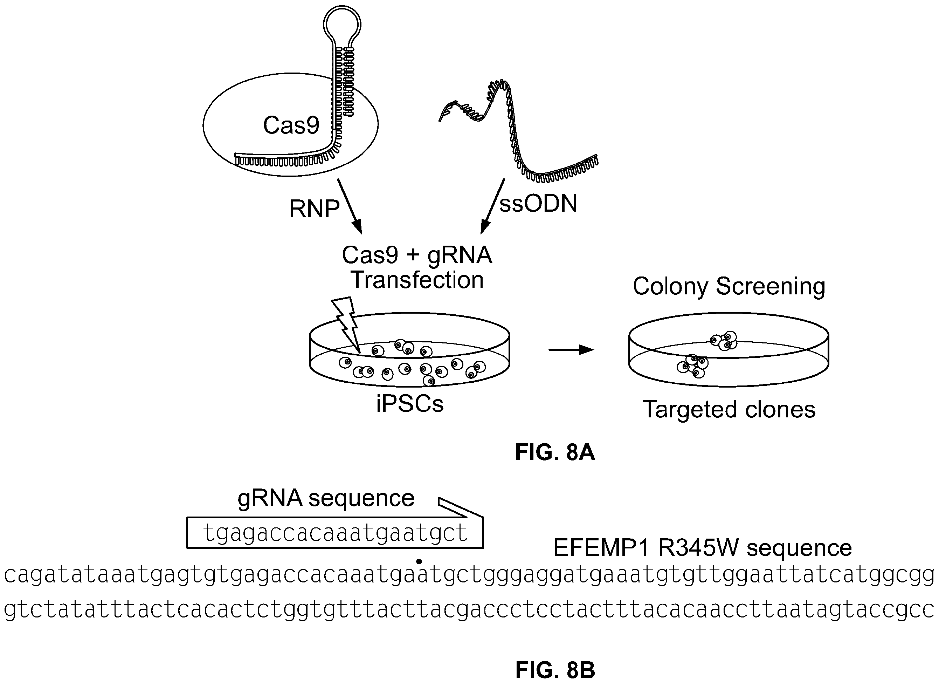

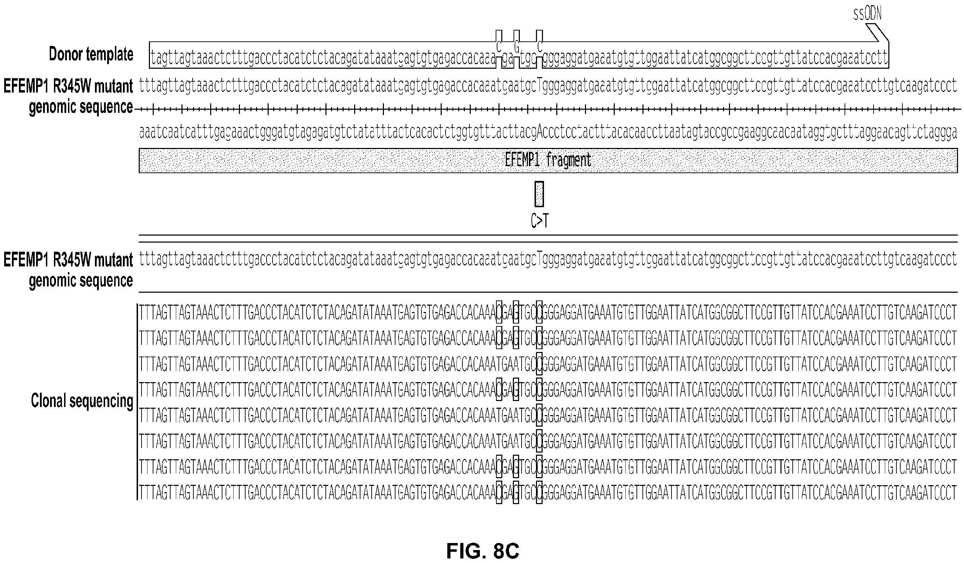

Figures 8A-C show that "ChopStick" (gene deletion or gene disruption) can be used to efficiently delete and correct a gene region of interest, such as one containing a mutation, in induced pluripotent stem (iPS) cell from a patient with juvenile macular degenerations (OMIM #126600). Figure 8A is a schematic illustration of the introduction of CRISPR components into human iPSCs. Cas9 protein/sgRNA complex (RNP) was co-nucleofected into human iPS cells with single strand donor template (ssODN). The clones were further selected and screened by restriction fragment length polymorphism (RFLP) assay. Figure 8B is a schematic representation of sgRNA targeting site in this case. The nucleotide marked with a dot corresponds to the mutation site. Figure 8C is the sequencing result of colony PCR, indicating replacement of donor template.

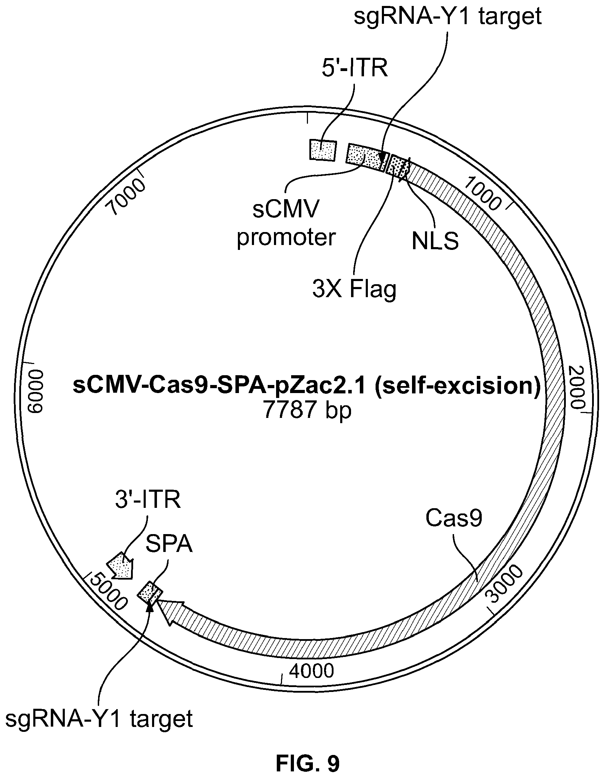

Figure 9 is a schematic representation of the self-excisional AAV/Cas9 vector. Cas9 from *S. pyogenes,* which is driven by a 173-bp short CMV promoter (sCMV) and terminated by a 50-bp synthetic poly-A signal (SPA), is flanked by sgRNA-Y1 (SEQ ID NO: 7) target sequences (GGTTTTGGACAATGGAACCGTGG, originated from Drosophila). Once the cell expresses Cas9 protein and sgRNA-Y1 simultaneously, this AAV/Cas9 vector is destroyed by Cas9 itself.

### DETAILED DESCRIPTION

The term "nuclease" is used to generally refer to any enzyme that hydrolyzes nucleic acid sequences.

The term "ocular cells" refers to any cell in, or associated with the function of, the eye. The term may refer to any one or more of photoreceptor cells, including rod, cone and photosensitive ganglion cells, retinal pigment epithelium (RPE) cells, Mueller cells, bipolar cells, horizontal cells, or amacrine cells. In one embodiment, the ocular cells are bipolar cells. In another embodiment, the ocular cells are horizontal cells. In yet a third embodiment, the ocular cells include ganglion cells.

The terms "polynucleotide", "nucleotide", "nucleotide sequence", "nucleic acid" and "oligonucleotide" are used interchangeably. These terms refer to a polymeric form of nucleotides of any length, either deoxyribonucleotides or ribonucleotides, or analogs. Examples of polynucleotides include, but are not limited to, coding or non-coding regions of a gene or gene fragment, exons, introns, messenger RNA (mRNA), transfer RNA, ribosomal RNA, short interfering RNA (siRNA), short-hairpin RNA (shRNA), micro-RNA (miRNA), ribozymes, cDNA, recombinant polynucleotides, branched polynucleotides, plasmids, vectors, isolated DNA of any sequence, isolated RNA of any sequence, nucleic acid probes, and primers. One or more nucleotides within a polynucleotide sequence can further be modified. The sequence of nucleotides may be interrupted by non-nucleotide components. A polynucleotide may also be modified after polymerization, such as by conjugation with a labeling agent.

The present disclosure is based, in part, on findings that gene editing can be used to correct the disease-causing mutant alleles, which in turn can be used for *in vivo* gene therapy for patients afflicted with autosomal dominant diseases.

The present disclosure takes advantage of the CRISPR gene-editing system, where the approach is to use a gene-editing enzyme with one or multiple unique single guide (sg) RNA sequences that target mutant allele(s) specifically or that target both the mutant and wild type alleles of a gene carrying an autosomal dominant mutation for destruction. This targeting is then followed by supplying the wild type gene cDNA, that is codon modified in order to evade recognition, by the sgRNA(s). Deletion of both the mutant and/or wild-type forms of the gene, followed by supplying the wild type gene cDNA that is codon modified and resistant to recognition by the guide RNAs results in the correction of the mutation, and thus, restoration of a phenotype found in the autosomal dominant diseases.

The inventors of the present disclosure refer to the gene-editing system described here as a "ChopStick" system. The "Chop" step involves partial or complete disruption of the i) mutant copy of a gene that is to be corrected; and/or ii) the wild-type copy of said gene in a patient afflicted with autosomal dominant disease. Thus, the "Chop" step results in partial or complete loss of mutant and/or wild-type activity of the said gene. The "Stick" step encompasses the introduction of a codon-modified cDNA of a gene of interest or fragment thereof (characterized by the autosomal dominant mutation), which is intended to restore, correct, supplement, or augment the gene or gene product function in the cells.

In one embodiment, the "Stick" step results in integration of a codon-modified donor template of a gene of interest or fragment (characterized by the autosomal dominant mutation) into the endogenous autosomal disease-related gene. Such targeted integration is accomplished by homologous recombination. In general, a Cas-family nuclease makes a DNA double-strand break at a defined site in the genome, which can then be repaired by homologous recombination or non-homologous end joining.

Alternatively, the "Stick" step does not result in integration of a codon-modified donor template of a gene of interest or fragment (characterized by the autosomal dominant mutation) into the endogenous autosomal disease-related gene. For example, extrachromosomal, or episomal (episomally), vectors persist in the nucleus in an extrachromosomal state, and offer transgene delivery and expression without integration into the host genome. Among such vectors are AAV vectors, which are particularly efficient in transduction of nondividing cells, and where the vector genome persists predominantly in an episomal form.

A codon-modified donor template can be delivered to cells or a patient via episomal vectors. Because episomal vectors persist in multiple copies per cell, the resulting expression of the gene of interest may be comparatively high at both the RNA as well as protein level. In non-dividing cells, the presence of the AAV vector as an episomal replicating element may be sufficient for stable expression of the gene, RNA, and/or protein.

Given the general principles of the "Chop-Stick" system outlined in the present disclosure, the "Chop-Stick" system can be used as a gene-editing tool for the correction of the mutation(s) found in any autosomal dominant disease. Thus, the methods of the present disclosure can be used to treat any autosomal dominant disease, including, but not limited to, Acropectoral syndrome, Acute intermittent porphyria, Adermatoglyphia, Albright's hereditary osteodystrophy, Arakawa's syndrome II, Aromatase excess syndrome, Autosomal dominant cerebellar ataxia, Autosomal dominant retinitis pigmentosa, Axenfeld syndrome, Bethlem myopathy, Birt-Hogg-Dubé syndrome, Boomerang dysplasia, Branchio-oto-renal syndrome, Buschke-Ollendorff syndrome, Camurati-Engelmann disease, Central core disease, Collagen disease, Collagenopathy, types II and XI, Congenital distal spinal muscular atrophy, Congenital stromal corneal dystrophy, Costello syndrome, Currarino syndrome, Darier's disease, De Vivo disease, Dentatorubral-pallidoluysian atrophy, Dermatopathia pigmentosa reticularis, DiGeorge syndrome, Doyne honeycomb disease, Dysfibrinogenemia, Familial amyloid polyneuropathy, Familial atrial fibrillation, Familial hypercholesterolemia, Familial male-limited precocious puberty, Feingold syndrome, Felty's syndrome, Flynn-Aird syndrome, Gardner's syndrome, Gillespie syndrome, Gray platelet syndrome, Greig cephalopolysyndactyly syndrome, Hajdu-Cheney syndrome, Hawkinsinuria, Hay-Wells syndrome, Hereditary elliptocytosis, Hereditary hemorrhagic telangiectasia, Hereditary mucoepithelial dysplasia, Hereditary spherocytosis, Holt-Oram syndrome, Huntington's disease, Hypertrophic cardiomyopathy, Hypoalphalipoproteinemia, Hypochondroplasia, Jackson-Weiss syndrome, Keratolytic winter erythema, Kniest dysplasia, Kostmann syndrome, Langer-Giedion syndrome, Larsen syndrome, Liddle's syndrome, Marfan syndrome, Marshall syndrome, Medullary cystic kidney disease, Metachondromatosis, Miller-Dieker syndrome, MOMO syndrome, Monilethrix, Multiple endocrine neoplasia, Multiple endocrine neoplasia type 1, Multiple endocrine neoplasia type 2, Multiple endocrine neoplasia type 2b, Myelokathexis, Myotonic dystrophy, Naegeli-Franceschetti-Jadassohn syndrome, Nail-patella syndrome, Noonan syndrome, Oculopharyngeal muscular dystrophy, Pachyonychia congenital, Pallister-Hall syndrome, PAPA syndrome, Papillorenal syndrome, Parastremmatic dwarfism, Pelger-Huet anomaly, Peutz-Jeghers syndrome, Polydactyly, Popliteal pterygium syndrome, Porphyria cutanea tarda, Pseudoachondroplasia, RASopathy, Reis-Bucklers corneal dystrophy, Romano-Ward syndrome, Rosselli-Gulienetti syndrome, Roussy-Lévy syndrome, Rubinstein-Taybi syndrome, Saethre-Chotzen syndrome, Schmitt Gillenwater Kelly syndrome, Short QT syndrome, Singleton Merten syndrome, Spinal muscular atrophy with lower extremity predominance, Spinocerebellar ataxia, Spinocerebellar ataxia type 6, Spondyloepiphyseal dysplasia congenital, Spondyloperipheral dysplasia, Stickler syndrome, Tietz syndrome, Timothy syndrome, Treacher Collins syndrome, Tuberous sclerosis, Upington disease, Variegate porphyria, Vitelliform macular dystrophy, Von Hippel-Lindau disease, Von Willebrand disease, Wallis-Zieff-Goldblatt syndrome, WHIM syndrome, White sponge nevus, Worth syndrome, Zaspopathy, Zimmermann-Laband syndrome, and Zori-Stalker-Williams syndrome. For example, in the Examples provided in the present disclosure, the inventors present the data performing "Chop" on human kidney cells and iPS cells (Figure 2 and Figure 8). These findings confirm the potential of the methods of the present disclosure to be used to prevent, correct, or treat autosomal dominant kidney diseases such as renal angiomyolipomas, medullary cystic kidney disease, or autosomal dominant polycystic kidney disease.

In further embodiments, the methods of the present disclosure can be used to prevent, correct, or treat ocular diseases that arise due to the presence of autosomal dominant mutation. Examples of such diseases include, but are not limited, autosomal dominant chorioretinal atrophy or degeneration, autosomal dominant cone or cone-rod dystrophy, autosomal dominant congenital stationary night blindness, autosomal dominant leber congenital amaurosis, autosomal dominant macular degeneration, autosomal dominant ocular-retinal developmental disease, autosomal dominant optic atrophy, autosomal dominant retinitis pigmentosa, autosomal dominant syndromic/systemic diseases with retinopathy, sorsby macular dystrophy, age-related macular degeneration, doyne honeycomb macular disease, and juvenile macular degeneration.

Generally, in the case of retinitis pigmentosa, patients with null rhodopsin mutations have a milder phenotype than those with severe dominant rhodopsin mutations. Even if the normal rhodopsin gene is destroyed together with the mutant one, the supply of the wild-type exon or cDNA (i.e., Stick) is still expected to improve retinal function in the recipient.

The methods of the present disclosure can be used for arresting progression of or ameliorating vision loss associated with retinitis pigmentosa (RP) in the subject. Vision loss linked to retinitis pigmentosa may include decrease in peripheral vision, central (reading) vision, night vision, day vision, loss of color perception, loss of contrast sensitivity, or reduction in visual acuity. The methods of the present disclosure can also be used to prevent, or arrest photoreceptor function loss, or increase photoreceptor function in the subject.

RP is diagnosed in part, through an examination of the retina. The eye exam usually reveals abnormal, dark pigment deposits that streak the retina. Additional tests for diagnosing RP include electroretinogram (ERG) and visual field testing.

Methods for measuring or assessing visual function, retinal function (such as responsiveness to light stimulation), or retinal structure in a subject are well known to one of skill in the art. See, e.g. Kanski's Clinical Ophthalmology: A Systematic Approach, Edition 8, Elsevier Health Sciences, 2015. Methods for measuring or assessing retinal response to light include may include detecting an electrical response of the retina to a light stimulus. This response can be detected by measuring an electroretinogram (ERG; for example full-field ERG, multifocal ERG, or ERG photostress test), visual evoked potential, or optokinetic nystagmus (see, e.g., Wester et al., Invest. Ophthalmol. Vis. Sci. 48:4542-4548, 2007). Furthermore, retinal response to light may be measured by directly detecting retinal response (for example by use of a microelectrode at the retinal surface). ERG has been extensively described by Vincent et al. Retina, 2013 Jan;33(1):5-12. Thus, methods of the present disclosure can be used to improve visual function, retinal function (such as responsiveness to light stimulation), retinal structure, or any other clinical symptoms or phenotypic changes associated with ocular diseases in subjects afflicted with ocular disease.

In one embodiment, the methods of the present disclosure can be used to prevent the development and progression of autosomal dominant disease. For example, a patient may be a carrier of autosomal dominant mutation, but the phenotypic expression of a disease has not been yet manifested, although the genomic defect has been identified by screening. The methods of the present disclosure may be applied to such patient to prevent the onset of disease.

Mutations in various genes have been identified to give rise to autosomal dominant diseases (such genes are also referred to as autosomal dominant disease-related genes). The methods of the present disclosure can be used to fully or partially correct mutations in such autosomal dominant disease-related genes, resulting in partial or full restoration of wild type.

In all cases where accession numbers are used, the accession numbers refer to one embodiment of the gene which may be used with the methods of the present disclosure. In one embodiment, the accession numbers are NCBI (National Center for Biotechnology Information) reference sequence (RefSeq) numbers.

For example, the autosomal dominant disease-related gene in retinitis pigmentosa may include, but are not limited to, ARL3(NC_000010.11 (102673727..102714433, complement)), BEST1(NG_009033.1), CA4(NG_012050.1), CRX(NG_008605.1), FSCN2(NG_ 015964.1), GUCA1B(NG_016216.1), HK1(NG_012077.1), IMPDH1(NG_009194.1),KLHL7(N G_016983.1), NR2E3(NG_009113.2), NRL(NG_011697.1), PRPF3(NG_008245.1), PRPF4(NG _034225.1), PRPF6(NG_029719.1), PRPF8(NG_009118.1), PRPF31(NG_009759.1),PRPH2(N G_009176.1), RDH12(NG_008321.1), RHO(NG_009115.1), ROM1(NG_009845.1), RP1(NG_0 09840.1), RP9(NG_012968.1), RPE65(NG_008472.1), SEMA4A(NG_027683.1), SNRNP200(NG_016973.1), SPP2(NG_008668.1), and TOPORS(NG_017050.1). Genes and mutations causing autosomal dominant retinitis pigmentosa are in detail discussed by Daiger et al. (Cold Spring Harb Perspect Med. 2014 Oct 10;5(10)).

Another type of the autosomal dominant disease- related gene is autosomal dominant chorioretinal atrophy or degeneration-related gene, which may include:
PRDM13(NC_000006.12 (99606774..99615578)), RGR(NG_009106.1), and TEAD1(NG_021302.1).

Another example of the autosomal dominant disease- related gene is autosomal dominant cone or cone-rod dystrophy-related gene, which can include: AIPL1(NG_008474.1), CRX(NG_008605.1), GUCA1A(NG_009938.1), GUCY2D(NG_009092.1), PITPNM3(NG_016 020.1), PROM1(NG_011696.1), PRPH2(NG_009176.1),RIMS1(NG_016209.1), SEMA4A(NG_ 027683.1), and UNC119(NG_012302.1). ).

In one embodiment, the autosomal dominant disease- related gene is autosomal dominant congenital stationary night blindness-related gene, including:
GNAT1(NG_009831.1), PDE6B(NG_009839.1), and RHO(NG_009115.1).

In another embodiment, the autosomal dominant disease- related gene is autosomal dominant deafness (alone or syndromic)-related gene such as WSF1(NC_000004.12 (6269850..6303265)).

Another type of the autosomal dominant disease- related gene is autosomal dominant leber congenital amaurosis-related gene, which may include: CRX(NG_008605.1), (NG_009194.1), and OTX2(NG_008204.1).

Another example of the autosomal dominant disease- related gene is autosomal dominant macular degeneration-related gene, which can include: BEST1(NG_009033.1), C1QTNF5(NG_012235.1), CTNNA1(NC_000005.10 (138753396..138935034)), *EFEMP1* (NG_009098.1), ELOVL4(NG_009108.1), FSCN2(NG_015964.1),GUCA1B(NG_016 216.1), HMCN1(NG_011841.1), IMPG1(NG_041812.1), OTX2(NG_008204.1), PRDM13(NC_ 000006.12 (99606774..99615578)), PROM1(NG_011696.1), PRPH2(NG_009176.1), RP1L1(NG_028035.1, and TIMP3(NG_009117.1).

In one embodiment, the autosomal dominant disease- related gene is autosomal dominant ocular retinal developmental disease-related gene such as VCAN(NG_012682.1). The accession numbers are provided as specific examples of each gene which may be used with the methods of the disclosure.

In another embodiment, the autosomal dominant disease-related gene is autosomal dominant optic atrophy-related gene, including: MFN2(NG_007945.1), NR2F1(NG_034119.1), and OPA1(NG_011605.1).

In one embodiment, the autosomal dominant disease-related gene is autosomal dominant syndromic/systemic disease with retinopathy-related gene, including: ABCC6(NG_007558.2), ATXN7(NG_008227.1), COL11A1(NG_008033.1), COL2A1(NG_008 072.1), JAG1(NG_007496.1), KCNJ13(NG_016742.1), KIF11(NG_032580.1), MFN2(NG_007945.1), OPA3(NG_013332.1), PAX2(NG_008680.2), TREX1(NG_009820.1), and VCAN(NG_012682.1).

Another example of the autosomal dominant disease-related gene is autosomal dominant retinopathy-related gene, including: BEST1(NG_009033.1), CAPN5(NG_033002.1), CRB1(NG_008483.2), FZD4(NG_011752.1), ITM2B(NG_013069.1), LRP5(NG_015835.1), M APKAPK3(NC_000003.12(50611862.. 50649297)),MIR204(NR_029621.1), OPN1SW(NG_009 094.1), RB1(NG_009009.1), TSPAN12(NG_023203.1), and ZNF408(NC_000011.10 (46700767..46705916).

In addition to being used for the prevention, correctness, or treatment of autosomal dominant diseases, the methods of the present disclosure can be used to prevent, correct, or treat any autosomal recessive diseases. Thus, all the methods described here as applicable to autosomal dominant diseases and autosomal dominant genes or fragments can be adopted for use in the treatment of autosomal recessive diseases.

In further embodiments, the methods of the present disclosure can be used to prevent, correct, or treat ocular diseases that arise due to the presence of autosomal recessive mutation. Examples of such diseases include, but are not limited to, autosomal recessive congenital stationary night, autosomal recessive deafness alone or syndromic, autosomal recessive leber congenital amaurosis, autosomal recessive optic atrophy, autosomal recessive retinitis pigmentosa, autosomal recessive syndromic/systemic diseases with retinopathy, autosomal recessive usher syndrome, other autosomal recessive retinopathy, autosomal recessive cone or cone-rod dystrophy, autosomal recessive macular degeneration, and autosomal recessive bardet-biedl syndrome.

According to the methods described here, autosomal recessive disease- related gene is corrected and can in-part or fully restore the function of a wild-type gene.

One type of the autosomal recessive disease- related gene is congenital stationary night -related gene, including: CABP4(NG_021211.1), GNAT1(NG_009831.1), GNB3(NG_009100.1), GPR179(NG_032655.2), GRK1(NC_000013.11(113667279..113671659 )), GRM6(NG_008105.1), LRIT3(NG_033249.1), RDH5(NG_008606.1),SAG(NG_009116.1), SLC24A1(NG_031968.2), and TRPM1(NG_016453.2).

Another type of the autosomal recessive disease- related gene is bardet-biedl syndrome-related gene, including:
ADIPOR1(NC_000001.1(202940825..202958572,complement)), ARL6(NG_008119.2), BBIP1( NG_041778.1), BBS1(NG_009093.1), BBS2(NG_009312.1), BBS4(NG_009416.2), BBS5(NG_ 011567.1), BBS7(NG_009111.1), BBS9(NG_009306.1),BBS10(NG_016357.1), BBS12(NG_02 1203.1), C8orf37(NG_032804.1), CEP290(NG_008417.1), IFT172(NG_034068.1), IFT27(NG_ 034205.1), INPP5E(NG_016126.1), KCNJ13(NG_016742.1),LZTFL1(NG_033917.1), MKKS( NG_009109.1), MKS1(NG_013032.1), NPHP1(NG_008287.1), SDCCAG8(NG_027811.1), TRI M32(NG_011619.1), and TTC8(NG_008126.1).

One example of the autosomal recessive disease-related gene is cone or cone-rod dystrophy- related gene, including, but not limited to, ABCA4(NG_009073.1), ADAM9(NG_016335.1), ATF6(NG_029773.1), C21orf2(NG_032952.1), C8orf37(NG_032804. 1), CACNA2D4(NG_012663.1), CDHR1(NG_028034.1),CERKL(NG_021178.1), CNGA3(NG _009097.1), CNGB3(NG_016980.1), CNNM4(NG_016608.1), GNAT2(NG_009099.1), KCNV 2(NG_012181.1), PDE6C(NG_016752.1),PDE6H(NG_016859.1), POC1B(NG_041783.1), RA B28(NG_033891.1), RAX2(NG_011565.1), RDH5(NG_008606.1), RPGRIP1(NG_008933.1), a nd TTLL5(NG_016974.1).

Another example of the autosomal recessive disease-related gene is deafness (alone or syndromic)-related gene including: CDH23(NG_008835.1), CIB2(NG_033006.1), DFNB31(NG_016700.1), MYO7A(NG_009086.1), PCDH15(NG_009191.2), PDZD7(NG_0280 30.1), and USH1C(NG_011883.1). ).

In one embodiment, the autosomal recessive disease-related gene is leber congenital amaurosis-related gene, including: AIPL1(NG_008474.1), CABP4(NG_021211.1), CEP290(NG_008417.1), CLUAP1(NC_000016.10(3500945..3539048)), CRB1(NG_008483.2), CRX(NG_008605.1), DTHD1(NG_032962.1), GDF6(NG_008981.1), GUCY2D(NG_009092.1), IFT140(NG_032783.1), IQCB1(NG_015887.1), KCNJ13(NG_01674 2.1), LCA5(NG_016011.1), LRAT(NG_009110.1), NMNAT1(NG_032954.1),PRPH2(NG_0091 76.1), RD3(NG_013042.1), RDH12(NG_008321.1), RPE65(NG_008472.1), RPGRIP1(NG_008 933.1), SPATA7(NG_021183.1), and TULP1(NG_009077.1).

In another embodiment, the autosomal recessive disease- related gene is optic atrophy-related gene, including: RTN4IP1(NC_000006.12 (106571028..106630500, complement)), SLC25A46(NC_000005.10(110738136..110765161)), and TMEM126A(NG_017157.1).

One example of the autosomal recessive disease- related gene is retinitis pigmentosa-related gene, including: ABCA4(NG_009073.1), AGBL5(NC_000002.12 (27051423..27070622)), ARL6(NG_008119.2), ARL2BP(NG_033905.1), BBS1(NG_009093.1), BBS2(NG_009312.1), BEST1(NG_009033.1), C2orf71(NG_021427.1),C8orf37(NG_032804.1) , CERKL(NG_021178.1), CLRN1(NG_009168.1), CNGA1(NG_009193.1), CNGB1(NG_01635 1.1), CRB1(NG_008483.2), CYP4V2(NG_007965.1),DHDDS(NG_029786.1), DHX38(NG_034 207.1), EMC1(NG_032948.1), EYS(NG_023443.2), FAM161A(NG_028125.1), GPR125(NC_0 00004.12 (22387374..22516058, complement)), HGSNAT(NG_009552.1), IDH3B(NG_012149.1), IFT140(NG_032783.1), IFT172(NG_034068.1), IMPG2(NG_028284.1) , KIAA1549(NG_032965.1), KIZ(NG_033122.1), LRAT(NG_009110.1), MAK(NG_030040.1), MERTK(NG_011607.1), MVK(NG_007702.1), NEK2(NG_029112.1), NEUROD1(NG_011820 .1), NR2E3(NG_009113.2), NRL(NG_011697.1), PDE6A(NG_009102.1), PDE6B(NG_009839. 1),PDE6G(NG_009834.1), POMGNT1(NG_009205.2), PRCD(NG_016702.1), PROM1(NG_01 1696.1), RBP3(NG_029718.1), RGR(NG_009106.1), RHO(NG_009115.1), RLBP1(NG_ 008116 .1),RPI(NG_009840.1), RP1L1(NG_028035.1), RPE65(NG_008472.1), SAG(NG_009116.1), S LC7A14(NG_034121.1), SPATA7(NG_021183.1), TTC8(NG_008126.1), TULP1(NG_009077. 1), USH2A(NG_009497.1), ZNF408(NC_000011.10 (46700767..46705916)), and ZNF513(NG_028219.1).

Another example of the autosomal recessive disease- related gene is syndromic/systemic disease with retinopathy- related gene, including: ABCC6(NG_007558.2), ABHD12(NG_028119.1), ACBD5(NG_032960.2), ADAMTS18(NG_031879.1), ADIPOR1(NC _000001.11(202940825..202958572, complement)), AHI1(NG_008643.1), ALMS1(NG_011690.1),CC2D2A(NG_013035.1), CEP164(NG_033032.1), CEP290(NG_00841 7.1), CLN3(NG_008654.2), COL9A1(NG_011654.1), CSPP1(NG_034100.1), ELOVL4(NG_00 9108.1), EXOSC2(NC_000009.12 (130693760..130704894)), FLVCR1(NG_028131.1), FLVCR1(NG_028131.1), GNPTG(NG_016985.1), HARS(NG_032158.1), HGSNAT(NG_0095 52.1), HMX1(NG_013062.2), IFT140(NG_032783.1),INPP5E(NG_016126.1), INVS(NG_0083 16.1), IQCB1(NG_015887.1), LAMA1(NG_034251.1), LRP5(NG_015835.1), MKS1(NG_0130 32.1), MTTP(NG_011469.1), NPHP1(NG_008287.1),NPHP3(NG_008130.1), NPHP4(NG_011 724.2), OPA3(NG_013332.1), PANK2(NG_008131.3), PCYT1A(NG_042817.1), PEX1(NG_00 8341.1), PEX2(NG_008371.1), PEX7(NG_008462.1),PHYH(NG_012862.1), PLK4(NG_04182 1.1), PNPLA6(NG_013374.1), POC1B(NG_041783.1), PRPS1(NG_008407.1), RDH11(NG_04 2282.1), RPGRIP1L(NG_008991.2),SDCCAG8(NG_027811.1), SLC25A46(NC_000005.10(11 0738136..110765161)), TMEM237(NG_032049.1), TRNT1(NG_041800.1), TTPA(NG_016123 .1), TUB(NG_029912.1),TUBGCP4(NG_042168.1), TUBGCP6(NG_032160.1), WDPCP(NG_ 028144.1), WDR19(NG_031813.1), WFS1(NG_011700.1), and ZNF423(NG_032972.2).

One type of the autosomal recessive disease- related gene is usher syndrome- related gene, including: ABHD12(NG_028119.1), CDH23(NG_008835.1), CEP250(NC_000020.11(35455139..35517531)), CIB2(NG_033006.1), CLRN1(NG_009168.1), DFNB31(NG_016700.1), GPR98(NG_007083.1), HARS(NG_032158.1),MYO7A(NG_009086. 1), PCDH15(NG_009191.2), USH1C(NG_011883.1), USH1G(NG_007882.1), and USH2A(NG_009497.1).

Another type of the autosomal recessive disease- related gene is retinopathy- related gene, including: BEST1(NG_009033.1), C12orf65(NG_027517.1), CDH3(NG_009096.1), CNGA3(NG_009097.1), CNGB3(NG_016980.1), CNNM4(NG_016608.1), CYP4V2(NG_0079 65.1), LRP5(NG_015835.1), MFRP(NG_012235.1), MVK(NG_007702.1), NBAS(NG_032964.1), NR2E3(NG_009113.2), OAT(NG_008861.1), PLA2G5(NG_032045.1), PROM1(NG_011696.1), RBP4(NG_009104.1), RGS9(NG_013021.1), RGS9BP(NG_016751.1), and RLBP1(NG_008116.1).

Yet another type of the autosomal recessive disease- related gene is macular degeneration-related gene, including: ABCA4(NG_009073.1), CFH(NG_007259.1), DRAM2(NC_000001.11(111117332..111140216, complement)), IMPG1(NG_041812.1), and MFSD8(NG_008657.1).

In addition to being used for the prevention, correctness, or treatment of autosomal dominant and recessive diseases, the methods of the present disclosure can be used to prevent, correct, or treat any X-linked diseases. Thus, all the methods described here as applicable to autosomal dominant diseases and autosomal dominant genes or fragments can be adopted for use in the treatment of X-linked diseases.

Furthermore, the methods of the present disclosure can be used to prevent, correct, or treat ocular diseases that arise due to the presence of X-linked mutation. Examples of such diseases include: X-linked cone or cone-rod dystrophy, X-linked congenital stationary night blindness, X-linked macular degeneration, X-linked retinitis pigmentosa, X-linked syndromic/systemic diseases with retinopathy, X-linked optic atrophy, and X-linked retinopathies. According to the methods described here, X-linked disease- related gene is corrected and can in part or fully restore the function of a wild-type gene.

One example of the X-linked disease-related gene is cone or cone-rod dystrophy-related gene, including: CACNA1F(NG_009095.2) and RPGR(NG_009553.1).

Another example of the X-linked disease-related gene is congenital stationary night blindness-related gene, including: CACNA1F(NG_009095.2) and NYX(NG_009112.1).

In one embodiment, the X-linked disease-related gene is macular degeneration-related gene, such as RPGR(NG_009553.1).

In another embodiment, the X-linked disease-related gene is optic atrophy-related gene, such as TIMM8A(NG_011734.1).

One type of the X-linked disease-related gene is retinitis pigmentosa-related gene, including: OFD1(NG_008872.1), RP2(NG_009107.1), and RPGR(NG_009553.1).

Another type of the X-linked disease-related gene is syndromic/systemic disease with retinopathy-related gene, including: OFD1(NG_008872.1) and TIMM8A(NG_011734.1).

Yet another example of the X-linked disease-related gene is retinopathy-related gene., including:CACNA1F(NG_009095.2), CHM(NG_009874.2), DMD(NG_012232.1), NDP(NG_0 09832.1), OPN1LW(NG_009105.2), OPN1MW(NG_011606.1), PGK1(NG_008862.1), and RS1(NG_008659.3).

In another embodiment, the methods of the present disclosure can be used to prevent, correct, or treat diseases that arise due to the presence of mutation in mitochondrial DNA. Such diseases may include, retinopathy caused by the gene mutations in mitochondrial DNA. Examples of genes that may be characterized by the mutation in mitochondrial DNA that causes the development of retinopathy include: MT-ATP6(NC_012920.1 (8527..9207)), MT-TH(NC_012920.1 (12138..12206)), MT-TL1(NC_012920.1 (3230..3304)), MT-TP(NC_012920.1 (15956..16023, complement), and MT-TS2(NC_012920.1 (12207..12265)).

Table 1 provides an exemplary list of diseases and disease-related genes (accompanied with corresponding accession numbers) that can be treated and/or corrected using methods of the present disclosure.

**Table 1: Disease Related Disorders and Genes**

| **Disease Category** | **Mapped and Identified Genes** |
|---|---|
| Bardet-Biedl syndrome, autosomal recessive | ADIPOR1(NC_000001.11 (202940825..202958572, complement)), ARL6(NG_008119.2), BBIP1(NG_041778.1), BBS1( NG_009093.1), BBS2(NG_009312.1), BBS4(NG_009416.2), BBS5( NG_011567.1), BBS7(NG_009111.1), BBS9(NG_009306.1),BBS10( NG_016357.1), BBS12(NG_021203.1), C8orf37(NG_032804.1), CE P290(NG_008417.1), IFT172(NG_034068.1), IFT27(NG_034205.1), INPP5E(NG_016126.1), KCNJ13(NG_016742.1),LZTFL1(NG_0339 17.1), MKKS(NG_009109.1), MKS1(NG_013032.1), NPHP1(NG_0 08287.1), SDCCAG8(NG_027811.1), TRIM32(NG_011619.1), TTC 8(NG_008126.1) |
| Chorioretinal atrophy or degeneration, autosomal dominant | PRDM13(NC_000006.12 (99606774..99615578)), RGR(NG_009106.1), TEAD1(NG_021302.1 ) |
| Cone or cone-rod dystrophy, autosomal dominant | AIPL1(NG_008474.1), CRX(NG_008605.1), GUCA1A(NG_009938. 1), GUCY2D(NG_009092.1), PITPNM3(NG_016020.1), PROM1(N G_011696.1), PRPH2(NG_009176.1),RIMS1(NG_016209.1), SEMA 4A(NG_027683.1),UNC119(NG_012302.1) |
| Cone or cone-rod dystrophy, autosomal recessive | ABCA4(NG_009073.1), ADAM9(NG_016335.1), ATF6(NG_02977 3.1), C21orf2(NG_032952.1), C8orf37(NG_032804.1), CACNA2D4( NG_012663.1), CDHR1(NG_028034.1),CERKL(NG_021178.1), CN GA3(NG_009097.1), CNGB3(NG_016980.1), CNNM4(NG_016608. 1), GNAT2(NG_009099.1), KCNV2(NG_012181.1), PDE6C(NG_01 6752.1),PDE6H(NG_016859.1), POC1B(NG_041783.1), RAB28(NG _033891.1), RAX2(NG_011565.1), RDH5(NG_008606.1), RPGRIP1 (NG_008933.1), TTLL5(NG_016974.1) |
| Cone or cone-rod dystrophy, X-linked | CACNA1F(NG_009095.2), RPGR(NG_009553.1) |
| Congenital stationary night blindness, autosomal dominant | GNAT1(NG_009831.1), PDE6B(NG_009839.1), RHO(NG_009115. 1) |
| Congenital stationary night blindness, autosomal recessive | CABP4(NG_021211.1), GNAT1(NG_009831.1), GNB3(NG_009100 .1), GPR179(NG_032655.2), GRK1(NC_000013.11 (113667279..113671659)), GRM6(NG_008105.1), LRIT3(NG_03324 9.1), RDH5(NG_008606.1),SAG(NG_009116.1), SLC24A1(NG_031 968.2), TRPM1(NG_016453.2) |
| Congenital stationary night blindness, X-linked | CACNA1F(NG_009095.2), NYX(NG_009112.1) |
| Deafness alone or syndromic, autosomal dominant | WSF1(NC_000004.12 (6269850..6303265)) |
| Deafness alone or syndromic, autosomal recessive | CDH23(NG_008835.1), CIB2(NG_033006.1), DFNB31(NG_016700 .1), MYO7A(NG_009086.1), PCDH15(NG_009191.2), PDZD7(NG_ 028030.1), USH1C(NG_011883.1) |
| Leber congenital amaurosis, autosomal dominant | CRX(NG_008605.1), IMPDH1(NG_009194.1), OTX2(NG 008204.1 ) |
| Leber congenital amaurosis, autosomal recessive | AIPL1(NG_008474.1), CABP4(NG_021211.1), CEP290(NG_008417 .1), CLUAP1(NC_000016.10 (3500945..3539048)), CRB1(NG_008483.2), CRX(NG_008605.1), D THD1(NG_032962.1), GDF6(NG_008981.1),GUCY2D(NG_009092. 1), IFT140(NG_032783.1), IQCB1(NG_015887.1), KCNJ13(NG_01 6742.1), LCA5(NG 016011.1), LRAT(NG_009110.1), NMNAT1(N G_032954.1),PRPH2(NG_009176.1), RD3(NG_013042.1), RDH12( NG_008321.1), RPE65(NG_008472.1), RPGRIP1(NG_008933.1), S PATA7(NG_021183.1), TULP1(NG_009077.1) |
| Macular degeneration, autosomal dominant | BEST1(NG_009033.1), C1QTNF5(NG_012235.1), CTNNA1(NC_00 0005.10 (138753396..138935034)), EFEMP1(NG_009098.1), ELOVL4(NG_0 09108.1), FSCN2(NG_015964.1),GUCA1B(NG_016216.1), HMCN1 (NG_011841.1), IMPG1(NG_041812.1), OTX2(NG_008204.1), PRD M13(NC_000006.12 (99606774..99615578)), PROM1(NG_011696.1), PRPH2(NG_00917 6.1),RP1L1(NG_028035.1), TIMP3(NG_009117.1) |
| Macular degeneration, autosomal recessive | ABCA4(NG_009073.1), CFH(NG_007259.1), DRAM2(NC_000001. 11 (111117332..111140216, complement)), IMPG1(NG_041812.1), MFSD8(NG_008657.1) |
| Macular degeneration, X-linked | RPGR(NG_009553.1) |
| Ocular-retinal developmental disease, autosomal dominant | VCAN(NG_012682.1) |
| Optic atrophy, autosomal dominant | MFN2(NG_007945.1), NR2F1(NG_034119.1), OPA1(NG_011605.1 ) |
| Optic atrophy, autosomal recessive | RTN41P1(NC_000006.12 (106571028..106630500, complement)), SLC25A46(NC _000005.10 (110738136..110765161)), TMEM126A(NG_017157.1) |
| Optic atrophy, X-linked | TIMM8A(NG_011734.1) |
| Retinitis pigmentosa, autosomal dominant | ARL3(NC_000010.11 (102673727..102714433, complement)), BEST1(NG_009033.1), CA4(NG_012050.1), CRX(N G_008605.1), FSCN2(NG_015964.1), GUCA1B(NG_016216.1), HK 1(NG_012077.1), IMPDH1(NG_009194.1),KLHL7(NG_016983.1), NR2E3(NG_009113.2), NRL(NG_011697.1), PRPF3(NG_008245.1), PRPF4(NG_034225.1), PRPF6(NG_029719.1), PRPF8(NG_009118. 1), PRPF31(NG_009759.1),PRPH2(NG_009176.1), RDH12(NG_008 321.1), RHO(NG_009115.1), ROM1(NG_009845.1), RP1(NG_0098 40.1), RP9(NG_012968.1), RPE65(NG_008472.1), SEMA4A(NG_02 7683.1), SNRNP200(NG_016973.1), SPP2(NG_008668.1), TOPORS(NG_01 7050.1) |
| Retinitis pigmentosa, autosomal recessive | ABCA4(NG_009073.1), AGBL5(NC _000002.12 (27051423..27070622)), ARL6(NG_008119.2), ARL2BP(NG_03390 5.1), BBS1(NG_009093.1), BBS2(NG_009312.1), BEST1(NG_0090 33.1), C2orf71(NG_021427.1),C8orf37(NG_032804.1), CERKL(NG _021178.1), CLRN1(NG_009168.1), CNGA1(NG_009193.1), CNGB 1(NG_016351.1), CRB1(NG_008483.2), CYP4V2(NG_007965.1),D HDDS(NG_029786.1), DHX38(NG_034207.1), EMC1(NG 032948. 1), EYS(NG_023443.2), FAM161A(NG_028125.1), GPR125(NC_00 0004.12 (22387374..22516058, complement)), HGSNAT(NG_009552.1),IDH3B(NG_012149.1), IFT 140(NG_032783.1), IFT172(NG_034068.1),IMPG2(NG_028284.1), KIAA1549(NG_032965.1), KIZ(NG_033122.1), LRAT(NG_009110. 1), MAK(NG_030040.1),MERTK(NG_011607.1), MVK(NG_00770 2.1), NEK2(NG_029112.1), NEUROD1(NG_011820.1), NR2E3(NG _009113.2), NRL(NG_011697.1), PDE6A(NG_009102.1), PDE6B(N G_009839.1),PDE6G(NG_009834.1), POMGNT1(NG_009205.2), P RCD(NG_016702.1), PROM1(NG_011696.1), RBP3(NG_029718.1), RGR(NG_009106.1), RHO(NG_009115.1), RLBP1(NG_008116.1), RP1(NG_009840.1), RP1L1(NG_028035.1), RPE65(NG_008472.1), SAG(NG_009116.1), SLC7A14(NG_034121.1), SPATA7(NG_0211 83.1), TTC8(NG_008126.1), TULP1(NG_009077.1), USH2A(NG_009497.1), ZNF408(NC_000011.10 (46700767..46705916)), ZNF513(NG_028219.1) |
| Retinitis pigmentosa, X-linked | OFD1(NG_008872.1), RP2(NG_009107.1, RPGR(NG_009553.1) |
| Syndromic/systemic diseases with retinopathy, autosomal dominant | ABCC6(NG_007558.2), ATXN7(NG_008227.1), COL11A1(NG_00 8033.1), COL2A1(NG_008072.1), JAG1(NG_007496.1), KCNJ13(N G_016742.1), KIF11(NG_032580.1),MFN2(NG_007945.1), OPA3(N G_013332.1), PAX2(NG_008680.2), TREX1(NG_009820.1), VCAN (NG_012682.1) |
| Syndromic/systemic diseases with retinopathy, autosomal recessive | ABCC6(NG_007558.2), ABHD12(NG_028119.1), ACBD5(NG_032 960.2), ADAMTS18(NG_031879.1), ADIPOR1(NC_000001.11 (202940825..202958572, complement)), AHI1(NG_008643.1), ALMS1(NG_011690.1),CC2D 2A(NG_013035.1), CEP164(NG_033032.1), CEP290(NG_008417.1) , CLN3(NG_008654.2), COL9A1(NG_011654.1), CSPP1(NG_03410 0.1), ELOVL4(NG_009108.1), EXOSC2(NC_000009.12 (130693760..130704894)), FLVCR1(NG_028131.1), GNPTG(NG_0 16985.1), HARS(NG_032158.1), HGSNAT(NG_009552.1), HMX1( NG_013062.2), IFT140(NG_032783.1), INPP5E(NG_016126.1), INVS(NG_008316.1), IQCB1(NG_015887. 1), LAMA1(NG_034251.1), LRP5(NG_015835.1), MKS1(NG_0130 32.1), MTTP(NG_011469.1), NPHP1(NG_008287.1),NPHP3(NG_00 8130.1), NPHP4(NG_011724.2), OPA3(NG_013332.1), PANK2(NG _008131.3), PCYT1A(NG_042817.1), PEX1(NG_008341.1), PEX2( NG_008371.1), PEX7(NG_ 008462.1), PHYH(NG_ 012862.1), PLK4(NG_ 041821.1), PNPLA6(NG_013374. 1), POC1B(NG_041783.1), PRPS1(NG_008407.1), RDH11(NG_042 282.1), RPGRIP1L(NG_008991.2),SDCCAG8(NG_027811.1), SLC2 5A46(NC_000005.10 (110738136..110765161)), TMEM237(NG_032049.1), TRNT1(NG_ 041800.1), TTPA(NG_016123.1), TUB(NG_029912.1), TUBGCP4(NG_042168.1), TUBGCP6(NG_032160.1), WDPCP(NG _028144.1), WDR19(NG_031813.1), WFS1(NG_011700.1), ZNF423 (NG_032972.2) |
| Syndromic/systemic diseases with retinopathy, X-linked | OFD1(NG_008872.1),TIMM8A(NG_011734.1) |
| Usher syndrome, autosomal recessive | ABHD12(NG_028119.1), CDH23(NG_008835.1), CEP250(NC_000 020.11 (35455139..35517531)), CIB2(NG_033006.1), CLRN1(NG_009168. 1), DFNB31(NG_016700.1), GPR98(NG_007083.1), HARS(NG_032 158.1), MYO7A(NG_009086.1), PCDH15(NG_009191.2), USH1C(NG_011 883.1), USH1G(NG_007882.1), USH2A(NG_009497.1) |
| Other retinopathy, autosomal dominant | BEST1(NG_009033.1), CAPN5(NG_033002.1), CRB1(NG_008483. 2), FZD4(NG_011752.1), ITM2B(NG_013069.1), LRP5(NG_015835 .1), MAPKAPK3(NC_000003.12 (50611862..50649297)), MIR204(NR_029621.1), OPN1SW(NG_009094.1), RB1(NG_009009 .1), TSPAN12(NG_023203.1), ZNF408(NC_000011.10 (46700767..46705916)) |
| Other retinopathy, autosomal recessive | BEST1(NG_009033.1), C12orf65(NG_027517.1), CDH3(NG_00909 6.1), CNGA3(NG_009097.1), CNGB3(NG_016980.1), CNNM4(NG _016608.1), CYP4V2(NG_007965.1), LRP5(NG_ 015835.1), MFRP(NG_ 012235.1), MVK(NG_ 007702.1), NBAS(NG 032964.1), NR2E3(NG 009113.2), OAT(NG 008861.1), PLA2G5(NG_032045.1), PROM1(NG_011696.1), RBP4(NG_00910 4.1), RGS9(NG_013021.1), RGS9BP(NG_016751.1), RLBP1(NG_008116 .1) |
| Other retinopathy, mitochondrial | MT-ATP6(NC_012920.1 (8527..9207)), MT-TH(NC_012920.1 (12138..12206)), MT-TL1(NC_012920.1 (3230..3304)), MT-TP(NC_012920.1 (15956..16023, complement)), MT-TS2(NC_012920.1 (12207..12265)) |
| Other retinopathy, X-linked | CACNA1F(NG_009095.2), CHM(NG_009874.2), DMD(NG_012232 .1), NDP(NG_009832.1), OPN1LW(NG_009105.2), OPN1MW(NG_ 011606.1), PGK1(NG_008862.1), RS1(NG_008659.3) |

The methods of the present disclosure can also be used to prevent, correct, or treat cancers that arise due to the presence of mutation in a tumor suppressor gene. Examples of tumor suppression genes include: retinoblastoma susceptibility gene (RB) gene, p53 gene, deleted in colon carcinoma (DCC) gene, adenomatous polyposis coli (APC) gene, p16, BRCA1, BRCA2, MSH2, and the neurofibromatosis type 1 (NF-1) tumor suppressor gene (Lee at al. Cold Spring Harb Perspect Biol. 2010 Oct; 2(10):).

Tumor suppressor genes are genes that, in their wild-type alleles, express proteins that suppress abnormal cellular proliferation. When the gene coding for a tumor suppressor protein is mutated or deleted, the resulting mutant protein or the complete lack of tumor suppressor protein expression may fail to correctly regulate cellular proliferation, and abnormal cellular proliferation may take place, particularly if there is already existing damage to the cellular regulatory mechanism. A number of well-studied human tumors and tumor cell lines have been shown to have missing or nonfunctional tumor suppressor genes. Thus, a loss of function or inactivation of tumor suppressor genes may play a central role in the initiation and/or progression of a significant number of human cancers.

The methods of the present disclosure may be used treat patients at a different stage of the disease (e.g. early, middle or late). The present methods may be used to treat a patient once or multiple times. Thus, the length of treatment may vary and may include multiple treatments.

As discussed in the present disclosure, the methods or the present disclosure can be used for correcting or treating autosomal dominant ocular disease in a subject. For example, the "Chop" step involves deletion of both the mutant copy of the autosomal dominant ocular disease-related gene that is to be corrected, and/or the endogenous wild-type copy of the same gene in a patient afflicted with autosomal dominant ocular disease. Thus, the "Chop" step results in complete or partial loss of both mutant and/or wild-type activity of a gene. The autosomal dominant ocular disease-related gene is then corrected using the "Stick" step, which involves the introduction of a sequence encoding a modified autosomal dominant ocular disease-related gene or fragment. The modified, autosomal dominant ocular disease-related gene sequence can be modified in such a way that it is not recognized (unrecognizable) by sgRNA, which targets the wild-type or mutant form of the gene (non-codon-modified form of the gene). This modification renders the codon-modified donor template resistant to the Cas-family nuclease.

The constructs encoding the "Chop" and "Stick" components can be delivered to the subject using one or more recombinant adeno-associated viral (AAV) vectors (e.g., 1, 2, 3, 4, 5, 6, 7, 8, 9, or more AAV vectors). One or more sgRNAs can be packaged into single (one) recombinant AAV vector. The recombinant AAV vector may also include codon-modified autosomal dominant ocular disease-related gene sequence (donor template). A Cas-family nuclease can be packaged into the same, or alternatively separate recombinant AAV vectors.

The method described here also provides for correcting autosomal dominant ocular disease in a subject, comprising administering to said subject by injection a therapeutically effective amount of a recombinant AAV virus encoding a nucleic acid sequence comprising a CRISPR system polynucleotide sequence, wherein the polynucleotide sequence comprises: (i) one or more guide RNA sequences that hybridize to an autosomal dominant disease-related gene sequence; (ii) a second sequence encoding a codon-modified autosomal dominant disease-related gene or fragment, wherein at least one disease related mutation in the modified autosomal dominant disease-related gene or fragment has been corrected and the codon-modified autosomal dominant disease related gene or fragment cannot be recognized by one or more sg RNA sequences that hybridize to an unmodified autosomal dominant disease-related gene sequence; and (iii) a sequence encoding a Cas family enzyme.

As the carrying capacity of AAV may pose challenges, two or more AAV vectors can be used simultaneously. For example, a Cas family nuclease may be packaged into a different AAV vectors. Furthermore, sequences encoding sgRNA(s), codon-modified autosomal dominant disease-related gene or fragment, and a Cas family nuclease can each be packaged into a separate AAV vector.

In the case of RP treatment, the methods of the present disclosure can comprise: administering to a subject by injection a therapeutically effective amount of a (1) recombinant AAV virus encoding a nucleic acid sequence comprising a CRISPR system polynucleotide sequence, wherein the polynucleotide sequence comprises: (i) two guide RNA sequences that hybridize to mutant and wild type RHO sequences; (ii) a second sequence encoding a codon-modified RHO gene or fragment, where the mutation(s) of the endogenous RHO gene has been corrected and the modified RHO gene or fragment cannot be recognized by one or more sgRNA sequences that hybridize to the mutant and wild type RHO gene sequence; and (2) a second recombinant AAV virus encoding a Cas family enzyme.

Generally, co-expression of a Cas-family enzyme and an autosomal dominant disease-related gene-specific sgRNAs in ocular cells, leads to truncation of the autosomal dominant disease-related gene, thereby preventing the expression of either the wild-type (wt) or disease-causing mutant gene. Simultaneously, codon-modified cDNA of the autosomal dominant disease-related gene may also be supplied to ocular cells, where the coding sequence of autosomal dominant disease-related gene is modified in such a way that is resistant to sgRNAs (and thus resistant to Cas family nuclease). This strategy results in the expression of codon-modified cDNA of the autosomal dominant disease-related gene, which can restore or correct the function of the autosomal dominant disease-related gene or fragment after the deletion of endogenous gene(s) or fragments.

The codon-modified cDNA (donor-template) may be modified in such a way as to render it unrecognizable by the sgRNA(s) used to target either mutant and wt disease-related gene(s). Thus, mutations need to be introduced into a donor-template gene or fragment to avoid this donor-template gene or fragment being recognized by sgRNA(s) and consequently degraded by Cas enzyme (for example a Cas9 nuclease) which has been introduced in cells. This can be accomplished by introducing a wobble base into donor-template, thus making sure that the change in DNA results in a silent mutation, leaving the expression product of wt gene intact. The term "wobble base" as used in the present disclosure refers to a change in a one or more nucleotide bases of a reference nucleotide sequence wherein the change does not change the sequence of the amino acid coded by the nucleotide relative to the reference sequence.

The number of wobble bases that need to be introduced into donor-template may range from about 1-30, about 1-20, about 2-19, about 3-18, about 4-17, about 5-16, about 6-15, about 7-14, about 8-13, about 9-12, about 10-11, about 9, about 8, about 7, about 6., or about 5. Additionally, given the numerous software applications available for in-silico predictive modeling of sgRNA, one can perform in-silico analysis to test whether codon-modified donor-template would be recognized by sgRNA. An example of publically available CRISPR sgRNA tool can be found at http://www.genscript.com/gRNA-design-tool.html: retrieved April 30, 2016.

Alternatively, if the goal of the treatment is to delete, destroy, or truncate only mutated form of a gene or a fragment, and leave the wild type form intact, donor template or wild type gene sequence that is supplemented to the cells or a patient may not be codon-modified. Under such circumstances, sgRNA(s) used as part of the CRISPR components would be designed to recognize and target only the mutated form of a disease-related gene (and not recognize and target a wild type (such as donor-template) form of said gene).

The methods of the present disclosure have been applied to various genes, including PDE6A, EFEMP1, mouse Rhodopsin (RHO), and human RHO genes. RP can be caused by autosomal recessive mutations in the PDE6A gene, or autosomal dominant mutations in RHO gene. Mutations in EFEMP1 are responsible for autosomal dominant Malattia Leventinese (ML) and Doyne honeycomb retinal dystrophy (DHRD). Moreover, the methods have been applied to various cell types, including, but not limited to, mouse retina cells as well as human iPS cells. Additionally, the methods described here have also been applied *in vivo* using a mouse model of ocular disease. Thus, methods of the present disclosure can be applied to both animal as well as human subjects.

Furthermore, methods of the present disclosure that have been applied to specific gene-humanized mouse model as well as patient-derived cells allow for determining the efficiency and efficacy of designed sgRNA and site-specific recombination frequency in human cells, which can be then used as a guide in a clinical setting.

In one embodiment, the "ChopStick" system comprises the following components: two recombinant AAV vectors: the first carrying a polynucleotide encoding the Cas9 enzyme to "Chop" the mutant and/or native rhodopsin genes, and the second carrying a nucleotide encoding the codon-modified human rhodopsin cDNA to "Stick" the normal rhodopsin back into the patient. The codon-modified or genetically engineered human rhodopsin sequence, which is driven by the CBh promoter is resistant to destruction by the gene-editing enzyme, rescues the patient's phenotype.

In one embodiment, the present method provides at least 50% rhodopsin levels from the CBh promoter-driven codon-modified RHO cDNA, which are sufficient to improve survival. In another embodiment, there is not an excessive amount of rhodopsin expressed using the codon-modified *RHO* donor sequence.

For studies using human and patient-derived cells, the inventors chose AAV2 vector as a backbone vector for all the constructs, as it has been shown that AAV2 may transduce human iPS more efficiently than other AAV vectors (Mitsui K et al. Biochem Biophys Res Commun. 2009 Oct 30;388(4):711-7; Deyle DR et al. Mol Ther. 2012 Jan;20(1):204-13; and Deyle DR et al. Nucleic Acids Res. 2014 Mar;42(5):3119-24). However, a variety of other AAV vectors may also be used to carry out the methods of the present disclosure.

The degree of improvement of the autosomal dominant disease by the present methods can vary. For example, the present methods may restore about 20%, about 30%, about 40%, greater than 10%, greater than 20%, greater than 30%, greater than 40%, greater than 50%, greater than 60%, greater than 70%, greater than 80%, or greater than 90%, of the autosomal dominant disease-related gene expression, of the normal levels of the gene product in a control subject, which may be age and sex matched.

In certain embodiments, expression of a wild-type gene (e.g., rhodopsin) can be observed in about 2 weeks following administration to a subject and/or cells. Expression may be maintained for unlimited period of time in nondividing somatic cells (e.g., photoreceptors, neuron cells, muscle cells, etc.). In one embodiment, expression of wild-type rhodopsin is observed in about 3 days, in about 1 week, in about 3 weeks, in about 1 month, in about 2 months, from about 1 week to about 2 weeks, or within different time-frames.

According to the various embodiments of the present disclosure, a variety of known viral constructs may be used to deliver desired (Chop and Stick) components such as Cas-family nuclease, sgRNA(s), codon-modified wild-type gene (also referred to as codon-modified donor template), donor template, etc. to the targeted cells and/or a subject. Nonlimiting examples of such recombinant viruses include recombinant adeno-associated virus (AAV), recombinant adenoviruses, recombinant lentiviruses, recombinant retroviruses, recombinant poxviruses, and other known viruses in the art, as well as plasmids, cosmids, and phages. Options for gene delivery viral constructs are well known (see, e.g., Ausubel et al., Current Protocols in Molecular Biology, John Wiley & Sons, New York, 1989; Kay, M. A., et al., 2001 Nat. Medic. 7(1):33-40; and Walther W. and Stein U., 2000 Drugs, 60(2): 249-71).

Additionally delivery vehicles such as nanoparticle- and lipid-based mRNA or protein delivery systems can be used as an alternative to AAV vectors. Further examples of alternative delivery vehicles include lentiviral vectors, lipid-based delivery system, gene gun, hydrodynamic, electroporation or nucleofection microinjection, and biolistics. Various gene delivery methods are discussed in detail by Nayerossadat et al. (Adv Biomed Res. 2012; 1: 27) and Ibraheem et al. (Int J Pharm. 2014 Jan 1;459(1-2):70-83).

The present methods may utilize adeno-associated virus (AAV) mediated genome engineering. AAV vectors possess a broad host range; transduce both dividing and non-dividing cells *in vitro* and *in vivo* and maintain high levels of expression of the transduced genes. Viral particles are heat stable, resistant to solvents, detergents, changes in pH, temperature, and can be concentrated on CsCl gradients. AAV is not associated with any pathogenic event, and transduction with AAV vectors has not been found to induce any lasting negative effects on cell growth or differentiation. In contrast to other vectors, such as lentiviral vectors, AAVs lack integration machinery and have been approved for clinical use (Wirth et al. Gene. 2013 Aug 10;525(2): 162-9).

The single-stranded DNA AAV viral vectors have high transduction rates in many different types of cells and tissues. Upon entering the host cells, the AAV genome is converted into double-stranded DNA by host cell DNA polymerase complexes and exist as an episome. In non-dividing host cells, the episomal AAV genome can persist and maintain long-term expression of a therapeutic transgene. (J Virol. 2008 Aug; 82(16): 7875-7885).

AAV vectors and viral particles of the present disclosure may be employed in various methods and uses. In one embodiment, a method encompasses delivering or transferring a heterologous polynucleotide sequence into a patient or a cell of a patient and includes administering a viral AAV particle, a plurality of AAV viral particles, or a pharmaceutical composition of a AAV viral particle or plurality of AAV viral particles to a patient or a cell of the patient, thereby delivering or transferring a heterologous polynucleotide sequence into the patient or cell of the patient.

In another embodiment, the method is for treating a patient deficient or in need of protein expression or function, or in need of reduced expression or function of an endogenous protein (e.g., an undesirable, aberrant or dysfunctional protein), that includes providing a recombinant AAV viral particle, a plurality of recombinant AAV viral particles, or a pharmaceutical composition of a recombinant AAV viral particle or plurality of AAV viral particles; and administering the recombinant AAV viral particle, plurality of recombinant AAV viral particles, or pharmaceutical composition of AAV viral particle or plurality of AV viral particles to the patient, where the heterologous polynucleotide sequence is expressed in the patient, or wherein the heterologous polynucleotide sequence encodes one or more sgRNA(s) that reduces and or deletes endogenous DNA segment (e.g. , an undesirable, aberrant or dysfunctional DNA segment) in the patient, and where the heterologous polynucleotide sequence encodes a codon modified gene or fragment thereof that is not recognizable by one or more sgRNA(s) used to reduce and or delete endogenous DNA segment.

The characterization of new AAV serotypes has revealed that they have different patterns of transduction in diverse tissues. For illustrative purposes, AAV2 and AAV8 were used in the Examples of the present disclosure; however, for the purposes of the present invention, AAV viral vectors may be selected from among any AAV serotype, including, without limitation, AAV1, AAV2, AAV3, AAV4, AAV5, AAV6, AAV7, AAV8, AAV9, AAV10 or other known and unknown AAV serotypes.

The term AAV covers all subtypes, serotypes and pseudotypes, and both naturally occurring and recombinant forms, except where required otherwise. Pseudotyped AAV refers to an AAV that contains capsid proteins from one serotype and a viral genome of a second serotype.

To minimize the intensity and duration of Cas9 expression and potential off-targeting effects, self-excisional AAV-Cas9 vectors have also been generated, which have the ability to self-inactivate Cas9 expression shortly after Cas9 production. This approach comprises flanking the Cas9 gene with two sgRNA-Y1 target sites (similar to loxP sites in Cre recombinase system) to terminate Cas9 own expression (as shown in Figure 9). It is anticipated that the amount of Cas9 enzyme present (before it terminates itself) is still sufficient to cut the desired locus (such as Rho or PDE6A locus for example).

The design of self-inactivating recombinant AAV vectors (see Figure 9) enables the inventors to control the amount and duration of Cas9 expression in target cells, and can prevent the unwanted off-target effects due to excessive expression of Cas9 protein.

Vectors of the present disclosure can comprise any of a number of promoters known to the art, wherein the promoter is constitutive, regulatable or inducible, cell type specific, tissue-specific, or species specific. In addition to the sequence sufficient to direct transcription, a promoter sequence of the invention can also include sequences of other regulatory elements that are involved in modulating transcription (e.g., enhancers, kozak sequences and introns). Many promoter/regulatory sequences useful for driving constitutive expression of a gene are available in the art and include, but are not limited to, for example, CMV (cytomegalovirus promoter), EF1a (human elongation factor 1 alpha promoter), SV40 (simian vacuolating virus 40 promoter), PGK (mammalian phosphoglycerate kinase promoter), Ubc (human ubiquitin C promoter), human beta-actin promoter, rodent beta-actin promoter, CBh (chicken beta-actin promoter), CAG (hybrid promoter contains CMV enhancer, chicken beta actin promoter, and rabbit beta-globin splice acceptor), TRE (Tetracycline response element promoter), HI (human polymerase III RNA promoter), U6 (human U6 small nuclear promoter), and the like. Moreover, inducible and tissue specific expression of an RNA, transmembrane proteins, or other proteins can be accomplished by placing the nucleic acid encoding such a molecule under the control of an inducible or tissue specific promoter/regulatory sequence. Examples of tissue specific or inducible promoter/regulatory sequences which are useful for this purpose include, but are not limited to, the rhodopsin promoter, the MMTV LTR inducible promoter, the SV40 late enhancer/promoter, synapsin 1 promoter, ET hepatocyte promoter, GS glutamine synthase promoter and many others. Various commercially available ubiquitous as well as tissue-specific promoters can be found at http://www.invivogen.com/prom-a-list. In addition, promoters which are well known in the art can be induced in response to inducing agents such as metals, glucocorticoids, tetracycline, hormones, and the like, are also contemplated for use with the invention. Thus, it will be appreciated that the present disclosure includes the use of any promoter/regulatory sequence known in the art that is capable of driving expression of the desired protein operably linked thereto.

Vectors according to the present disclosure can be transformed, transfected or otherwise introduced into a wide variety of host cells. Transfection refers to the taking up of a vector by a host cell whether or not any coding sequences are in fact expressed. Numerous methods of transfection are known to the ordinarily skilled artisan, for example, lipofectamine, calcium phosphate co-precipitation, electroporation, DEAE-dextran treatment, microinjection, viral infection, and other methods known in the art. Transduction refers to entry of a virus into the cell and expression (e.g., transcription and/or translation) of sequences delivered by the viral vector genome. In the case of a recombinant vector, "transduction" generally refers to entry of the recombinant viral vector into the cell and expression of a nucleic acid of interest delivered by the vector genome.

The method of treating an autosomal dominant ocular disease in a patient can comprise administering to the patient an effective concentration of a composition comprising any of the recombinant AAVs described herein and a pharmaceutically acceptable carrier. In one embodiment, an effective concentration of virus is 1×10⁶ - 11×10¹³ GC/ml (genome copies/ml). The range of viral concentration effective for the treatment can vary depending on factors including, but not limited to specific mutation, patient's age, and other clinical parameters.

Recombinant AAV vectors(s) encoding CRISPR-Cas components and/or codon-modified donor-template comprising autosomal dominant disease-related gene or fragment can be produced *in vitro,* prior to administration into a patient. Production of recombinant AAV vectors and their use in *in vitro* and *in vivo* administration has been discussed in detail by Gray et al. (Curr. Protoc. Neurosci. 2011 Oct, Chapter:Unit 4.17).

The recombinant AAV containing the desired recombinant DNA can be formulated into a pharmaceutical composition intended for subretinal or intravitreal injection. Such formulation involves the use of a pharmaceutically and/or physiologically acceptable vehicle or carrier, particularly one suitable for administration to the eye, e.g., by subretinal injection, such as buffered saline or other buffers, e.g., HEPES, to maintain pH at appropriate physiological levels, and, optionally, other medicinal agents, pharmaceutical agents, stabilizing agents, buffers, carriers, adjuvants, diluents, etc. For injection, the carrier will typically be a liquid. Exemplary physiologically acceptable carriers include sterile, pyrogen-free water and sterile, pyrogen-free, phosphate buffered saline.

In one embodiment, the carrier is an isotonic sodium chloride solution. In another embodiment, the carrier is balanced salt solution. In one embodiment, the carrier includes tween. If the virus is to be stored long-term, it may be frozen in the presence of glycerol or Tween-20. In another embodiment, the pharmaceutically acceptable carrier comprises a surfactant, such as perfluorooctane (Perfluoron liquid). In certain embodiments, the pharmaceutical composition described above is administered to the subject by subretinal injection. In other embodiments, the pharmaceutical composition is administered by intravitreal injection. Other forms of administration that may be useful in the methods described herein include, but are not limited to, direct delivery to a desired organ (e.g., the eye), oral, inhalation, intranasal, intratracheal, intravenous, intramuscular, subcutaneous, intradermal, and other parental routes of administration. Additionally, routes of administration may be combined, if desired.

In preferred embodiments, route of administration is subretinal injection or intravitreal injection.

Methods for modification of genomic DNA are well known in the art. For example, methods may use a DNA digesting agent to modify the DNA by either the non-homologous end joining DNA repair pathway (NHEJ) or the homology directed repair (HDR) pathway. The term "DNA digesting agent" refers to an agent that is capable of cleaving bonds (i.e. phosphodiester bonds) between the nucleotide subunits of nucleic acids.

In one embodiment, the DNA digesting agent is a nuclease. Nucleases are enzymes that hydrolyze nucleic acids. Nucleases may be classified as endonucleases or exonucleases. An endonuclease is any of a group of enzymes that catalyze the hydrolysis of bonds between nucleic acids in the interior of a DNA or RNA molecule. An exonuclease is any of a group of enzymes that catalyze the hydrolysis of single nucleotides from the end of a DNA or RNA chain. Nucleases may also be classified based on whether they specifically digest DNA or RNA. A nuclease that specifically catalyzes the hydrolysis of DNA may be referred to as a deoxyribonuclease or DNase, whereas a nuclease that specifically catalyses the hydrolysis of RNA may be referred to as a ribonuclease or an RNase. Some nucleases are specific to either single-stranded or double-stranded nucleic acid sequences. Some enzymes have both exonuclease and endonuclease properties. In addition, some enzymes are able to digest both DNA and RNA sequences.

Non-limiting examples of the endonucleases include a zinc finger nuclease (ZFN), a ZFN dimer, a ZFNickase, a transcription activator-like effector nuclease (TALEN), or a RNA-guided DNA endonuclease (e.g., CRISPR/Cas9). Meganucleases are endonucleases characterized by their capacity to recognize and cut large DNA sequences (12 base pairs or greater). Any suitable meganuclease may be used in the present methods to create double-strand breaks in the host genome, including endonucleases in the LAGLIDADG and PI-Sce family.

One example of a sequence-specific nuclease system that can be used with the methods and compositions described herein includes the CRISPR system (Wiedenheft, B. et al. Nature 482, 331-338 (2012); Jinek, M. et al. Science 337, 816-821 (2012); Mali, P. et al. Science 339, 823-826 (2013); Cong, L. et al. Science 339, 819-823 (2013)). The CRISPR (Clustered Regularly interspaced Short Palindromic Repeats) system exploits RNA-guided DNA-binding and sequence-specific cleavage of target DNA. The guide RNA/Cas combination confers site specificity to the nuclease. A single guide RNA (sgRNA) contains about 20 nucleotides that are complementary to a target genomic DNA sequence upstream of a genomic PAM (protospacer adjacent motifs) site (NGG) and a constant RNA scaffold region. The Cas (CRISPR-associated) protein binds to the sgRNA and the target DNA to which the sgRNA binds and introduces a double-strand break in a defined location upstream of the PAM site. Cas9 harbors two independent nuclease domains homologous to HNH and RuvC endonucleases, and by mutating either of the two domains, the Cas9 protein can be converted to a nickase that introduces single-strand breaks (Cong, L. et al. Science 339, 819-823 (2013)). It is specifically contemplated that the methods and compositions of the present disclosure can be used with the single- or double-strand-inducing version of Cas9, as well as with other RNA-guided DNA nucleases, such as other bacterial Cas9-like systems. The sequence-specific nuclease of the present methods and compositions described herein can be engineered, chimeric, or isolated from an organism. The nuclease can be introduced into the cell in form of a DNA, mRNA and protein.

In one embodiment, the methods of the present disclosure comprise using one or more sgRNAs to "Chop", remove, or suppress an autosomal dominant disease- related gene. In another embodiment, one sgRNA(s) is used to "Chop", remove, or suppress an autosomal dominant disease- related gene. In yet further embodiment, two or more sgRNA(s) are used to "Chop", remove, or suppress an autosomal dominant disease- related gene.

In one embodiment, the DNA digesting agent can be a site-specific nuclease. In another embodiment, the site-specific nuclease may be a Cas-family nuclease. In a more specific embodiment, the Cas nuclease may be a Cas9 nuclease.

In one embodiment, Cas protein may be a functional derivative of a naturally occurring Cas protein.

In addition to well characterized CRISPR-Cas system, a new CRISPR enzyme, called Cpfl (Cas protein 1 of PreFran subtype) has recently been described (Zetsche et al. Cell. pii: S0092-8674(15)01200-3. doi: 10.1016/j.cell.2015.09.038 (2015)). Cpfl is a single RNA-guided endonuclease that lacks tracrRNA, and utilizes a T-rich protospacer-adjacent motif. The authors demonstrated that Cpfl mediates strong DNA interference with characteristics distinct from those of Cas9. Thus, in one embodiment of the present invention, CRISPR-Cpfl system can be used to cleave a desired region within the targeted gene.

In further embodiment, the DNA digesting agent is a transcription activator-like effector nuclease (TALEN). TALENs are composed of a TAL effector domain that binds to a specific nucleotide sequence and an endonuclease domain that catalyzes a double strand break at the target site (PCT Patent Publication No. WO2011072246; Miller et al., Nat. Biotechnol. 29, 143-148 (2011); Cermak et al., Nucleic Acid Res. 39, e82 (2011)). Sequence-specific endonucleases may be modular in nature, and DNA binding specificity is obtained by arranging one or more modules. Bibikova et al., Mol. Cell. Biol. 21, 289-297 (2001). Boch et al., Science 326, 1509-1512 (2009).

ZFNs can be composed of two or more (e.g., 2 - 8, 3 - 6, 6 - 8, or more) sequence-specific DNA binding domains (e.g., zinc finger domains) fused to an effector endonuclease domain (e.g., the FokI endonuclease). Porteus et al., Nat. Biotechnol. 23, 967-973 (2005). Kim et al. (2007) Hybrid restriction enzymes: Zinc finger fusions to Fok I cleavage domain, Proceedings of the National Academy of Sciences of USA, 93: 1156-1160. U.S. Patent No. 6,824,978. PCT Publication Nos. WO1995/09233 and WO1994018313.

In one embodiment, the DNA digesting agent is a site-specific nuclease of the group or selected from the group consisting of omega, zinc finger, TALE, and CRISPR/Cas.

The sequence-specific endonuclease of the methods and compositions described here can be engineered, chimeric, or isolated from an organism. Endonucleases can be engineered to recognize a specific DNA sequence, by, e.g., mutagenesis. Seligman et al. (2002) Mutations altering the cleavage specificity of a homing endonuclease, Nucleic Acids Research 30: 3870-3879. Combinatorial assembly is a method where protein subunits form different enzymes can be associated or fused. Arnould et al. (2006) Engineering of large numbers of highly specific homing endonucleases that induce recombination to novel DNA targets, Journal of Molecular Biology 355: 443-458. In certain embodiments, these two approaches, mutagenesis and combinatorial assembly, can be combined to produce an engineered endonuclease with desired DNA recognition sequence.

The sequence-specific nuclease can be introduced into the cell in the form of a protein or in the form of a nucleic acid encoding the sequence-specific nuclease, such as an mRNA or a cDNA. Nucleic acids can be delivered as part of a larger construct, such as a plasmid or viral vector, or directly, e.g., by electroporation, lipid vesicles, viral transporters, microinjection, and biolistics. Similarly, the construct containing the one or more transgenes can be delivered by any method appropriate for introducing nucleic acids into a cell.

Single guide RNA(s) used in the methods of the present disclosure can be designed so that they direct binding of the Cas-sgRNA complexes to pre-determined cleavage sites in a genome. In one embodiment, the cleavage sites may be chosen so as to release a fragment or sequence that contains a region of autosomal dominant disease-related gene. In further embodiment, the cleavage sites may be chosen so as to release a fragment or sequence that contains a region *of RHO.*

For Cas family enzyme (such as Cas9) to successfully bind to DNA, the target sequence in the genomic DNA should be complementary to the sgRNA sequence and must be immediately followed by the correct protospacer adjacent motif or "PAM" sequence. "Complementarity" refers to the ability of a nucleic acid to form hydrogen bond(s) with another nucleic acid sequence by either traditional Watson-Crick or other non-traditional types. A percent complementarity indicates the percentage of residues in a nucleic acid molecule, which can form hydrogen bonds (e.g., Watson-Crick base pairing) with a second nucleic acid sequence. Full complementarity is not necessarily required, provided there is sufficient complementarity to cause hybridization and promote formation of a CRISPR complex. A target sequence may comprise any polynucleotide, such as DNA or RNA polynucleotides. The Cas9 protein can tolerate mismatches distal from the PAM, however, mismatches within the 12 base pairs (bps) of sequence next to the PAM sequence can dramatically decrease the targeting efficiency. The PAM sequence is present in the DNA target sequence but not in the sgRNA sequence. Any DNA sequence with the correct target sequence followed by the PAM sequence will be bound by Cas9. The PAM sequence varies by the species of the bacteria from which Cas9 was derived. The most widely used CRISPR system is derived from *S. pyogenes* and the PAM sequence is NGG located on the immediate 3' end of the sgRNA recognition sequence. The PAM sequences of CRISPR systems from exemplary bacterial species include: *Streptococcus pyogenes* (NGG), *Neisseria meningitidis* (NNNNGATT), *Streptococcus thermophilus* (NNAGAA) and *Treponema denticola* (NAAAAC).

sgRNA(s) used in the present disclosure can be between about 5 and 100 nucleotides long, or longer (e.g., 5, 6, 7, 8, 9, 10, 11, 12, 13, 14, 15, 16, 17, 18, 19, 20, 21, 22, 23, 24, 25, 26, 27, 28, 29, 30, 31 , 32, 33, 34, 35, 36, 37, 38, 39, 40, 41, 42, 43, 44, 45, 46, 47, 48, 49, 50, 51, 52, 53, 54, 55, 56, 57, 58, 59 60, 61, 62, 63, 63, 64, 65, 66, 67, 68, 69, 70, 71, 72, 73, 74, 75, 76, 77, 78, 79, 80, 81 , 82, 83, 84, 85, 86, 87, 88, 89, 90, 91 92, 93, 94, 95, 96, 97, 98, 99, or 100 nucleotides in length, or longer). In one embodiment, sgRNA(s) can be between about 15 and about 30 nucleotides in length (e.g., about 15-29, 15-26, 15-25; 16-30, 16-29, 16-26, 16-25; or about 18-30, 18-29, 18-26, or 18-25 nucleotides in length).

To facilitate sgRNA design, many computational tools have been developed (See Prykhozhij et al. (PLoS ONE, 10(3): (2015)); Zhu et al. (PLoS ONE, 9(9) (2014)); Xiao et al. (Bioinformatics. Jan 21 (2014)); Heigwer et al. (Nat Methods, 11(2): 122-123 (2014)). Methods and tools for guide RNA design are discussed by Zhu (Frontiers in Biology, 10 (4) pp 289-296 (2015)), which is incorporated by reference herein. Additionally, there is a publically available software tool that can be used to facilitate the design of sgRNA(s) (http: //www.genscript.com/gRNA-design-tool.html).

A modified autosomal dominant disease-related gene or fragment sequence is a donor sequence that has been codon modified to be unrecognizable by sgRNA(s) used for targeting or recognition of the mutated autosomal dominant disease-related gene and resistant to sgRNA targeting. Such modified autosomal dominant disease-related gene sequence is a donor sequence encoding at least a functional fragment of the protein lacking or deficient in the subject with autosomal dominant disease.

As previously mentioned, the codon-modified cDNA (donor-template) may be modified in such a way as to render it unrecognizable by the sgRNA(s) used to target either mutant and wildtype disease-related gene(s). To achieve this, mutations need to be introduced into a donor-template gene or fragment to render donor-template gene or fragment unrecognizable by sgRNA(s) and consequently resistant to degradation by Cas enzyme (such as Cas9 nuclease) which has been introduced in cells. The donor-template gene may be modified by introducing a wobble base(s) into donor-template. Introduction of wobble base(s) in DNA results in a silent mutation, leaving the expression product of wt gene intact, but if nucleotide sequence has been sufficiently changed, it will render donor-template sequence unrecognizable by sgRNA(s) used to target either mutant and wt disease-related gene(s), ultimately resistant to Cas nuclease cleavage. The number of wobble bases that needs to be introduced into a donor-template may vary, but needs to be sufficient to prevent sgRNA hybridization and formation of a CRISPR complex.

In one embodiment, the donor template sequence may be delivered using the same gene transfer system as used to deliver the Cas nuclease (included on the same vector) or may be delivered using a different delivery system. In another embodiment, the donor template sequence may be delivered using the same transfer system as used to deliver sgRNA(s). In specific embodiments, the donor is delivered using a viral vector (e.g., AAV).

In one embodiment, the present disclosure comprises integration of codon-modified autosomal dominant disease-related gene sequence (donor template sequence) into the endogenous autosomal disease-related gene.

In another embodiment, the donor sequence or modified autosomal dominant disease-related gene sequence is integrated into endogenous gene by homologous recombination (HR).

In further embodiments, the donor sequence or modified autosomal dominant disease-related gene sequence is flanked by an upstream and a downstream homology arm. The homology arms, which flank the donor sequence or modified autosomal dominant disease-related gene sequence, correspond to regions within the targeted locus of autosomal dominant disease-related gene. For example, the corresponding regions within the targeted locus are referred to herein as "target sites". Thus, in one example, a vector that carries a donor or modified autosomal dominant disease-related gene sequence can comprise a donor or modified autosomal dominant disease-related gene sequence flanked by a first and a second homology arm.

A homology arm of the vector that carries a donor or modified autosomal dominant disease-related gene sequence can be of any length that is sufficient to promote a homologous recombination event with a corresponding target site, including for example, 50-100 base pairs, 100-1000 base pairs or at least 5-10, 5-15, 5-20, 5-25, 5-30, 5-35, 5-40, 5-45, 5-50, 5-55, 5-60, 5-65, 5-70, 5-75, 5-80, 5-85, 5-90, 5-95, 5-100, 100-200, or 200-300 base pairs in length or greater.

In one embodiment, the donor template is delivered as a double-stranded DNA. Under such circumstances, the homologous arm may comprise 15-4000 base pairs of each arm. In other embodiments, the donor template is delivered as a single-stranded DNA format. Under such circumstances, the homologous arm may comprise 8-1000 bps of each arm.

A homology arm and a target site "correspond" or are "corresponding" to one another when the two regions share a sufficient level of sequence identity to one another to act as substrates for a homologous recombination reaction. By "homology" is meant DNA sequences that are either identical or share sequence identity to a corresponding sequence. The sequence identity between a given target site and the corresponding homology arm found on the vector that carries a donor or modified autosomal dominant disease-related gene sequence can be any degree of sequence identity that allows for homologous recombination to occur. For example, the amount of sequence identity shared by the homology arm of the vector that carries a donor or modified autosomal dominant disease-related gene sequence (or a fragment thereof) and the target site (or a fragment thereof) should be 100% sequence identity, except the codon-modified region, such that the sequences undergo homologous recombination. Less than 100% sequence identity may be tolerated, provided that the Cas enzyme (Cas 9) cuts only the patient DNA and not the donor template or the patient DNA which is repaired/replaced by the donor template.

Alternatively, donor template (whether codon-modified or not) of a gene of interest or fragment is not integrated into the endogenous disease-related gene. Donor-template may be packaged into an extrachromosomal, or episomal vector (such as AAV vector), which persists in the nucleus in an extrachromosomal state, and offers donor-template delivery and expression without integration into the host genome. Use of extrachromosomal gene vector technologies has been discussed in detail by Wade-Martins R (Methods Mol Biol. 2011;738:1-17).

The nucleases, polynucleotides encoding these nucleases, donor polynucleotides and compositions comprising the proteins and/or polynucleotides described in the present disclosure can be delivered by any suitable means. In certain embodiments, the nucleases and/or donors are delivered *in vivo.* In other embodiments, the nucleases and/or donors are delivered to isolated cells (e.g., autologous iPS cells) for the provision of modified cells useful in *in vivo* delivery to patients afflicted with ocular autosomal dominant disease.

An alternative to injection of viral particles encoding CRISPR components described in the present disclosure (including sgRNA(s), codon-modified donor template gene of fragment sequences, and Cas family nuclease), cell replacement therapy can be used to prevent, correct or treat diseases, where the methods of the present disclosure are applied to isolated patient's cells *(ex vivo),* which is then followed by the injection of "corrected" cells back into the patient.

For the treatment of ocular diseases, patient's iPS cells can be isolated and differentiated into retinal pigment epithelium RPE cells *ex vivo.* RPE cells characterized by the mutation in autosomal dominant disease-related gene may then be manipulated using methods of the present disclosure in a manner that results in the deletion of autosomal dominant disease-related gene, and expression of a corrected autosomal dominant disease-related gene.

Thus, the present disclosure provides methods for correcting autosomal dominant ocular disease in a subject, wherein the method results in functional recovery of the autosomal dominant ocular disease- related gene, comprising administering to the subject a therapeutically effective amount of autologous differentiated retinal pigment RPE cells expressing a corrected autosomal dominant ocular disease- related gene. Administration of the pharmaceutical preparations comprising autologous RPE cells that express a corrected autosomal dominant ocular disease-related gene may be effective to reduce the severity of symptoms and/or to prevent further deterioration in the patient's condition. Such administration may be effective to fully restore any vision loss or other symptoms.

For example, patient fibroblast cells can be collected from the skin biopsy and transformed into iPS cells. Dimos JT et al. (2008) Induced pluripotent stem cells generated from patients with ALS can be differentiated into motor neurons.Science 321: 1218-1221, Nature Reviews Neurology 4, 582-583 (November 2008). Luo et al., Generation of induced pluripotent stem cells from skin fibroblasts of a patient with olivopontocerebellar atrophy, Tohoku J. Exp. Med. 2012, 226(2): 151-9. The CRISPR-mediated correction can be done at this stage. The corrected cell clone can be screened and selected by RFLP assay. The corrected cell clone is then differentiated into RPE cells and tested for its RPE-specific markers (Bestrophin1, RPE65, Cellular Retinaldehyde-binding Protein, and MFRP). Well-differentiated RPE cells can be transplanted autologously back to the donor patient.

The well-differentiated autologous RPE cells described in the present disclosure may be formulated with a pharmaceutically acceptable carrier. For example, autologous RPE cells can be administered alone or as a component of a pharmaceutical formulation. The autologous RPE cells of the present disclosure can be administered in combination with one or more pharmaceutically acceptable sterile isotonic aqueous or nonaqueous solutions (e.g., balanced salt solution (BSS)), dispersions, suspensions or emulsions, or sterile powders which may be reconstituted into sterile injectable solutions or dispersions just prior to use, which may contain antioxidants, buffers, bacteriostats, solutes or suspending or thickening agents.

The autologous RPE cells of the present disclosure may be delivered in a pharmaceutically acceptable ophthalmic formulation by intraocular injection. Concentrations for injections may be at any amount that is effective and nontoxic. The pharmaceutical preparations of autologous RPE cells of the present disclosure for treatment of a patient may be formulated at doses of at least about 10⁴ cells/mL. The RPE cell preparations for treatment of a patient can be formulated at doses of at least about 10³, 10⁴, 10⁵, 10⁶, 10⁷, 10⁸, 10⁹, or 10¹⁰ RPE cells/mL.

Subjects, which may be treated according to the present invention, include all animals which may benefit from the present invention. Such subjects include mammals, preferably humans (infants, children, adolescents and/or adults), but can also be an animal such as dogs and cats, farm animals such as cows, pigs, sheep, horses, goats and the like, and laboratory animals (e.g., rats, mice, guinea pigs, and the like).

### EXAMPLES

### Surveyor assay

Surveyor mutation detection assay provides a simple and robust method to detect mutations and polymorphisms in DNA mixture. The key component of the kit is Surveyor Nuclease, a member of the CEL family of mismatch-specific nucleases derived from celery. Surveyor Nuclease recognizes and cleaves mismatches due to the presence of single nucleotide polymorphisms (SNPs) or small insertions or deletions.

Surveyor nuclease cleaves with high specificity at the 3' side of any mismatch site in both DNA strands, including all base substitutions and insertion/deletions up to at least 12 nucleotides. Surveyor nuclease technology involves four steps: (i) PCR to amplify target DNA from the cell or tissue samples underwent Cas9 nuclease-mediated cleavage (here we expect to see an nonhomogeneous or mosaic pattern of nuclease treatment on cells, some cells got cuts, some cells don't); (ii) hybridization to form heteroduplexes between affected and unaffected DNA (Because the affected DNA sequence will be different from the affected, a bulge structure resulted from the mismatch can form after denature and renature); (iii) treatment of annealed DNA with Surveyor nuclease to cleave heteroduplexes (cut the bulges); and (iv) analysis of digested DNA products using the detection/separation platform of choice, for instance, agarose gel electrophoresis. The Cas9 nuclease-mediated cleavage efficacy can be estimated by the ratio of Surveyor nuclease-digested over undigested DNA. The technology is highly sensitive, detecting rare mutants present at as low as 1 in 32 copies. Surveyor mutation assay kits are commercially available from Integrated DNA Technologies (IDT), Coraville, IA.

### RFLP analysis

Restriction fragment length polymorphism (RFLP) analysis is a technique well-known to those skilled in the art. RFLP exploits variations in homologous DNA sequences. The basic technique for the detecting RFLPs involves fragmenting a sample of DNA by a restriction enzyme, which can recognize and cut DNA wherever a specific short sequence occurs, in a process known as a restriction digest. The resulting DNA fragments are then separated by length on agarose gel electrophoresis for analysis. For the detection of donor-template replacement (also known as gene-correction), one or multiple kinds of additional restriction enzyme sites are introduced into the donor template by codon-modification, without affecting the overall length. After using PCR to amplify the target DNA sequence from tissue samples, the PCR amplicon can be evaluated by the aforementioned restriction enzyme(s) for the detection of the samples that underwent gene correction.

The following examples of specific aspects for carrying out the present invention are offered for illustrative purposes only, and are not intended to limit the scope of the present invention in any way.

### Example 1

### ChopStick AAV Gene Therapy Strategy Evaluation

The present Example outlines the strategy behind ChopStick AAV gene therapy. The approach is based on using a gene-editing enzyme with one or more unique single guide RNA (sgRNA) sequence that target both mutant and wild type forms of rhodopsin for destruction. This initial step is then followed by supplying a wild-type codon modified rhodopsin cDNA to the cells. A significant advantage of this system is that the codon modified rhodopsin cDNA is not recognizable by the sgRNA(s) and thus is resistant to the cleavage by the nuclease.

As shown in Figure 1, the "ChopStick" system described here is packaged into two recombinant AAV vectors (Fig. 1A). The first vector carries the polynucleotide sequence encoding the Cas9 enzyme (SEQ ID NO: 17), which is able to "chop" the mutant and native rhodopsin genes, while the second vector contains a polynucleotide encoding the codon-modified human rhodopsin to "stick" the normal rhodopsin back into the patient. The codon-modified engineered human rhodopsin sequence, which in this example is driven by the CBh promoter (SEQ ID NO: 10), is resistant to destruction by the gene-editing enzyme (Cas9 in this instance), and allows for the rescue of patient's phenotype. In addition to carrying a codon-modified engineered human rhodopsin sequence, the second vector carries a two single guide RNAs (sgRNA1 and sgRNA2) which act as a guide to define the target site to introduce DNA double-stranded break and thus acts as a homing device for directing the Cas9 nuclease. Each pair of recombinant AAV vectors can be used to target rhodopsin genes. The codon-modified sequence is shown in Figure 1B section II. Each sgRNA targeting site comprises four mismatches which are underlined.

AAV has a packaging capacity of 4.5~4.9Kb. Since the coding sequence of spCas9 is ~4.2Kb and the two inverted terminal repeat (ITRs) of AAV is ~0.3 Kb, there is about 0.4 Kb of space for promoter and poly-adenine termination signal. The inventors of the present disclosure used a 173 bp short CMV promoter and a 50 bp synthetic poly-adenine signal to construct the Cas AAV vector. The detailed sequence is listed and all of the components of the recombinant AAV vector are shown below: Underline and bold: ITR (SEQ ID NO: 13); Underlined: short CMV promoter; **Bold:** Flag tag (SEQ ID NO: 15) and SV40 NLS (SEQ ID NO: 16); UPPERCASE: spCas9 CDS (SEQ ID NO: 17); Framed: synthetic poly A site

(SPA)ggccttaattaggctgcgcgctcgctcgctcactgaggccgcccgggcaaagcccgggcgtcgggcgacctttggtcgccc
ggcctcagtgagcgagcgagcgcgcagagagggagtggccaactccatcactaggggttccttgtagttaatgattaacccgccatg
ctacttatctacgtagccatgctctaggaagatccactcacggggatttccaagtctccaccccattgacgtcaatgggagtttgttttggcacc
aaaatcaacgggactttccaaaatgtcgtaataaccccgccccgttgacgcaaatgggcggtaggcgtgtacggtgggaggtctatataag
cagagctcgtttagtgaaccgtgctagcatggactataaggaccacgacggagactacaaggatcatgatattgattacaaagacga
tgacgataagatggccccaaagaagaagcggaaggtcggtATCCACGGAGTCCCAGCAGCCGACAAGAA
GTACAGCATCGGCCTGGACATCGGCACCAACTCTGTGGGCTGGGCCGTGATCACCG
ACGAGTACAAGGTGCCCAGCAAGAAATTCAAGGTGCTGGGCAACACCGACCGGCAC
AGCATCAAGAAGAACCTGATCGGAGCCCTGCTGTTCGACAGCGGCGAAACAGCCGA
GGCCACCCGGCTGAAGAGAACCGCCAGAAGAAGATACACCAGACGGAAGAACCGG
ATCTGCTATCTGCAAGAGATCTTCAGCAACGAGATGGCCAAGGTGGACGACAGCTT
CTTCCACAGACTGGAAGAGTCCTTCCTGGTGGAAGAGGATAAGAAGCACGAGCGGC
ACCCCATCTTCGGCAACATCGTGGACGAGGTGGCCTACCACGAGAAGTACCCCACC
ATCTACCACCTGAGAAAGAAACTGGTGGACAGCACCGACAAGGCCGACCTGCGGCT
GATCTATCTGGCCCTGGCCCACATGATCAAGTTCCGGGGCCACTTCCTGATCGAGGG
CGACCTGAACCCCGACAACAGCGACGTGGACAAGCTGTTCATCCAGCTGGTGCAGA
CCTACAACCAGCTGTTCGAGGAAAACCCCATCAACGCCAGCGGCGTGGACGCCAAG
GCCATCCTGTCTGCCAGACTGAGCAAGAGCAGACGGCTGGAAAATCTGATCGCCCA
GCTGCCCGGCGAGAAGAAGAATGGCCTGTTCGGAAACCTGATTGCCCTGAGCCTGG
GCCTGACCCCCAACTTCAAGAGCAACTTCGACCTGGCCGAGGATGCCAAACTGCAG

CTGAGCAAGGACACCTACGACGACGACCTGGACAACCTGCTGGCCCAGATCGGCGA
CCAGTACGCCGACCTGTTTCTGGCCGCCAAGAACCTGTCCGACGCCATCCTGCTGAG
CGACATCCTGAGAGTGAACACCGAGATCACCAAGGCCCCCCTGAGCGCCTCTATGA
TCAAGAGATACGACGAGCACCACCAGGACCTGACCCTGCTGAAAGCTCTCGTGCGG
CAGCAGCTGCCTGAGAAGTACAAAGAGATTTTCTTCGACCAGAGCAAGAACGGCTA
CGCCGGCTACATTGACGGCGGAGCCAGCCAGGAAGAGTTCTACAAGTTCATCAAGC
CCATCCTGGAAAAGATGGACGGCACCGAGGAACTGCTCGTGAAGCTGAACAGAGAG
GACCTGCTGCGGAAGCAGCGGACCTTCGACAACGGCAGCATCCCCCACCAGATCCA
CCTGGGAGAGCTGCACGCCATTCTGCGGCGGCAGGAAGATTTTTACCCATTCCTGAA
GGACAACCGGGAAAAGATCGAGAAGATCCTGACCTTCCGCATCCCCTACTACGTGG
GCCCTCTGGCCAGGGGAAACAGCAGATTCGCCTGGATGACCAGAAAGAGCGAGGA
AACCATCACCCCCTGGAACTTCGAGGAAGTGGTGGACAAGGGCGCTTCCGCCCAGA
GCTTCATCGAGCGGATGACCAACTTCGATAAGAACCTGCCCAACGAGAAGGTGCTG
CCCAAGCACAGCCTGCTGTACGAGTACTTCACCGTGTATAACGAGCTGACCAAAGT
GAAATACGTGACCGAGGGAATGAGAAAGCCCGCCTTCCTGAGCGGCGAGCAGAAA
AAGGCCATCGTGGACCTGCTGTTCAAGACCAACCGGAAAGTGACCGTGAAGCAGCT
GAAAGAGGACTACTTCAAGAAAATCGAGTGCTTCGACTCCGTGGAAATCTCCGGCG
TGGAAGATCGGTTCAACGCCTCCCTGGGCACATACCACGATCTGCTGAAAATTATCA
AGGACAAGGACTTCCTGGACAATGAGGAAAACGAGGACATTCTGGAAGATATCGTG
CTGACCCTGACACTGTTTGAGGACAGAGAGATGATCGAGGAACGGCTGAAAACCTA
TGCCCACCTGTTCGACGACAAAGTGATGAAGCAGCTGAAGCGGCGGAGATACACCG
GCTGGGGCAGGCTGAGCCGGAAGCTGATCAACGGCATCCGGGACAAGCAGTCCGGC
AAGACAATCCTGGATTTCCTGAAGTCCGACGGCTTCGCCAACAGAAACTTCATGCAG
CTGATCCACGACGACAGCCTGACCTTTAAAGAGGACATCCAGAAAGCCCAGGTGTC
CGGCCAGGGCGATAGCCTGCACGAGCACATTGCCAATCTGGCCGGCAGCCCCGCCA
TTAAGAAGGGCATCCTGCAGACAGTGAAGGTGGTGGACGAGCTCGTGAAAGTGATG
GGCCGGCACAAGCCCGAGAACATCGTGATCGAAATGGCCAGAGAGAACCAGACCA
CCCAGAAGGGACAGAAGAACAGCCGCGAGAGAATGAAGCGGATCGAAGAGGGCAT
CAAAGAGCTGGGCAGCCAGATCCTGAAAGAACACCCCGTGGAAAACACCCAGCTGC

AGAACGAGAAGCTGTACCTGTACTACCTGCAGAATGGGCGGATATGTACGTGGAC
CAGGAACTGGACATCAACCGGCTGTCCGACTACGATGTGGACCATATCGTGCCTCA
GAGCTTTCTGAAGGACGACTCCATCGACAACAAGGTGCTGACCAGAAGCGACAAGA
ACCGGGGCAAGAGCGACAACGTGCCCTCCGAAGAGGTCGTGAAGAAGATGAAGAA
CTACTGGCGGCAGCTGCTGAACGCCAAGCTGATTACCCAGAGAAAGTTCGACAATC
TGACCAAGGCCGAGAGAGGCGGCCTGAGCGAACTGGATAAGGCCGGCTTCATCAAG
AGACAGCTGGTGGAAACCCGGCAGATCACAAAGCACGTGGCACAGATCCTGGACTC
CCGGATGAACACTAAGTACGACGAGAATGACAAGCTGATCCGGGAAGTGAAAGTGA
TCACCCTGAAGTCCAAGCTGGTGTCCGATTTCCGGAAGGATTTCCAGTTTTACAAAG
TGCGCGAGATCAACAACTACCACCACGCCCACGACGCCTACCTGAACGCCGTCGTG
GGAACCGCCCTGATCAAAAAGTACCCTAAGCTGGAAAGCGAGTTCGTGTACGGCGA
CTACAAGGTGTACGACGTGCGGAAGATGATCGCCAAGAGCGAGCAGGAAATCGGCA
AGGCTACCGCCAAGTACTTCTTCTACAGCAACATCATGAACTTTTTCAAGACCGAGA
TTACCCTGGCCAACGGCGAGATCCGGAAGCGGCCTCTGATCGAGACAAACGGCGAA
ACCGGGGAGATCGTGTGGGATAAGGGCCGGGATTTTGCCACCGTGCGGAAAGTGCT
GAGCATGCCCCAAGTGAATATCGTGAAAAAGACCGAGGTGCAGACAGGCGGCTTCA
GCAAAGAGTCTATCCTGCCCAAGAGGAACAGCGATAAGCTGATCGCCAGAAAGAAG
GACTGGGACCCTAAGAAGTACGGCGGCTTCGACAGCCCCACCGTGGCCTATTCTGTG
CTGGTGGTGGCCAAAGTGGAAAAGGGCAAGTCCAAGAAACTGAAGAGTGTGAAAG
AGCTGCTGGGGATCACCATCATGGAAAGAAGCAGCTTCGAGAAGAATCCCATCGAC
TTTCTGGAAGCCAAGGGCTACAAAGAAGTGAAAAAGGACCTGATCATCAAGCTGCC
TAAGTACTCCCTGTTCGAGCTGGAAAACGGCCGGAAGAGAATGCTGGCCTCTGCCG
GCGAACTGCAGAAGGGAAACGAACTGGCCCTGCCCTCCAAATATGTGAACTTCCTG
TACCTGGCCAGCCACTATGAGAAGCTGAAGGGCTCCCCCGAGGATAATGAGCAGAA
ACAGCTGTTTGTGGAACAGCACAAGCACTACCTGGACGAGATCATCGAGCAGATCA
GCGAGTTCTCCAAGAGAGTGATCCTGGCCGACGCTAATCTGGACAAAGTGCTGTCCG
CCTACAACAAGCACCGGGATAAGCCCATCAGAGAGCAGGCCGAGAATATCATCCAC
CTGTTTACCCTGACCAATCTGGGAGCCCCTGCCGCCTTCAAGTACTTTGACACCACC
ATCGACCGGAAGAGGTACACCAGCACCAAAGAGGTGCTGGACGCCACCCTGATCCA

CCAGAGCATCACCGGCCTGTACGAGACACGGATCGACCTGTCTCAGCTGGGAGGCG
ACAAAAGGCCGGCGGCCACGAAAAAGGCCGGCCAGGCAAAAAGAAAAAGTAAgtc
gacaataaaagatctttattttcattagatctgtgtgttggttttttgtgtgacgcgtgtggtaaaatcgataaggatcttcctagagcatggctacg
tagataagtagcatggcgggttaatcattaactacaaggaacccctagtgatggagttggccactccctctctgcgcgctcgctcgctca
ctgaggccgggcgaccaaaggtcgcccgacgcccgggctttgcccgggcggcctcagtgagcgagcgagcgcgcagccttaatta
acctaattcactggccgtcgttttacaacgtcgtgactgggaaaaccctggcgttacccaacttaatcgccttgcagcacatccccctttcgcc
agctggcgtaatagcgaagaggcccgcaccgatcgcccttcccaacagttgcgcagcctgaatggcgaatgggacgcgccctgtagcgg
cgcattaagcgcggcgggtgtggtggttacgcgcagcgtgaccgctacacttgccagcgccctagcgcccgctcctttcgctttcttcccttc
ctttctcgccacgttcgccggctttccccgtcaagctctaaatcgggggctccctttagggttccgatttagtgctttacggcacctcgacccca
aaaaacttgattagggtgatggttcacgtagtgggccatcgccctgatagacggtttttcgccctttgacgttggagtccacgttctttaatagtg
gactcttgttccaaactggaacaacactcaaccctatctcggtctattcttttgatttataagggattttgccgatttcggcctattggttaaaaaat
gagctgatttaacaaaaatttaacgcgaattttaacaaaatattaacgcttacaatttaggtggcacttttcggggaaatgtgcgcggaacccct
atttgtttatttttctaaatacattcaaatatgtatccgctcatgagacaataaccctgataaatgcttcaataatattgaaaaaggaagagtatgag
tattcaacatttccgtgtcgcccttattcccttttttgcggcattttgccttcctgtttttgctcacccagaaacgctggtgaaagtaaaagatgctga
agatcagttgggtgcacgagtgggttacatcgaactggatctcaacagcggtaagatccttgagagttttcgccccgaagaacgttttccaat
gatgagcacttttaaagttctgctatgtggcgcggtattatcccgtattgacgccgggcaagagcaactcggtcgccgcatacactattctcag
aatgacttggttgagtactcaccagtcacagaaaagcatcttacggatggcatgacagtaagagaattatgcagtgctgccataaccatgagt
gataacactgcggccaacttacttctgacaacgatcggaggaccgaaggagctaaccgcttttttgcacaacatgggggatcatgtaactcg
ccttgatcgttgggaaccggagctgaatgaagccataccaaacgacgagcgtgacaccacgatgcctgtagcaatggcaacaacgttgcg
caaactattaactggcgaactacttactctagcttcccggcaacaattaatagactggatggaggcggataaagttgcaggaccacttctgcg
ctcggcccttccggctggctggtttattgctgataaatctggagccggtgagcgtgggtctcgcggtatcattgcagcactggggccagatg
gtaagccctcccgtatcgtagttatctacacgacggggagtcaggcaactatggatgaacgaaatagacagatcgctgagataggtgcctc
actgattaagcattggtaactgtcagaccaagtttactcatatatactttagattgatttaaaacttcatttttaatttaaaaggatctaggtgaagat
cctttttgataatctcatgaccaaaatcccttaacgtgagttttcgttccactgagcgtcagaccccgtagaaaagatcaaaggatcttcttgaga
tcctttttttctgcgcgtaatctgctgcttgcaaacaaaaaaaccaccgctaccagcggtggtttgtttgccggatcaagagctaccaactcttttt
ccgaaggtaactggcttcagcagagcgcagataccaaatactgttcttctagtgtagccgtagttaggccaccacttcaagaactctgtagca
ccgcctacatacctcgctctgctaatcctgttaccagtggctgctgccagtggcgataagtcgtgtcttaccgggttggactcaagacgatagt
taccggataaggcgcagcggtcgggctgaacggggggttcgtgcacacagcccagcttggagcgaacgacctacaccgaactgagata

cctacagcgtgagctatgagaaagcgccacgcttcccgaagggagaaaggcggacaggtatccggtaagcggcagggtcggaacagg
agagcgcacgagggagcttccagggggaaacgcctggtatctttatagtcctgtcgggtttcgccacctctgacttgagcgtcgatttttgtg
atgctcgtcaggggggcggagcctatggaaaaacgccagcaacgcggcctttttacggttcctggccttttgctggccttttgctcacatgttc
tttcctgcgttatcccctgattctgtggataaccgtattaccgcctttgagtgagctgataccgctcgccgcagccgaacgaccgagcgcagc
gagtcagtgagcgaggaagcggaagagcgcccaatacgcaaaccgcctctccccgcgcgttggccgattcattaatgcagctggcacga
caggtttcccgactggaaagcgggcagtgagcgcaacgcaattaatgtgagttagctcactcattaggcaccccaggctttacactttatgct
tccggctcgtatgttgtgtggaattgtgagcggataacaatttcacacaggaaacagctatgaccatgattacgccagatttaattaa

In this Example, the inventors next tested above described CRISPR *RHO* strategy in human embryonic kidney cell line (HEK293FT). HEK293FT cells were transfected with Cas9 vector (pX459) (SEQ ID NO: 22) carrying either no sgRNA, sgRNA1 (SEQ ID NO: 1), sgRNA2 (SEQ ID NO: 2), or both. Ninety-six hours later, DNA was extracted, and the RHO locus was amplified and analyzed by mismatch detection SURVEYOR assay. Applying two sgRNAs together resulted in gene deletion (~30-40%), which indicated that "Chop" strategy works efficiently in mammalian cells (Fig. 2B, left, lane 4). Using one sgRNA (lanes 2 and 3) at a time does not result in change in size. Approximately 30% of the genomic DNA underwent non-homologous end joining (NHEJ) by one sgRNA, and up to 80% was edited (deletion and NHEJ) when two sgRNAs were used. Equal amounts of plasmid DNA (1 µg / 1 × 10⁵ 293FT cells) were used in each group. These findings indicate that applying two sgRNAs can destroy, remove, or degrade endogenous human *RHO* sequence more efficiently.

The inventors further tested if the "Chop" can decrease wt *RHO* gene expression. In this experiment, a bicistronic construct was transfected into HEK293 cells to express wt *RHO* cDNA (SEQ ID NO: 8) and EGFP (Fig. 3A). Both *RHO* cDNA and EGFP expression (SEQ ID NO: 21) were driven by a CMV promoter (SEQ ID NO: 20) independently and simultaneously, so that EGFP expression can be used as an internal control in western blot, which normalizes the difference in transfection efficiency and protein loading. Figure 3A also illustrates the target sites of sgRNA1 (SEQ ID NO: 1) and sgRNA2 (SEQ ID NO: 2) on this *RHO* expression vector. When HEK293FT cells were co-transfected with RHO expression vector and another vector expressing Cas9 components (pX459) carrying sgRNA1 and sgRNA2, the *RHO* protein expression level was much lower (Fig. 3B). The sg3 group is a non-specific control sgRNA. These result were further normalized with internal control, which is shown in Figure 3C. These findings indicate that, applying two sgRNAs together lowers *RHO* expression about 70%, while using single sgRNA only reduced 0-30% compared to the control group (sg3). Together, these results indicate that "Chop" strategy can significantly abolish or inactivate wt *RHO* protein expression.

### Example 2

### CRISPRlCas9-Induced

### Gene Editing in a Mouse Model of Retinitis Pigmentosa Delays Disease Progression

Next, the inventors verified the feasibility and efficacy of the CRISPR/Cas9 endonuclease system as a gene-editing treatment modality in a mouse model of RP with the dominant D190N rhodopsin mutation. In these experiments, two AAV8 vectors containing the Cas9 coding sequence and the sgRNA (SEQ ID NO: 4)/donor template marked with an *Afl*II restriction site were used. Insertion of *Afl*II restriction site allows for the identification of cells that have undergone homologous recombination (Figs. 4A-4C). Briefly, heterozygous Rho^{D190N}/⁺ was transduced into the right eye before post-natal day 5 with above described recombinant AAV8 vectors. The sgRNA targeting frequency and recombination of donor template (SEQ ID # 23) were verified by TIDE indel tracking tool (Brinkman et al. Nucleic Acid Res. 2014 Dec 16, 42(22): e168) and *Afl*II enzyme digestion (Fig. 4). About 50% of cells underwent NHEJ (mostly are 1 bp insertion), and about 10% of cells incorporated donor template successfully. Structural preservation was assessed by H&E staining, and retinal function rescue was assessed by electroretinography (ERG) at 3 months of age (Figs. 5A and 5B). In the Rho^{D190N}/⁺ 3 month old mice, treated eyes showed greater photoreceptor survival than did eyes that did not receive AAV injection (Fig. 5A). Furthermore, retinal function as measured by ERG was also increased in treated eyes compared with control eyes at 3 months of age (Fig. 5B). Collectively, these results demonstrate that CRISPR-Cas9 gene editing described in the present disclosure can be used to *in vivo* correct the phenotype of RP. The codon-modified donor sequence is shown as follows: Underline and bold: homologous arm; **Bold**: AflII site; Underlined: 5 codon-modified wobble nucleotides;

tcccttaaccaccgaaggcagggcagcaggctagtggagcagagctgcgtggtcaagtggcagggagcttaagaatcgtccaag
ggcggagaccagtaagtctcattaggtgatggggccagcaggtaaaagccattcatGcttatgtccagctgggcgtgtgttctcttc
ctgttttatcatcccttgcgctgaccatcaggtacatccctgagggcatgcaatgttcatgcgggattgactattataccttaagccgg
aggtcaacaacgaatcctttgtcatctacatgttcgtggtccacttcaccattcctatgatcgtcatcttcttctgctatgggcagctggt
cttcacagtcaaggaggtatgagcaggg.

### Example 3

### CRISPR-Mediated Humanized Exon 1 at the Mouse Rho Locus

The inventors of the present disclosure next tested the ability to replace mouse Rho locus with wild-type (wt) (SEQ ID NO: 24) or mutant human (h) (SEQ ID NO: 25) *RHO* exon 1 in mouse embryonic stem (ES) cells (Fig. 6). Briefly, ES cells were co-transfected (via electroporation) with the Cas9 expression vector carrying a *Rho* exon 1-specific sgRNA (sgRNA-Rho Exon 1, SEQ ID NO: 5) targeting mouse *Rho* exon 1) and a targeting vector carrying with human RHO donor template, which contained a sequence of hRHO exon 1 flanked with ~750 bp homologous arm on each side (Fig. 6A). Human *RHO* donor template is expected to replace mouse exon 1 and confer resistance to sgRNA-Rho Exon 1. Seven days after electroporation, ES clones were picked and DNA was extracted and amplified with screening primers. Two out of 96 clones were detected with replacement of human exon 1 by RFLP analysis (Fig. 6B). As shown in Fig. 6C, sequence electropherograms of amplicons show perfect fused human and mouse sequence of one targeted ES clone (lane 2, Fig 6B). The correct targeted clones can be further used to produce the humanized RHO exon 1 mouse model. This patient-specific humanized mouse system enables the inventors to test various sgRNAs that may be used for targeting human genomic sequence for ChopStick strategy in vivo. The advantages of using these mouse models also enables the validation of the "ChopStick" efficacy and safety via functional evaluation methods like visual function, imaging of rescued tissue in live animals for long term observations. The sgRNA sequence is listed above and the donor template sequence is listed below: Underline and bold: homologous arm; UPPERCASE: human *RHO* exon 1

atgctcacctgaataacctggcagcctgctccctcatgcagggaccacgtcctgctgcacccagcaggccatcccgtctccatagcc
catggtcatccctccctggacaggaatgtgtctcctccccgggctgagtcttgctcaagctagaagcactccgaacagggttatggg
cgcctcctccatctcccaagtggctggcttatgaatgtttaatgtacatgtgagtgaacaaattccaattgaacgcaacaaatagtta
tcgagccgctgagccgggggcgggggtgtgagactggaggcgatggacggagctgacggcacacacagctcagatctgtca
agtgagccattgtcagggcttggggactggataagtcagggggtctcctgggaagagatgggataggtgagttcaggaggagac
attgtcaactggagccatgtggagaagtgaatttagggcccaaaggttccagtcgcagcctgaggccaccagactgacatgggga
ggaattcccagaggactctggggcagacaagatgagacaccctttcctttctttacctaagggcctccacccgatgtcaccttggcc
cctctgcaagccaattaggccccggtggcagcagtgggattagcgttagtatgatatctcgcggatgctgaatcagcctctggctta
gggagagaaggtcactttataagggtctgggggggtcagtgcctggagttgcgctgtgggagcGAGTCATCCAGCTG
GAGCCCTGAGTGGCTGAGCTCAGGCCTTCGCAGCATTCTTGGGTGGGAGCAGCCAC
GGGTCAGCCACAAGGGCCACAGCCATGAATGGCACAGAAGGCCCTAACTTCTACGT
GCCCTTCTCCAATGCGACGGGTGTGGTACGCAGCCCCTTCGAGTACCCACAGTACTA
CCTGGCTGAGCCATGGCAGTTCTCCATGCTGGCCGCCTACATGTTTCTGCTGATCGTG
CTGGGCTTCCCCATCAACTTCCTCACGCTCTACGTCACCGTCCAGCACAAGAAGCTG
CGCACGCCTCTCAACTACATCCTGCTCAACCTAGCCGTGGCTGACCTCTTCATGGTC
CTAGGTGGCTTCACCAGCACCCTCTACACCTCTCTGCATGGATACTTCGTCTTCGGGC
CCACAGGATGCAATTTGGAGGGCTTCTTTGCCACCCTGGGCGgtatgagcagagagactggggc
gggggggtgtagcatgggagccaaggggccacgaaagggcctgggagggtctgcagcttacttgagtctctttaattggtctcatc
taaaggcccagcttattcattggcaaacactgtgaccctgagctaggctgctgttgagagcaggcacggaacattcatctatctcat
cttgagcaatgcaagaaacatgggttcagagaggccaaggactcaccgaggagtcacagagtgtgggggtgtcctctgaggcag
ctgagctggggcacacacagactgagcaccaggagtgagctctagcttttttttttctatgtgtcttttctaaaagacacataggttta
ggactgtccctggtccaggtaagaactggttcagtaaacttgtacatctcactgcctggccagccctgtcagcttccaccagagtgcg
tgcactacacacccggcatctcaaaggattcattcctatctttcctatctttggagtgaggcacagtctcacgtagtccagtccagact
ggccttaaattctgcagctgaggatgtacttaaacttgtcatcctcctgccccagcctctcaagtgctgtgatcacaggcacggacca
ctatgctacgccaggtgtttccaaacattttctctcccttaactggaaggtcaatgaggctctttcgagaagcaacagagcc

### Example 4

### Repair of Mouse Pde6a D670G Allele in Stem Cells

Mutations in genes encoding subunits of the rod-specific enzyme, cyclic guanosine monophosphate (cGMP) phosphodiesterase 6 (PDE6A and PDE6B), are responsible for approximately 72,000 cases of RP worldwide each year, making therapeutic modeling highly relevant to developing mechanisms based therapies. In the present Example, the inventors used the CRISPR/ Cas9 gene editing system to correct photoreceptor gene mutations in mouse ES cells.

For these studies, the inventors used ES cells isolated from Pde6a^{D670G/D670G} mouse model (Wert KJ et al. Hum Mol Genet. 2013 Feb 1;22(3):558-67; Wert KJ et al. J Vis Exp. 2012 Nov 25;(69)). As shown in Fig. 7A, a donor construct (SEQ ID NO: 26) used in this Example contains two modifications: 1) a Pde6a-codon modification which creates an additional SphI site upstream from the D670G codon, where SphI enzyme-digestion can identify ES cells that underwent CRISPR-mediated homologous recombination; and 2) eight wobble base pairs were introduced, which make the donor template unrecognizable to sgRNA (SEQ ID NO: 6) and thus resistant to Cas9, and which resulted in the change of the mutant amino acid sequence to that of the wt amino acid sequence. Thus, upon Cas9 cutting and homologous recombination, the endogenous mutant allele is replaced (i.e., repaired). In Figure 7A, triangle indicates the sgRNA target site while the two arrows represent the primer pairs used for PCR amplification. As shown in Figure 7B, amplicons generated from recombined cells were 303 bp and 402 bp while the unedited amplicon is 705 bp. Moreover, using direct sequencing of genomic DNA from a target clone, the inventors confirmed predicted replacement of the D670G exon with donor template (Fig. 7C). This is an example where there is no sgRNA target site on the mutation site, but researchers can still design sgRNA nearby and successfully replace mutant allele through homologous recombination.

Thus, in this Example, the inventors verified the ability of the CRISPR/Cas9 system to edit the mouse *PDE6a* locus and rescue photoreceptors.

### Example 5

### CRISPR/Cas9-Induced In Vivo Gene Editing in Pde6a^{D670G} /Pde6a^{D670G} Mouse Model

The inventors have verified the use of CRISPR/Cas9 system to edit the mouse *Rho* locus and rescue photoreceptors (Example 2). Furthermore, as shown in Example 4, the inventors were able to repair mouse Pde6a D670G allele in ES cells. Next, the inventors will perform *in vivo* experiments, where post-natal day (P) 5 *Pde6a^{D670G}* /*Pde6a^{D670G}* mice will receive subretinal transductions of both recombinant AAV8-Cas9 and AAV8-sgRNA with the codon-optimized Cas9 resistant donor DNA (validated in Example 4) into one eye. In control animals, one eye will be transduced with an empty AAV8 vector or AAV8-Cas9 as negative control.

The inventors will next perform quantitative validation of recombination and correction of one of the *Pde6a^{D670G}* allele in homozygous mutant. Briefly, one month after injection (before degeneration onset), retinas will be dissected, DNA isolated, PCR performed, and SphI restriction site verified (using RFLP). PCR samples will be run in triplicate. At 3 weeks of age, retinas from 3 mice will be collected, and Pde6a levels quantified by immunoblotting, as described.

To quantitatively assess photoreceptor function and survival, the inventors will perform quantitative AF of outer segment thinning on SD-OCT, ERG, and rod-cone density at 8, 16, and 24 weeks (n = 36 total animals) using previously published techniques (Woodruff et al. J Neurosci. 2008 Feb 27;28(9):2064-74; Janisch et al. Biochem Biophys Res Commun 390, 1149-1153 (2009); Tsang et al. Science. 1996 May 17;272(5264):1026-9; Davis et al. Invest Ophthalmol Vis Sci. 2008 Nov;49(11):5067-76.) All measurements will be performed on both eyes.

The inventors will also determine the efficacy of PDE function. As a key biochemical indicator of rescue, the inventors will measure whether total cGMP levels and PDE activity from light- and dark-adapted retinas are restored. Three additional sample right eyes, treated at P18, P21, P28, and P35, and control fellow left eyes of Pde6a^{D670G/D670G} will be assayed. GUCY2E (guanylate cyclase) should remain stable for all experiments and will be determined as previously described (Tsang et al. Science. 1996 May 17; 272(5264): 1026-1029; Science. 1998 Oct 2;282(5386):117-21).

### Example 6

### Efficacy and frequency of homologous recombination vs. non-homologous end joining (NEHJ) for editing PD6A in patient specific stem cells

In this Example, the inventors will verify that the AAV2-Cas9 system can edit the human *PDE6A* locus. In this aim, 0.25×10⁶ patient iPS in a 6-well matrigel coated plate (in NutriStem XF/FF Culture media, Stemgent, Cambridge) will be co-transduced with AAV2-Cas9 and AAV2 vector-donor template mix (MOI: 2000) to repair *PDE6A.* After 48 hours, iPS will be passaged with Accutase onto regular 10-cm matrigel coated culture dishes.

Next, 1000 *PDE6A^{R102C}* /*PDE6A^{S303C}* patient iPS clones will be picked, and DNA isolated for PCR; (Primers: forward: GCAGACTGCAAAACTGCCAT; reverse: TGTCACCAGCCTTGTCTTGG). PCR products will be cut with *BsiWI* to identify clones that have undergone homologous recombination. After *BsiWI* digestion, the amplicon generated from iPS that underwent homologous recombination gives bands at 271 bp and 380 bp, compared to the parental sequence, which gives only one band at 651 bp.

To determine the percentage of clones that have undergone NHEJ (as opposed to those that underwent homology-directed repair), DNA will be analyzed from clones without the *BsiWI* site (i.e., not transduced, transduced off-target or NHEJ), and the frequency of the disruption of the PDE6A allele determined. DNA will be analyzed by SURVEYOR mismatch detection assay and positive DNA samples will be subjected to subcloned into plasmid vectors such as pCR^{™}4Blunt-TOPO^{®} vector and then send for Sanger sequencing. In addition, the assessment of off-targeting in iPS and live mice are prerequisites before application to humans. Off-target sites will be analyzed by full-genome sequencing using Illumina next-generation sequencing.

The inventors anticipate a much higher rate of homologous recombination mediated by AAV8 in photoreceptors *in vivo,* compared to patient iPS. This is because AAV8 introduces sgRNA into photoreceptors at a much higher frequency than transduction into iPS. AAV8 introduces into photoreceptors ~10,000 copies of the sgRNA and Cas9, as opposed to both lipofection and electroporation, which generally introduce a single-copy DNA into each cell.

The percentage of transduced clones that undergo NHEJ is likely to be higher than those undergoing homologous recombination-approximately 10% vs. 1%, respectively, with a 90-bp donor template in human- induced pluripotent stem cells.

### Example 7

### Use of CRISPR system to replace mutant allele R345W in iPS cells isolated from Doyne Honeycomb patient

Doyne Honeycomb retinal dystrophy (DHRD) is an inherited disease that affects the eyes and causes vision loss. It is characterized by small, round, white spots (drusen) that accumulate around the retinal pigment epithelium. Over time, drusen may grow and come together, creating a honeycomb pattern. It usually begins in early adulthood, but the age of onset varies. The degree of vision loss also varies. DHRD is caused by R345W mutations in the EFEMP1 gene, which are inherited in an autosomal dominant manner.

In this Example, the inventors used CRISPR components and a donor template (SEQ ID NO: SEQ ID 27) to correct the R345W mutation in the iPS cells derived from Doyne Honeycomb patient fibroblast (Figure 8A). The resulting iPS cells comprise wild type EFEMP1 sequence with codon-modification, which confers resistance to further cutting by the Cas9. These cells can be used for autologous transplantation after the differentiation into RPE cells for the cure of DHRD

Briefly, the inventors collected the iPS derived from the DHRD patient. Cas9 protein and sgRNA-EFEMP1 (SEQ ID NO: 3) (Figure 8B) were mixed and co-transduced with donor template in the form of single strand oligodeoxynucleotide (ssODN) (sequence: tagttagtaaactctttgaccctacatctctacagatataaatgagtgtgagaccacaaaCgaGtgcCgggaggatgaaatgtgttggaatt atcatggcggcttccgttgttatccacgaaatcctt) into iPS cells by nucleofection. The donor template is codon-modified to prevent repeating recognizing and cutting by the CRISPR components. The colony with corrected sequence was confirmed by RFLP assay with the additional ScrFI restriction site. The genotype of the iPS cell is further confirmed by sequencing (Figure 8C).

This experiment provides the evidence that the "Chop" strategy has potential to treat autosomal dominant diseases other than autosomal dominant retinitis pigmentosa.

Table 2 provides sgRNA sequences, donor-template modified sequences, and additional experiments used in the Examples of the present disclosure.

**Table 2.**

| **Sequence ID Number** | **Sequence** | **Species** |
|---|---|---|
| SEQ ID NO: 1 (sgRNA1) | GGACGGTGACGTAGAGCGTG | *Homo sapiens* |
| SEQ ID NO: 2 (sgRNA2) | GACGAAGTATCCATGCAGAG | *Homo sapiens* |
| SEQ ID NO: 3 (sgRNA-EFEMP1) | TGAGACCACAAATGAATGCT | *Homo sapiens* |
| SEQ ID NO: 4 (sgRNA-D190N) | AACTACTACACACTCAAGCCTG | *Mus musculus* |
| SEQ ID NO: 5 (sgRNA-Rho Exon 1) | GTAGTACTGCGGCTGCTCGA | *Mus musculus* |
| SEQ ID NO: 6 (sgRNA-*Pde6a*^{D670G}) | GCTCATGCTGCCGGCGATTC | *Mus musculus* |
| SEQ ID NO: 7 (sgRNA-Y1) | GGTTTTGGACAATGGAACCG | *Drosophila melanogaster* |
| SEQ ID NO: 8 (wt *RHO* cDNA) | atgaatggcacagaaggccctaacttctacgtgcccttctccaatgcga cgggtgtggtacgcagccccttcgagtacccacagtactacctggctg agccatggcagttctccatgctggccgcctacatgtttctgctgatcgtg ctgggcttccccatcaacttcctcacgctctacgtcaccgtccagcacaa gaagctgcgcacgcctctcaactacatcctgctcaacctagccgtggct gacctcttcatggtcctaggtggcttcaccagcaccctctacacctctctg catggatacttcgtcttcgggcccacaggatgcaatttggagggcttcttt gccaccctgggcggtgaaattgccctgtggtccttggtggtcctggcca tcgagcggtacgtggtggtgtgtaagcccatgagcaacttccgcttcgg ggagaaccatgccatcatgggcgttgccttcacctgggtcatggcgctg gcctgcgccgcacccccactcgccggctggtccaggtacatccccga gggcctgcagtgctcgtgtggaatcgactactacacgctcaagccgga ggtcaacaacgagtcttttgtcatctacatgttcgtggtccacttcaccatc cccatgattatcatctttttctgctatgggcagctcgtcttcaccgtcaagg aggccgctgcccagcagcaggagtcagccaccacacagaaggcag agaaggaggtcacccgcatggtcatcatcatggtcatcgctttcctgatc tgctgggtgccctacgccagcgtggcattctacatcttcacccaccagg gctccaacttcggtcccatcttcatgaccatcccagcgttctttgccaaga gcgccgccatctacaaccctgtcatctatatcatgatgaacaagcagttc cggaactgcatgctcaccaccatctgctgcggcaagaacccactgggt gacgatgaggcctctgctaccgtgtccaagacggagacgagccaggt ggccccggcctaa | *Homo sapiens* |
| SEQ ID NO: 9 ( cm*RHO* cDNA) | atgaatggcacagaaggccctaacttctacgtgcccttctccaatgcga cgggtgtggtacgcagccccttcgagtacccacagtactacctggctg agccatggcagttctccatgctggccgcctacatgtttctgctgatcgtg ctgggcttccccatcaactttcttacactgtacgtcaccgtccagcacaa gaagctgcgcacgcctctcaactacatcctgctcaacctagccgtggct gacctcttcatggtcctaggtggcttcaccagcaccctctacacgtcgct tcacggatatttcgtcttcgggcccacaggatgcaatttggagggcttctt tgccaccctgggcggtgaaattgccctgtggtccttggtggtcctggcc atcgagcggtacgtggtggtgtgtaagcccatgagcaacttccgcttcg gggagaaccatgccatcatgggcgttgccttcacctgggtcatggcgct ggcctgcgccgcacccccactcgccggctggtccaggtacatccccg agggcctgcagtgctcgtgtggaatcgactactacacgctcaagccgg aggtcaacaacgagtcttttgtcatctacatgttcgtggtccacttcaccat ccccatgattatcatctttttctgctatgggcagctcgtcttcaccgtcaag gaggccgctgcccagcagcaggagtcagccaccacacagaaggca gagaaggaggtcacccgcatggtcatcatcatggtcatcgctttcctgat ctgctgggtgccctacgccagcgtggcattctacatcttcacccaccag ggctccaacttcggtcccatcttcatgaccatcccagcgttctttgccaag agcgccgccatctacaaccctgtcatctatatcatgatgaacaagcagtt ccggaactgcatgctcaccaccatctgctgcggcaagaacccactggg tgacgatgaggcctctgctaccgtgtccaagacggagacgagccagg tggccccggcc | *Homo sapiens* |
| SEQ ID NO: 10 (CBh promoter) | cgttacataacttacggtaaatggcccgcctggctgaccgcccaacga cccccgcccattgacgtcaatagtaacgccaatagggactttccattga cgtcaatgggtggagtatttacggtaaactgcccacttggcagtacatca agtgtatcatatgccaagtacgccccctattgacgtcaatgacggtaaat ggcccgcctggcattgtgcccagtacatgaccttatgggactttcctactt ggcagtacatctacgtattagtcatcgctattaccatggtcgaggtgagc cccacgttctgcttcactctccccatctcccccccctccccacccccaatt ttgtatttatttattttttaattattttgtgcagcgatgggggcgggggggg ggggggggcgcgccaggcggggcggggcggggcgaggggcgg ggcggggcgaggcggagaggtgcggcggcagccaatcagagcgg cgcgctccgaaagtttccttttatggcgaggcggcggcggcggcggc cctataaaaagcgaagcgcgcggcgggcgggagtcgctgcgacgct gccttcgccccgtgccccgctccgccgccgcctcgcgccgcccgccc cggctctgactgaccgcgttactcccacaggtgagcgggcgggacgg cccttctcctccgggctgtaattagctgagcaagaggtaagggtttaag ggatggttggttggtggggtattaatgtttaattacctggagcacctgcct gaaatcactttttttcaggtt | *Gallus gallus* |
| SEQ ID NO: 11 (BGH poly-A) | taagagctcgctgatcagcctcgactgtgccttctagttgccagccatct gttgtttgcccctcccccgtgccttccttgaccctggaaggtgccactcc cactgtcctttcctaataaaatgaggaaattgcatcgcattgtctgagtag gtgtcattctattctggggggtggggtggggcaggacagcaaggggg aggattgggaagagaatagcaggcatgctgggga | *Bos taurus* |
| SEQ ID NO: 12 (U6 promoter) | gagggcctatttcccatgattccttcatatttgcatatacgatacaaggctg ttagagagataattggaattaatttgactgtaaacacaaagatattagtac aaaatacgtgacgtagaaagtaataatttcttgggtagtttgcagttttaaa attatgttttaaaatggactatcatatgcttaccgtaacttgaaagtatttcg atttcttggctttatatatcttgtggaaaggacgaaacacc | *Homo sapiens* |
| SEQ ID NO: 13 (ITR) | cgcgctcgctcgctcactgaggccgcccgggcaaagcccgggcgtc gggcgacctttggtcgcccggcctcagtgagcgagcgagcgcgcag agagggagtggccaactccatcactaggggttccttgt | *Adeno-associated virus-2* |
| SEQ ID NO: 14 (sCMV promoter) | actcacggggatttccaagtctccaccccattgacgtcaatgggagtttg ttttggcaccaaaatcaacgggactttccaaaatgtcgtaataaccccgc cccgttgacgcaaatgggcggtaggcgtgtacggtgggaggtctatat aagcagagctcgtttagtgaaccgt | *Human herpesvirus 5* |
| SEQ ID NO: 15 (3xFlag) | GACTATAAGGACCACGACGGAGACTACAA GGATCATGATATTGATTACAAAGACGATGA CGATAAG | |
| SEQ ID NO: 16 (SV40-NLS) | CCAAAGAAGAAGCGGAAGGTC | *Simian virus 40* |
| SEQ ID No. 17 (Cas9) | gacaagaagtacagcatcggcctggac atcggcaccaactctgtgggctgggcc gtgatcaccgacgagtacaaggtgccc agcaagaaattcaaggtgctgggcaac accgaccggcacagcatcaagaagaa cctgatcggagccctgctgttcgacagc ggcgaaacagccgaggccacccggct gaagagaaccgccagaagaagataca ccagacggaagaaccggatctgctatct gcaagagatcttcagcaacgagatggc caaggtggacgacagcttcttccacaga ctggaagagtccttcctggtggaagagg ataagaagcacgagcggcaccccatct tcggcaacatcgtggacgaggtggcct accacgagaagtaccccaccatctacc acctgagaaagaaactggtggacagca ccgacaaggccgacctgcggctgatct atctggccctggcccacatgatcaagttc cggggccacttcctgatcgagggcgac ctgaaccccgacaacagcgacgtggac aagctgttcatccagctggtgcagacct acaaccagctgttcgaggaaaacccat caacgccagcggcgtggacgccaagg ccatcctgtctgccagactgagcaagag cagacggctggaaaatctgatcgccca gctgcccggcgagaagaagaatggcct gttcggaaacctgattgccctgagcctg ggcctgacccccaacttcaagagcaact tcgacctggccgaggatgccaaactgc agctgagcaaggacacctacgacgac gacctggacaacctgctggcccagatc ggcgaccagtacgccgacctgtttctgg ccgccaagaacctgtccgacgccatcct gctgagcgacatcctgagagtgaacac cgagatcaccaaggcccccctgagcgc ctctatgatcaagagatacgacgagcac caccaggacctgaccctgctgaaagct ctcgtgcggcagcagctgcctgagaag tacaaagagattttcttcgaccagagcaa gaacggctacgccggctacattgacgg cggagccagccaggaagagttctacaa gttcatcaagcccatcctggaaaagatg gacggcaccgaggaactgctcgtgaag ctgaacagagaggacctgctgcggaag cagcggaccttcgacaacggcagcatc ccccaccagatccacctgggagagctg | *Streptococcus pyogenes* |
| | cacgccattctgcggcggcaggaagat ttttacccattcctgaaggacaaccggga aaagatcgagaagatcctgaccttccgc atcccctactacgtgggccctctggcca ggggaaacagcagattcgcctggatga ccagaaagagcgaggaaaccatcacc ccctggaacttcgaggaagtggtggac aagggcgcttccgcccagagcttcatcg agcggatgaccaacttcgataagaacct gcccaacgagaaggtgctgcccaagc acagcctgctgtacgagtacttcaccgt gtataacgagctgaccaaagtgaaatac gtgaccgagggaatgagaaagcccgc cttcctgagcggcgagcagaaaaaggc catcgtggacctgctgttcaagaccaac cggaaagtgaccgtgaagcagctgaaa gaggactacttcaagaaaatcgagtgct tcgactccgtggaaatctccggcgtgga agatcggttcaacgcctccctgggcaca taccacgatctgctgaaaattatcaagga caaggacttcctggacaatgaggaaaa cgaggacattctggaagatatcgtgctg accctgacactgtttgaggacagagaga tgatcgaggaacggctgaaaacctatgc ccacctgttcgacgacaaagtgatgaag cagctgaagcggcggagatacaccgg ctggggcaggctgagccggaagctgat caacggcatccgggacaagcagtccg gcaagacaatcctggatttcctgaagtcc gacggcttcgccaacagaaacttcatgc agctgatccacgacgacagcctgacctt taaagaggacatccagaaagcccaggt gtccggccagggcgatagcctgcacga gcacattgccaatctggccggcagccc cgccattaagaagggcatcctgcagac agtgaaggtggtggacgagctcgtgaa agtgatgggccggcacaagcccgaga acatcgtgatcgaaatggccagagaga accagaccacccagaagggacagaag aacagccgcgagagaatgaagcggat cgaagagggcatcaaagagctgggca gccagatcctgaaagaacaccccgtgg aaaacacccagctgcagaacgagaag ctgtacctgtactacctgcagaatgggc gggatatgtacgtggaccaggaactgg acatcaaccggctgtccgactacgatgt | |
| | ggaccatatcgtgcctcagagctttctga aggacgactccatcgacaacaaggtgc tgaccagaagcgacaagaaccggggc aagagcgacaacgtgccctccgaaga ggtcgtgaagaagatgaagaactactg gcggcagctgctgaacgccaagctgat tacccagagaaagttcgacaatctgacc aaggccgagagaggcggcctgagcga actggataaggccggcttcatcaagaga cagctggtggaaacccggcagatcaca aagcacgtggcacagatcctggactcc cggatgaacactaagtacgacgagaat gacaagctgatccgggaagtgaaagtg atcaccctgaagtccaagctggtgtccg atttccggaaggatttccagttttacaaag tgcgcgagatcaacaactaccaccacg cccacgacgcctacctgaacgccgtcg tgggaaccgccctgatcaaaaagtacc ctaagctggaaagcgagttcgtgtacgg cgactacaaggtgtacgacgtgcggaa gatgatcgccaagagcgagcaggaaat cggcaaggctaccgccaagtacttcttct acagcaacatcatgaactttttcaagacc gagattaccctggccaacggcgagatc cggaagcggcctctgatcgagacaaac ggcgaaaccggggagatcgtgtgggat aagggccgggattttgccaccgtgcgg aaagtgctgagcatgccccaagtgaata tcgtgaaaaagaccgaggtgcagacag gcggcttcagcaaagagtctatcctgcc caagaggaacagcgataagctgatcgc cagaaagaaggactgggaccctaaga agtacggcggcttcgacagccccaccg tggcctattctgtgctggtggtggccaaa gtggaaaagggcaagtccaagaaactg aagagtgtgaaagagctgctggggatc accatcatggaaagaagcagcttcgag aagaatcccatcgactttctggaagcca agggctacaaagaagtgaaaaaggac ctgatcatcaagctgcctaagtactccct gttcgagctggaaaacggccggaaga gaatgctggcctctgccggcgaactgc agaagggaaacgaactggccctgccct ccaaatatgtgaacttcctgtacctggcc agccactatgagaagctgaagggctcc cccgaggataatgagcagaaacagctg | |
| | tttgtggaacagcacaagcactacctgg acgagatcatcgagcagatcagcgagtt ctccaagagagtgatcctggccgacgct aatctggacaaagtgctgtccgcctaca acaagcaccgggataagcccatcagag agcaggccgagaatatcatccacctgttt accctgaccaatctgggagcccctgcc gccttcaagtactttgacaccaccatcga ccggaagaggtacaccagcaccaaag aggtgctggacgccaccctgatccacc agagcatcaccggcctgtacgagacac ggatcgacctgtctcagctgggaggcg ac | |
| SEQ ID No. 18 (nucleoplasmin NLS) | aaaaggccggcggccacgaaaaaggc cggccaggcaaaaaagaaaaag | *Xenopus laevis* |
| SEQ ID No. 19 (synthetic poly A) | aataaaagatctttattttcattagatctgt gtgttggtttttttgtgtg | *Oryctolagus cuniculus* |
| SEQ ID No. 20 (CMV promoter) | cgttacataacttacggtaaatggcccgc ctggctgaccgcccaacgacccccgcc cattgacgtcaataatgacgtatgttccc atagtaacgccaatagggactttccattg acgtcaatgggtggagtatttacggtaaa ctgcccacttggcagtacatcaagtgtat catatgccaagtacgccccctattgacgt caatgacggtaaatggcccgcctggcat tatgcccagtacatgaccttatgggacttt cctacttggcagtacatctacgtattagtc atcgctattaccatggtgatgcggttttgg cagtacatcaatgggcgtggatagcggt ttgactcacggggatttccaagtctccac cccattgacgtcaatgggagtttgttttgg caccaaaatcaacgggactttccaaaat gtcgtaacaactccgccccattgacgca aatgggcggtaggcgtgtacggtggga ggtctatataagcagagct | *Human herpesvirus 5* |
| S EQ ID No. 21 (EGFP) | atggtgagcaagggcgaggagctgttc accggggtggtgcccatcctggtcgag ctggacggcgacgtaaacggccacaa gttcagcgtgtccggcgagggcgagg gcgatgccacctacggcaagctgaccc tgaagttcatctgcaccaccggcaagct gcccgtgccctggcccaccctcgtgac caccctgacctacggcgtgcagtgcttc agccgctaccccgaccacatgaagcag cacgacttcttcaagtccgccatgcccg aaggctacgtccaggagcgcaccatctt cttcaaggacgacggcaactacaagac ccgcgccgaggtgaagttcgagggcg acaccctggtgaaccgcatcgagctga agggcatcgacttcaaggaggacggca acatcctggggcacaagctggagtaca actacaacagccacaacgtctatatcatg gccgacaagcagaagaacggcatcaa ggtgaacttcaagatccgccacaacatc gaggacggcagcgtgcagctcgccga ccactaccagcagaacacccccatcgg cgacggccccgtgctgctgcccgacaa ccactacctgagcacccagtccgccctg agcaaagaccccaacgagaagcgcga tcacatggtcctgctggagttcgtgaccg ccgccgggatcactctcggcatggacg agctgtacaagtaa | *Aequorea victoria* |
| SEQ ID No. 22 [pSpCas9(BB)-2A-puro(pX459)] | gagggcctatttcccatgattccttcatatt tgcatatacgatacaaggctgttagaga gataattggaattaatttgactgtaaacac aaagatattagtacaaaatacgtgacgta gaaagtaataatttcttgggtagtttgcag ttttaaaattatgttttaaaatggactatcat atgcttaccgtaacttgaaagtatttcgatt tcttggctttatatatcttGTGGAAA GGACGAAACACCggGTCT TCgaGAAGACctgttttagagcta GAAAtagcaagttaaaataaggcta gtccgttatcaacttgaaaaagtggcacc gagtcggtgcTTTTTTgttttagagc tagaaatagcaagttaaaataaggctagt ccgtTTTTagcgcgtgcgccaattct gcagacaaatggctctagaggtacccgt tacataacttacggtaaatggcccgcctg gctgaccgcccaacgacccccgcccat tgacgtcaatagtaacgccaatagggac tttccattgacgtcaatgggtggagtattt acggtaaactgcccacttggcagtacat caagtgtatcatatgccaagtacgcccc ctattgacgtcaatgacggtaaatggcc cgcctggcattGtgcccagtacatgacc ttatgggactttcctacttggcagtacatc tacgtattagtcatcgctattaccatggtc gaggtgagccccacgttctgcttcactct ccccatctcccccccctccccaccccca attttgtatttatttattttttaattattttgtgc agcgatgggggcgggggggggggggg gggcgcgcgccaggcggggcggggc ggggcgaggggcggggcggggcga ggcggagaggtgcggcggcagccaat cagagcggcgcgctccgaaagtttcctt ttatggcgaggcggcggcggcggcgg ccctataaaaagcgaagcgcgcggcg ggcgggagtcgctgcgacgctgccttc gccccgtgccccgctccgccgccgcct cgcgccgcccgccccggctctgactga ccgcgttactcccacaggtgagcgggc gggacggcccttctcctccgggctgtaa ttagctgagcaagaggtaagggtttaag ggatggttggttggtggggtattaatgttt aattacctggagcacctgcctgaaatca ctttttttcaggttGGaccggtgccacc ATGGACTATAAGGACCA | *Streptococcus pyogenes* |
| | CGACGGAGACTACAAGG ATCATGATATTGATTAC AAAGACGATGACGATAA GATGGCCCCAAAGAAGA AGCGGAAGGTCGGTATC CACGGAGTCCCAGCAGC CGACAAGAAGTACAGCA TCGGCCTGGACATCGGC ACCAACTCTGTGGGCTG GGCCGTGATCACCGACG AGTACAAGGTGCCCAGC AAGAAATTCAAGGTGCT GGGCAACACCGACCGGC ACAGCATCAAGAAGAAC CTGATCGGAGCCCTGCT GTTCGACAGCGGCGAAA CAGCCGAGGCCACCCGG CTGAAGAGAACCGCCAG AAGAAGATACACCAGAC GGAAGAACCGGATCTGC TATCTGCAAGAGATCTT CAGCAACGAGATGGCCA AGGTGGACGACAGCTTC TTCCACAGACTGGAAGA GTCCTTCCTGGTGGAAG AGGATAAGAAGCACGAG CGGCACCCCATCTTCGG CAACATCGTGGACGAGG TGGCCTACCACGAGAAG TACCCCACCATCTACCA CCTGAGAAAGAAACTGG TGGACAGCACCGACAAG GCCGACCTGCGGCTGAT CTATCTGGCCCTGGCCC ACATGATCAAGTTCCGG GGCCACTTCCTGATCGA GGGCGACCTGAACCCCG ACAACAGCGACGTGGAC AAGCTGTTCATCCAGCT GGTGCAGACCTACAACC AGCTGTTCGAGGAAAAC CCCATCAACGCCAGCGG CGTGGACGCCAAGGCCA TCCTGTCTGCCAGACTG AGCAAGAGCAGACGGCT GGAAAATCTGATCGCCC | |
| | AGCTGCCCGGCGAGAAG AAGAATGGCCTGTTCGG AAACCTGATTGCCCTGA GCCTGGGCCTGACCCCC AACTTCAAGAGCAACTT CGACCTGGCCGAGGATG CCAAACTGCAGCTGAGC AAGGACACCTACGACGA CGACCTGGACAACCTGC TGGCCCAGATCGGCGAC CAGTACGCCGACCTGTT TCTGGCCGCCAAGAACC TGTCCGACGCCATCCTG CTGAGCGACATCCTGAG AGTGAACACCGAGATCA CCAAGGCCCCCCTGAGC GCCTCTATGATCAAGAG ATACGACGAGCACCACC AGGACCTGACCCTGCTG AAAGCTCTCGTGCGGCA GCAGCTGCCTGAGAAGT ACAAAGAGATTTTCTTC GACCAGAGCAAGAACGG CTACGCCGGCTACATTG ACGGCGGAGCCAGCCAG GAAGAGTTCTACAAGTT CATCAAGCCCATCCTGG AAAAGATGGACGGCACC GAGGAACTGCTCGTGAA GCTGAACAGAGAGGACC TGCTGCGGAAGCAGCGG ACCTTCGACAACGGCAG CATCCCCCACCAGATCC ACCTGGGAGAGCTGCAC GCCATTCTGCGGCGGCA GGAAGATTTTTACCCATT CCTGAAGGACAACCGGG AAAAGATCGAGAAGATC CTGACCTTCCGCATCCC TACTACGTGGGCCCTCT GGCCAGGGGAAACAGCA GATTCGCCTGGATGACC AGAAAGAGCGAGGAAA CCATCACCCCCTGGAAC TTCGAGGAAGTGGTGGA CAAGGGCGCTTCCGCCC | |
| | AGAGCTTCATCGAGCGG ATGACCAACTTCGATAA GAACCTGCCCAACGAGA AGGTGCTGCCCAAGCAC AGCCTGCTGTACGAGTA CTTCACCGTGTATAACG AGCTGACCAAAGTGAAA TACGTGACCGAGGGAAT GAGAAAGCCCGCCTTCC TGAGCGGCGAGCAGAAA AAGGCCATCGTGGACCT GCTGTTCAAGACCAACC GGAAAGTGACCGTGAAG CAGCTGAAAGAGGACTA CTTCAAGAAAATCGAGT GCTTCGACTCCGTGGAA ATCTCCGGCGTGGAAGA TCGGTTCAACGCCTCCCT GGGCACATACCACGATC TGCTGAAAATTATCAAG GACAAGGACTTCCTGGA CAATGAGGAAAACGAGG ACATTCTGGAAGATATC GTGCTGACCCTGACACT GTTTGAGGACAGAGAGA TGATCGAGGAACGGCTG AAAACCTATGCCCACCT GTTCGACGACAAAGTGA TGAAGCAGCTGAAGCGG CGGAGATACACCGGCTG GGGCAGGCTGAGCCGGA AGCTGATCAACGGCATC CGGGACAAGCAGTCCGG CAAGACAATCCTGGATT TCCTGAAGTCCGACGGC TTCGCCAACAGAAACTT CATGCAGCTGATCCACG ACGACAGCCTGACCTTT AAAGAGGACATCCAGAA AGCCCAGGTGTCCGGCC AGGGCGATAGCCTGCAC GAGCACATTGCCAATCT GGCCGGCAGCCCCGCCA TTAAGAAGGGCATCCTG CAGACAGTGAAGGTGGT GGACGAGCTCGTGAAAG | |
| | TGATGGGCCGGCACAAG CCCGAGAACATCGTGAT CGAAATGGCCAGAGAGA ACCAGACCACCCAGAAG GGACAGAAGAACAGCCG CGAGAGAATGAAGCGGA TCGAAGAGGGCATCAAA GAGCTGGGCAGCCAGAT CCTGAAAGAACACCCCG TGGAAAACACCCAGCTG CAGAACGAGAAGCTGTA CCTGTACTACCTGCAGA ATGGGCGGGATATGTAC GTGGACCAGGAACTGGA CATCAACCGGCTGTCCG ACTACGATGTGGACCAT ATCGTGCCTCAGAGCTTT CTGAAGGACGACTCCAT CGACAACAAGGTGCTGA CCAGAAGCGACAAGAAC CGGGGCAAGAGCGACAA CGTGCCCTCCGAAGAGG TCGTGAAGAAGATGAAG AACTACTGGCGGCAGCT GCTGAACGCCAAGCTGA TTACCCAGAGAAAGTTC GACAATCTGACCAAGGC CGAGAGAGGCGGCCTGA GCGAACTGGATAAGGCC GGCTTCATCAAGAGACA GCTGGTGGAAACCCGGC AGATCACAAAGCACGTG GCACAGATCCTGGACTC CCGGATGAACACTAAGT ACGACGAGAATGACAAG CTGATCCGGGAAGTGAA AGTGATCACCCTGAAGT CCAAGCTGGTGTCCGAT TTCCGGAAGGATTTCCA GTTTTACAAAGTGCGCG AGATCAACAACTACCAC CACGCCCACGACGCCTA CCTGAACGCCGTCGTGG GAACCGCCCTGATCAAA AAGTACCCTAAGCTGGA AAGCGAGTTCGTGTACG | |
| | GCGACTACAAGGTGTAC GACGTGCGGAAGATGAT CGCCAAGAGCGAGCAGG AAATCGGCAAGGCTACC GCCAAGTACTTCTTCTAC AGCAACATCATGAACTT TTTCAAGACCGAGATTA CCCTGGCCAACGGCGAG ATCCGGAAGCGGCCTCT GATCGAGACAAACGGCG AAACCGGGGAGATCGTG TGGGATAAGGGCCGGGA TTTTGCCACCGTGCGGA AAGTGCTGAGCATGCCC CAAGTGAATATCGTGAA AAAGACCGAGGTGCAGA CAGGCGGCTTCAGCAAA GAGTCTATCCTGCCCAA GAGGAACAGCGATAAGC TGATCGCCAGAAAGAAG GACTGGGACCCTAAGAA GTACGGCGGCTTCGACA GCCCCACCGTGGCCTAT TCTGTGCTGGTGGTGGC CAAAGTGGAAAAGGGCA AGTCCAAGAAACTGAAG AGTGTGAAAGAGCTGCT GGGGATCACCATCATGG AAAGAAGCAGCTTCGAG AAGAATCCCATCGACTT TCTGGAAGCCAAGGGCT ACAAAGAAGTGAAAAA GGACCTGATCATCAAGC TGCCTAAGTACTCCCTGT TCGAGCTGGAAAACGGC CGGAAGAGAATGCTGGC CTCTGCCGGCGAACTGC AGAAGGGAAACGAACTG GCCCTGCCCTCCAAATA TGTGAACTTCCTGTACCT GGCCAGCCACTATGAGA AGCTGAAGGGCTCCCCC GAGGATAATGAGCAGAA ACAGCTGTTTGTGGAAC AGCACAAGCACTACCTG GACGAGATCATCGAGCA | |
| | GATCAGCGAGTTCTCCA AGAGAGTGATCCTGGCC GACGCTAATCTGGACAA AGTGCTGTCCGCCTACA ACAAGCACCGGGATAAG CCCATCAGAGAGCAGGC CGAGAATATCATCCACC TGTTTACCCTGACCAATC TGGGAGCCCCTGCCGCC TTCAAGTACTTTGACACC ACCATCGACCGGAAGAG GTACACCAGCACCAAAG AGGTGCTGGACGCCACC CTGATCCACCAGAGCAT CACCGGCCTGTACGAGA CACGGATCGACCTGTCT CAGCTGGGAGGCGACAA AAGGCCGGCGGCCACGA AAAAGGCCGGCCAGGCA AAAAAGAAAAAGgaattcG GCAGTGGAGAGGGCAGA GGAAGTCTGCTAACATG CGGTGACGTCGAGGAGA ATCCTGGCCCAATGACC GAGTACAAGCCCACGGT GCGCCTCGCCACCCGCG ACGACGTCCCCAGGGCC GTACGCACCCTCGCCGC CGCGTTCGCCGACTACC CCGCCACGCGCCACACC GTCGATCCGGACCGCCA CATCGAGCGGGTCACCG AGCTGCAAGAACTCTTC CTCACGCGCGTCGGGCT CGACATCGGCAAGGTGT GGGTCGCGGACGACGGC GCCGCGGTGGCGGTCTG GACCACGCCGGAGAGCG TCGAAGCGGGGGCGGTG TTCGCCGAGATCGGCCC GCGCATGGCCGAGTTGA GCGGTTCCCGGCTGGCC GCGCAGCAACAGATGGA AGGCCTCCTGGCGCCGC ACCGGCCCAAGGAGCCC GCGTGGTTCCTGGCCAC | |
| | CGTCGGAGTCTCGCCCG ACCACCAGGGCAAGGGT CTGGGCAGCGCCGTCGT GCTCCCCGGAGTGGAGG CGGCCGAGCGCGCCGGG GTGCCCGCCTTCCTGGA GACCTCCGCGCCCCGCA ACCTCCCCTTCTACGAGC GGCTCGGCTTCACCGTC ACCGCCGACGTCGAGGT GCCCGAAGGACCGCGCA CCTGGTGCATGACCCGC AAGCCCGGTGCCTGAgaat tctaaCTAGAGCTCGCTGAT CAGCCTCGACTGTGCCTT CTAGTTGCCAGCCATCT GTTGTTTGCCCCTCCCCC GTGCCTTCCTTGACCCTG GAAGGTGCCACTCCCAC TGTCCTTTCCTAATAAAA TGAGGAAATTGCATCGC ATTGTCTGAGTAGGTGT CATTCTATTCTGGGGGGT GGGGTGGGGCAGGACAG CAAGGGGGAGGATTGGG AAGAgAATAGCAGGCAT GCTGGGGAgcggccgcaggaa cccctagtgatggagttggccactccct ctctgcgcgctcgctcgctcactgaggc cgggcgaccaaaggtcgcccgacgcc cgggctttgcccgggcggcctcagtga gcgagcgagcgcgcagctgcctgcag gggcgcctgatgcggtattttctccttac gcatctgtgcggtatttcacaccgcatac gtcaaagcaaccatagtacgcgccctgt agcggcgcattaagcgcggcgggtgtg gtggttacgcgcagcgtgaccgctaca cttgccagcgccctagcgcccgctccttt cgctttcttcccttcctttctcgccacgttc gccggctttccccgtcaagctctaaatcg ggggctccctttagggttccgatttagtg ctttacggcacctcgaccccaaaaaactt gatttgggtgatggttcacgtagtgggcc atcgccctgatagacggtttttcgcccttt gacgttggagtccacgttctttaatagtg gactcttgttccaaactggaacaacactc | |
| | aaccctatctcgggctattcttttgatttat aagggattttgccgatttcggcctattggt taaaaaatgagctgatttaacaaaaattta acgcgaattttaacaaaatattaacgttta caattttatggtgcactctcagtacaatct gctctgatgccgcatagttaagccagcc ccgacacccgccaacacccgctgacg cgccctgacgggcttgtctgctcccggc atccgcttacagacaagctgtgaccgtct ccgggagctgcatgtgtcagaggttttc accgtcatcaccgaaacgcgcgagacg aaagggcctcgtgatacgcctatttttata ggttaatgtcatgataataatggtttcttag acgtcaggtggcacttttcggggaaatg tgcgcggaacccctatttgtttatttttcta aatacattcaaatatgtatccgctcatgag acaataaccctgataaatgcttcaataat attgaaaaaggaagagtatgagtattca acatttccgtgtcgcccttattcccttttttg cggcattttgccttcctgtttttgctcaccc agaaacgctggtgaaagtaaaagatgct gaagatcagttgggtgcacgagtgggtt acatcgaactggatctcaacagcggtaa gatccttgagagttttcgccccgaagaa cgttttccaatgatgagcacttttaaagttc tgctatgtggcgcggtattatcccgtattg acgccgggcaagagcaactcggtcgc cgcatacactattctcagaatgacttggtt gagtactcaccagtcacagaaaagcatc ttacggatggcatgacagtaagagaatt atgcagtgctgccataaccatgagtgat aacactgcggccaacttacttctgacaa cgatcggaggaccgaaggagctaacc gcttttttgcacaacatgggggatcatgt aactcgccttgatcgttgggaaccggag ctgaatgaagccataccaaacgacgag cgtgacaccacgatgcctgtagcaatgg caacaacgttgcgcaaactattaactgg cgaactacttactctagcttcccggcaac aattaatagactggatggaggcggataa agttgcaggaccacttctgcgctcggcc cttccggctggctggtttattgctgataaa tctggagccggtgagcgtggaagccgc ggtatcattgcagcactggggccagatg gtaagccctcccgtatcgtagttatctac acgacggggagtcaggcaactatggat | |
| | gaacgaaatagacagatcgctgagata ggtgcctcactgattaagcattggtaact gtcagaccaagtttactcatatatacttta gattgatttaaaacttcatttttaatttaaaa ggatctaggtgaagatcctttttgataatc tcatgaccaaaatcccttaacgtgagtttt cgttccactgagcgtcagaccccgtaga aaagatcaaaggatcttcttgagatccttt ttttctgcgcgtaatctgctgcttgcaaac aaaaaaaccaccgctaccagcggtggt ttgtttgccggatcaagagctaccaactc tttttccgaaggtaactggcttcagcaga gcgcagataccaaatactgtccttctagt gtagccgtagttaggccaccacttcaag aactctgtagcaccgcctacatacctcg ctctgctaatcctgttaccagtggctgctg ccagtggcgataagtcgtgtcttaccgg gttggactcaagacgatagttaccggat aaggcgcagcggtcgggctgaacggg gggttcgtgcacacagcccagcttgga gcgaacgacctacaccgaactgagata cctacagcgtgagctatgagaaagcgc cacgcttcccgaagggagaaaggcgg acaggtatccggtaagcggcagggtcg gaacaggagagcgcacgagggagctt ccagggggaaacgcctggtatctttata gtcctgtcgggtttcgccacctctgactt gagcgtcgatttttgtgatgctcgtcagg ggggcggagcctatggaaaaacgcca gcaacgcggcctttttacggttcctggcc ttttgctggccttttgctcacatgt | |
| SEQ ID No. 23 (*Rho*^{D190N} donor template) | tcccttaaccaccgaaggcagggcagc aggctagtggagcagagctgcgtggtc aagtggcagggagcttaagaatcgtcc aagggcggagaccagtaagtctcatta ggtgatggggccagcaggtaaaagcc attcatgcttatgtccagctgggcgtgtgt tctcttcctgttttatcatcccttgcgctga ccatcaggtacatccctgagggcatgca atgttcatgcgggattgactattataccct taagccggaggtcaacaacgaatccttt gtcatctacatgttcgtggtccacttcacc attcctatgatcgtcatcttcttctgctatg ggcagctggtcttcacagtcaaggaggt atgagcaggg | *Mus musculus* |
| SEQ ID No. 24 (wt *RHO* exon1 donor template) | atgctcacctgaataacctggcagcctg ctccctcatgcagggaccacgtcctgct gcacccagcaggccatcccgtctccata gcccatggtcatccctccctggacagga atgtgtctcctccccgggctgagtcttgc tcaagctagaagcactccgaacagggtt atgggcgcctcctccatctcccaagtgg ctggcttatgaatgtttaatgtacatgtga gtgaacaaattccaattgaacgcaacaa atagttatcgagccgctgagccggggg gcgggggggtgtgagactggaggcgat ggacggagctgacggcacacacagct cagatctgtcaagtgagccattgtcagg gcttggggactggataagtcagggggt ctcctgggaagagatgggataggtgag ttcaggaggagacattgtcaactggagc catgtggagaagtgaatttagggcccaa aggttccagtcgcagcctgaggccacc agactgacatggggaggaattcccaga ggactctggggcagacaagatgagaca ccctttcctttctttacctaagggcctcca cccgatgtcaccttggcccctctgcaag ccaattaggccccggtggcagcagtgg gattagcgttagtatgatatctcgcggat gctgaatcagcctctggcttagggagag aaggtcactttataagggtctggggggg gtcagtgcctggagttgcgctgtgggag cgagtcatccagctggagccctgagtg gctgagctcaggccttcgcagcattcttg ggtgggagcagccacgggtcagccac aagggccacagccatgaatggcacag aaggccctaacttctacgtgcccttctcc aatgcgacgggtgtggtacgcagcccc ttcgagtacccacagtactacctggctga gccatggcagttctccatgctggccgcc tacatgtttctgctgatcgtgctgggcttc cccatcaacttcctcacgctctacgtcac cgtccagcacaagaagctgcgcacgcc tctcaactacatcctgctcaacctagccg tggctgacctcttcatggtcctaggtggc ttcaccagcaccctctacacctctctgca tggatacttcgtcttcgggcccacaggat gcaatttggagggcttctttgccaccctg ggcggtatgagcagagagactggggc gggggggtgtagcatgggagccaagg ggccacgaaagggcctgggagggtct | *Mus musculus* & *Homo sapiens* |
| | gcagcttacttgagtctctttaattggtctc atctaaaggcccagcttattcattggcaa acactgtgaccctgagctaggctgctgtt gagagcaggcacggaacattcatctatc tcatcttgagcaatgcaagaaacatggg ttcagagaggccaaggactcaccgagg agtcacagagtgtgggggtgtcctctga ggcagctgagctggggcacacacaga ctgagcaccaggagtgagctctagctttt tttttctatgtgtcttttctaaaagacacat aggtttaggactgtccctggtccaggtaa gaactggttcagtaaacttgtacatctca ctgcctggccagccctgtcagcttccac cagagtgcgtgcactacacacccggca tctcaaaggattcattcctatctttcctatct ttggagtgaggcacagtctcacgtagtc cagtccagactggccttaaattctgcagc tgaggatgtacttaaacttgtcatcctcct gccccagcctctcaagtgctgtgatcac aggcacggaccactatgctacgccagg tgtttccaaacattttctctcccttaactgg aaggtcaatgaggctctttcgagaagca acagagcctgtttagctgagaaaactga ggcagggagcaggcaa | |
| SEQ ID No. 25 (mutant *RHO* exon1 donor template) | atgctcacctgaataacctggcagcctg ctccctcatgcagggaccacgtcctgct gcacccagcaggccatcccgtctccata gcccatggtcatccctccctggacagga atgtgtctcctccccgggctgagtcttgc tcaagctagaagcactccgaacagggtt atgggcgcctcctccatctcccaagtgg ctggcttatgaatgtttaatgtacatgtga gtgaacaaattccaattgaacgcaacaa atagttatcgagccgctgagccggggg gcgggggggtgtgagactggaggcgat ggacggagctgacggcacacacagct cagatctgtcaagtgagccattgtcagg gcttggggactggataagtcaggggggt ctcctgggaagagatgggataggtgag ttcaggaggagacattgtcaactggagc catgtggagaagtgaatttagggcccaa aggttccagtcgcagcctgaggccacc agactgacatggggaggaattcccaga ggactctggggcagacaagatgagaca ccctttcctttctttacctaagggcctcca cccgatgtcaccttggcccctctgcaag ccaattaggccccggtggcagcagtgg gattagcgttagtatgatatctcgcggat gctgaatcagcctctggcttagggagag aaggtcactttataagggtctggggggg gtcagtgcctggagttgcgctgtgggag cgagtcatccagctggagccctgagtg gctgagctcaggccttcgcagcattcttg ggtgggagcagccacgggtcagccac aagggccacagccatgaatggcacag aaggccctaacttctacgtgcccttctcc aatgcgacgggtgtggtacgcagcccc ttcgagtacccacagtactacctggctga gccatggcagttctccatgctggccgcc tacatgtttctgctgatcgtgctgggcttc cccatcaacttcctcacgctctacgtcac cgtccagcacaagaagctgcgcacgcc tctcaactacatcctgctcaacctagccg tggctgacctcttcatggtcctaggtggc ttcaccagcaccctctacacctctctgca tggatacttcgtcttcgggcccacagga cgcaatttggagggcttctttgccaccct gggcggtatgagcagagagactgggg cgggggggtgtagcatgggagccaag gggccacgaaagggcctgggagggtc | *Mus musculus* & *Homo sapiens* |
| | tgcagcttacttgagtctctttaattggtct catctaaaggcccagcttattcattggca aacactgtgaccctgagctaggctgctg ttgagagcaggcacggaacattcatcta tctcatcttgagcaatgcaagaaacatgg gttcagagaggccaaggactcaccgag gagtcacagagtgtgggggtgtcctctg aggcagctgagctggggcacacacag actgagcaccaggagtgagctctagctt tttttttctatgtgtcttttctaaaagacaca taggtttaggactgtccctggtccaggta agaactggttcagtaaacttgtacatctc actgcctggccagccctgtcagcttcca ccagagtgcgtgcactacacacccggc atctcaaaggattcattcctatctttcctat ctttggagtgaggcacagtctcacgtagt ccagtccagactggccttaaattctgcag ctgaggatgtacttaaacttgtcatcctcc tgccccagcctctcaagtgctgtgatcac aggcacggaccactatgctacgccagg tgtttccaaacattttctctcccttaactgg aaggtcaatgaggctctttcgagaagca acagagcctgtttagctgagaaaactga ggcagggagcaggcaa | |
| SEQ ID No. 26 (*Pde6a*^{D670G} donor template) | tgagagatgaggtagggtggcgcccat ctcgagggcagcttgcgtgagcacagg cagccttcttgccattggctgaggctgtc attgccgtcaccacttcgggtgggcacc ggaagaagagtgaccttattgccagcac catttctcaaacgttgtctaattcttttctct agagcctgaatatcttccagaatctcaac cgacgtcaacacgagcatgcgatccac atgatggacatcgcgatcattgccacag accttgccttgtatttcaagtgggtatttct cctcactttaatagtagcagtgtgggggc tggagagatggttcagtggttaacagca ctgactgctcttccagaggtcctgagttc aaatcccagcaaccacatggtggctcac aactatctgtaatgggatctgataccctct tctggtgtgtgtctgaagacagcgatgg agtactcacat | *Mus musculus* |
| SEQ ID NO: 27 (*EFEMP1*^{R345W} donor template) | tagttagtaaactctttgaccctacatctctacagatataaatgagtgtgagacca caaaCgaGtgcCgggaggatgaaatgtgttggaattatcatggcggcttcc gttgttatccacgaaatcctt | *Homo sapiens* |

The following items are herein described.
1. A method for treating an autosomal dominant ocular disease in a subject, comprising, administering to the subject a therapeutically effective amount of at least one type of recombinant adeno-associated viral (AAV) vector encoding a CRISPR-Cas system directed to an autosomal dominant disease-related gene, wherein the at least one type of the AAV vector comprises:
   (i) a first sequence(s) encoding at least one guide RNA that hybridizes to the endogenous autosomal dominant disease-related gene in the subject;
   (ii) a second sequence comprising a codon-modified autosomal dominant disease-related gene or fragment thereof, wherein at least one disease related mutation has been corrected in the codon-modified autosomal dominant disease-related gene or fragment thereof, and wherein the codon-modified autosomal dominant disease-related gene or fragment thereof is not recognized by the guide RNA; and,
   (iii) a third sequence encoding a Cas nuclease.
2. The method of item 1, wherein two types of recombinant AAV vectors are administered to the subject, wherein a first type of recombinant AAV vector comprises the first sequence(s) and the second sequence, and wherein a second type of recombinant AAV vector comprises the third sequence.
3. The method of item 1, wherein the ocular disease is selected from the group consisting of, autosomal dominant chorioretinal atrophy or degeneration, autosomal dominant cone or cone-rod dystrophy, autosomal dominant congenital stationary night blindness, autosomal dominant leber congenital amaurosis, autosomal dominant macular degeneration, autosomal dominant ocular-retinal developmental disease, autosomal dominant optic atrophy, autosomal dominant retinitis pigmentosa, autosomal dominant syndromic/systemic diseases with retinopathy, sorsby macular dystrophy, age-related macular degeneration, doyne honeycomb macular disease, and juvenile macular degeneration.
4. The method of item 1, wherein the ocular disease is retinitis pigmentosa.
5. The method of item 1, wherein the autosomal dominant disease-related gene is the *RHO* gene.
6. The method of item 1, wherein the ocular disease is age-related macular degeneration.
7. The method of item 1, wherein the ocular disease is doyne honeycomb.
8. The method of item 1, wherein the autosomal dominant disease-related gene is the *EFEMP1* gene.
9. The method of items 1 or 2, wherein the recombinant AAV vector is an AAV2 vector.
10. The method of items 1 or 2, wherein the AAV vector is an AAV8 vector.
11. The method of item 1, wherein the Cas nuclease is Cas9.
12. The method of item 1, wherein the CRISPR-Cas system is under the control of a promoter which controls expression of the codon-modified autosomal dominant disease-related gene product in ocular cells.
13. The method of item 1, wherein the codon-modified autosomal dominant disease-related gene or fragment thereof is integrated into the endogenous autosomal dominant disease-related gene.
14. The method of item 1, wherein the codon-modified autosomal dominant disease-related gene or fragment thereof is not integrated into the endogenous autosomal dominant disease-related gene.
15. The method of item 1, wherein the first sequence encoding at least one guide RNA is selected from the group consisting of, SEQ ID NO: 1, SEQ ID NO: 2, SEQ ID NO: 3, SEQ ID NO: 4, SEQ ID NO: 5, SEQ ID NO: 6, SEQ ID NO: 7, or combinations thereof.
16. The method of item 1, wherein autosomal dominant disease-related gene is a gene selected from the group consisting of, PRDM13, RGR, TEAD1, AIPL1, CRX, GUCA1A, GUCY2D, PITPNM3, PROM1, PRPH2,RIMS1, SEMA4A, UNC119, GNAT1, PDE6B, RHO, WSF1, IMPDH1, OTX2, BEST1, C1QTNF5, CTNNA1, EFEMP1, ELOVL4, FSCN2,GUCA1B, HMCN1, IMPG1, RP1L1, TIMP3, VCAN, MFN2, NR2F1, OPA1, ARL3, CA4, HK1, KLHL7, NR2E3, NRL, PRPF3, PRPF4, PRPF6, PRPF8, PRPF31, RDH12, ROM1, RP1, RP9, RPE65, SNRNP200, SPP2, TOPORS, ABCC6, ATXN7, COL11A1, COL2A1, JAG1, KCNJ13, KIF11, OPA3, PAX2, TREX1, CAPN5, CRB1, FZD4, ITM2B, LRP5, MAPKAPK3, MIR204, OPN1SW, RB1, TSPAN12, and ZNF408.
17. The method of item 1, wherein the recombinant AAV vector is administered by injection into the eye.
18. The method of item 1, wherein the codon-modified autosomal dominant disease-related gene or fragment thereof is selected from the group consisting of, SEQ ID NO: 8, SEQ ID NO: 9, SEQ ID NO: 23, SEQ ID NO: 24, SEQ ID NO: 25, SEQ ID NO: 26, and SEQ ID NO: 27, or combinations thereof.
19. A method for treating an autosomal dominant ocular disease in a subject, comprising administering to the subject a therapeutically effective amount of:
   (a) a first recombinant adeno-associated viral (AAV) vector encoding a CRISPR-Cas system directed to an autosomal dominant disease-related gene, wherein the first recombinant AAV comprises,
      (i) a first sequence(s) encoding at least one guide RNA that hybridizes to the endogenous autosomal dominant disease-related gene in the subject;
      (ii) a second sequence comprising a codon-modified autosomal dominant disease-related gene or fragment thereof, wherein at least one disease related mutation has been corrected in the modified autosomal dominant disease-related gene or fragment thereof, and wherein the modified autosomal dominant disease related gene or fragment thereof is not recognized by the guide RNA; and,
   (b) a second recombinant AAV viral vector comprising a nucleic acid sequence encoding a Cas nuclease.
20. The method of item 19, wherein the ocular disease is selected from the group consisting of: autosomal dominant chorioretinal atrophy or degeneration, autosomal dominant cone or cone-rod dystrophy, autosomal dominant congenital stationary night blindness, autosomal dominant leber congenital amaurosis, autosomal dominant macular degeneration, autosomal dominant ocular-retinal developmental disease, autosomal dominant optic atrophy, autosomal dominant retinitis pigmentosa, autosomal dominant syndromic/systemic diseases with retinopathy, sorsby macular dystrophy, age-related macular degeneration, doyne honeycomb macular disease, and juvenile macular degeneration.
21. The method of item 19, wherein the ocular disease is retinitis pigmentosa.
22. The method of item 21, wherein the autosomal dominant disease-related gene is the *RHO* gene.
23. The method of item 19, wherein the ocular disease is age-related macular degeneration.
24. The method of item 19, wherein the ocular disease is doyne honeycomb.
25. The method of item 24, wherein the autosomal dominant disease-related gene is the *EFEMP1* gene.
26. The method of item 19, wherein the recombinant AAV vector is an AAV2 vector.
27. The method of item 19, wherein the AAV vector is an AAV8 vector.
28. The method of item 19, wherein the Cas nuclease is Cas9.
29. The method of item 19, wherein the CRISPR-Cas system is under the control of a promoter which controls expression of the modified autosomal dominant disease-related gene product in ocular cells.
30. The method of item 19, where the the codon-modified autosomal dominant disease-related gene or fragment thereof is integrated into the endogenous autosomal disease-related gene.
31. The method of item 19, wherein the codon-modified autosomal dominant disease-related gene or fragment thereof is not integrated into the endogenous autosomal disease-related gene.
32. The method of item 19, wherein the first sequence encoding at least one guide RNA is selected from the group consisting of, SEQ ID NO: 1, SEQ ID NO: 2, SEQ ID NO: 3, SEQ ID NO: 4, SEQ ID NO: 5, SEQ ID NO: 6, and SEQ ID NO: 7 or combinations thereof.
33. The method of item 19, wherein autosomal dominant disease-related gene is gene selected from the group consisting of, PRDM13, RGR, TEAD1, AIPL1, CRX, GUCA1A, GUCY2D, PITPNM3, PROM1, PRPH2,RIMS1, SEMA4A, UNC119, GNAT1, PDE6B, RHO, WSF1, IMPDH1, OTX2, BEST1, C1QTNF5, CTNNA1, EFEMP1, ELOVL4, FSCN2, GUCA1B, HMCN1, IMPG1, RP1L1, TIMP3, VCAN, MFN2, NR2F1, OPA1, ARL3, CA4, HK1, KLHL7, NR2E3, NRL, PRPF3, PRPF4, PRPF6, PRPF8, PRPF31, RDH12, ROM1, RP1, RP9, RPE65, SNRNP200, SPP2, TOPORS, ABCC6, ATXN7, COL11A1, COL2A1, JAG1, KCNJ13, KIF11, OPA3, PAX2, TREX1, CAPN5, CRB1, FZD4, ITM2B, LRP5, MAPKAPK3, MIR204, OPN1SW, RB1, TSPAN12, and ZNF408.
34. The method of item 19, wherein the recombinant AAV viral vector is administered by injection.
35. The method of item 19, wherein the codon-modified autosomal dominant disease-related gene or fragment thereof is selected from the group consisting of, SEQ ID NO: 8, SEQ ID NO: 9, SEQ ID NO: 23, SEQ ID NO: 24, SEQ ID NO: 25, SEQ ID NO: 26, SEQ ID NO: 27, or combinations thereof.
36. The method of items 1 or 19, wherein the first sequences encode two guide RNAs.
37. The method of items 1 or 19, wherein the endogenous autosomal dominant disease-related gene is wildtype and/or mutant.

## Claims

1. A composition for use in treating an autosomal dominant ocular disease in a subject, the use comprising administering to the subject a therapeutically effective amount of:
(a) a first recombinant adeno-associated viral (AAV) vector encoding a CRISPR-Cas system directed to an autosomal dominant disease-related gene, wherein the first recombinant AAV comprises:
(i) a first sequence encoding at least two guide RNAs that hybridize to the endogenous autosomal dominant disease-related gene in the subject;
(ii) a second sequence comprising a codon-modified autosomal dominant disease-related gene or fragment thereof, wherein at least one disease related mutation has been corrected in the modified autosomal dominant disease-related gene or fragment thereof, and wherein the modified autosomal dominant disease related gene or fragment thereof is not recognized by the guide RNAs; and,
(b) a second recombinant AAV viral vector comprising a nucleic acid sequence encoding a Cas nuclease.

2. The composition for use according to claim 1, wherein the ocular disease is selected from the group consisting of: autosomal dominant chorioretinal atrophy or degeneration, autosomal dominant cone or cone-rod dystrophy, autosomal dominant congenital stationary night blindness, autosomal dominant leber congenital amaurosis, autosomal dominant macular degeneration, autosomal dominant ocular-retinal developmental disease, autosomal dominant optic atrophy, autosomal dominant retinitis pigmentosa, autosomal dominant syndromic/systemic diseases with retinopathy, sorsby macular dystrophy, age-related macular degeneration, doyne honeycomb macular disease, and juvenile macular degeneration.

3. The composition for use according to claim 1, wherein the ocular disease is retinitis pigmentosa, age-related macular degeneration, or doyne honeycomb.

4. The composition for use according to claim 3, wherein the autosomal dominant disease-related gene is the RHO gene, or the EFEMP1 gene.

5. The composition for use according to claim 1, wherein the recombinant AAV vector is an AAV2 vector, or an AAV8 vector.

6. The composition for use according to claim 1, wherein the Cas nuclease is Cas9.

7. The composition for use according to claim 19, wherein the CRISPR-Cas system is under the control of a promoter, which controls expression of the modified autosomal dominant disease-related gene product in ocular cells.

8. The composition for use according to claim 1, wherein:
(i) the codon-modified autosomal dominant disease-related gene or fragment thereof is integrated into the endogenous autosomal disease-related gene; or
(ii) the codon-modified autosomal dominant disease-related gene or fragment thereof is not integrated into the endogenous autosomal disease-related gene.

9. The composition for use according to claim 1, wherein at least one guide RNA is selected from SEQ ID NO: 1, SEQ ID NO: 2, SEQ ID NO: 3, SEQ ID NO: 4, SEQ ID NO: 5, SEQ ID NO: 6, SEQ ID NO: 7, or combinations thereof.

10. The composition for use according to claim 1, wherein the autosomal dominant disease-related gene is gene selected from PRDM13, RGR, TEAD1, AIPL1, CRX, GUCA1A, GUCY2D, PITPNM3, PROM1, PRPH2,RIMS 1, SEMA4A, UNCI 19, GNAT1, PDE6B, RHO, WSF1, IMPDH1, OTX2, BEST1, C1QTNF5, CTNNA1, EFEMP1, ELOVL4, FSCN2, GUCA1B, HMCN1, IMPG1, RP1L1, TIMP3, VCAN, MFN2, NR2F1, OPA1, ARL3, CA4, HK1, KLHL7, NR2E3, NRL, PRPF3, PRPF4, PRPF6, PRPF8, PRPF31, RDH12, ROM1, RP1, RP9, RPE65, SNRNP200, SPP2, TOPORS, ABCC6, ATXN7, COL11A1, COL2A1, JAG1, KCNJ13, KIF11, OPA3, PAX2, TREX1, CAPN5, CRB 1, FZD4, ITM2B, LRP5, MAPKAPK3, MIR204, OPN1SW, RB I, TSPAN12, or ZNF408.

11. The composition for use according to claim 1, wherein the recombinant AAV viral vector is administered by injection.

12. The composition for use according to claim 1, wherein the codon-modified autosomal dominant disease-related gene or fragment thereof is selected from SEQ ID NO: 8, SEQ ID NO: 9, SEQ ID NO: 23, SEQ ID NO: 24, SEQ ID NO: 25, SEQ ID NO: 26, SEQ ID NO: 27, or combinations thereof.

13. The composition for use according to claim 1, wherein the two guide RNAs are SEQ ID NO: 1 and SEQ ID NO: 2.

14. The composition for use according to claim 1, wherein the endogenous autosomal dominant disease-related gene is wild-type and/or mutant.
